# EUROPEAN PATENT SPECIFICATION

(11) **EP 4 626 255 B1**
(45) Date of publication and mention of the grant of the patent: **09.09.2026**
(21) Application number: 23813758.2
(22) Date of filing: 29.11.2023
(51) Int. Cl.: A23L 27/20, A61Q 13/00, C11B 9/00, C11D 9/44

(54) **ACETIC ACID FOR INTENSIFYING OR MODIFYING THE AROMA OF AROMA CHEMICALS**
ESSIGSÄURE ZUR INTENSIVIERUNG ODER MODIFIZIERUNG DES AROMAS VON AROMACHEMIKALIEN
ACIDE ACÉTIQUE POUR INTENSIFIER OU MODIFIER L'ARÔME DE PRODUITS CHIMIQUES AROMATIQUES

(30) Priority: 30.11.2022 EP 22210633
(43) Date of publication of application: 08.10.2025
(73) Proprietor: BASF SE, 67056 Ludwigshafen am Rhein (DE)
(72) Inventor: MORMUL, Jaroslaw Michael, 67056 Ludwigshafen am Rhein (DE); HUNDEMER, Fabian, 67056 Ludwigshafen am Rhein (DE); HARTMANN, Marco, 67056 Ludwigshafen am Rhein (DE); GARLICHS, Florian, 67056 Ludwigshafen am Rhein (DE); PELZER, Ralf, 67056 Ludwigshafen am Rhein (DE)
(74) Representative: BASF IP Association
(86) International application number: PCT/EP2023/083596
(87) International publication number: WO 2024/115594

(56) References cited:
- EP-B1- 0 998 444
- WO-A1-2022/248579
- CN-A- 108 219 944
- CN-A- 110 897 142
- CN-A- 112 574 819
- JP-A- 2002 003 886
- US-A1- 2010 210 496

## Description

The present invention relates to the use of acetic acid for intensifying and/or modifying the aroma, preferably the fragrance, of an aroma chemical which contains one or more acetate or propionate groups (i.e. acetic acid ester or propionic acid ester groups); to the use of a composition containing acetic acid and at least one aroma chemical which contains one or more acetate or propionate groups as an aroma composition, preferably as a fragrance; to a method for intensifying and/or modifying the aroma of an aroma chemical which contains one or more acetate or propionate groups by providing a composition containing said aroma chemical and 50 to 600 ppm of acetic acid, relative to the total weight of acetic acid and said aroma chemical; to a composition comprising at least one aroma chemical which contains one or more acetate or propionate groups, acetic acid in an amount of from 50 to 600 ppm, relative to the total weight of acetic acid and said aroma chemical(s), and at least one further component selected from the group consisting of aroma chemicals different from said aroma chemicals which contain one or more acetate or propionate groups, non-aroma chemical carriers, anti-oxidants and deodorant-active agents; to a method for preparing an aroma chemical composition, and to a method for conferring an aroma to a composition.

### TECHNICAL BACKGROUND

Aroma chemicals, especially fragrances, are of great interest especially in the field of cosmetics and cleaning and laundry compositions.

The market for aroma chemicals is constantly changing. Customers and retailers are always on the lookout for new fragrances that match current trends. However, the development of new fragrances is a risky and costly affair. New products must be elaborately identified, the developed route must be economically viable, and the final products biodegradable and toxicologically harmless. From a sustainability point of view, it would also be advantageous if existing and established fragrances could simply be modified instead of doing a full redevelopment each time. Additionally or alternatively, it would be an advantage if the intensity of existing fragrance components could be increased in a simple way in order to be able to use less material.

It was thus the object of the present invention to find a way to intensify and/or modify the aroma of known aroma chemicals. Besides, the substance used to this purpose should be well combinable with said known aroma chemicals, economically available, environmentally friendly and toxicologically harmless.

EP 0998444 B1 relates to a process for preparing 4-tert-butylcyclohexyl acetate, which has woody and fruity fragrance and is used as a perfume in soaps, detergents or shampoos. This acetate is to have a content of the cis isomer of at least 90%, and is to contain less than 50 ppm of acetic acid, since, if the content of acetic acid exceeds 50 ppm, allegedly a desirable perfume cannot be obtained. In a comparative example, 4-tert-butylcyclohexyl acetate which contains 94 ppm of acetic acid is prepared.

It was surprisingly found that acetic acid, when used in certain concentrations, advantageously intensifies and/or modifies the aroma, in particular the fragrance, of known aroma chemicals having acetate or propionate groups. Just by way of example. in the case of 1,4-butanediol diacetate, the fragrance thereof is intensified by the presence of acetic acid in certain concentrations, and is also perceived as more natural or "greener". This advantageous effect is observed both when acetic acid and the respective aroma chemical are mixed and when the preparation, purification or isolation process in the preparation of aroma chemicals having one or more acetate groups is carried out thus that the resulting product contains acetic acid in certain amounts.

### SUMMARY OF THE INVENTION

The invention relates to the use of acetic acid for intensifying and/or modifying the aroma, preferably the fragrance, of an aroma chemical (I) which contains one or more acetate or propionate groups (i.e. acetic acid ester or propionic acid ester groups) or of a mixture of different aroma chemicals (I), where acetic acid is used in an amount of from 50 to 600 ppm, relative to the total weight of acetic acid and said aroma chemical(s) (I).

The aroma chemical which contains one or more acetate or propionate groups is also abbreviated as aroma chemical (I).

The invention relates also to the use of a composition containing acetic acid and at least one aroma chemical (I) which contains one or more acetate or propionate groups (i.e. acetic acid ester or propionic acid ester groups), where acetic acid is contained in an amount of from 50 to 600 ppm, relative to the total weight of acetic acid and said aroma chemical(s) (I), as an aroma composition, preferably as a fragrance.

The invention relates moreover to a method for intensifying and/or modifying the aroma, preferably the fragrance, of an aroma chemical (I) which contains one or more acetate or propionate groups (i.e. acetic acid ester or propionic acid ester groups) or of a mixture of different aroma chemicals (I), comprising providing a composition containing at least one of said aroma chemicals and 50 to 600 ppm of acetic acid, relative to the total weight of acetic acid and said aroma chemical(s) (I). Preferably, the aroma chemical (I) is not 4-tert-butylcyclohexyl acetate if the composition contains less than 100 ppm of acetic acid.

The invention relates furthermore to a composition comprising
- at least one aroma chemical (I) which contains one or more acetate or propionate groups (i.e. acetic acid ester or propionic acid ester groups),
- acetic acid in an amount of from 50 to 600 ppm, relative to the total weight of acetic acid and said aroma chemical(s) (I), and
- at least one further component selected from the group consisting of aroma chemicals different from said aroma chemicals (I) which contain one or more acetate or propionate groups, non-aroma chemical carriers, anti-oxidants and deodorant-active agents. Preferably, the aroma chemical (I) is not 4-tert-butylcyclohexyl acetate if the composition contains less than 100 ppm of acetic acid.

Aroma chemicals different from aroma chemicals (I) which contain one or more acetate or propionate groups are also abbreviated below as aroma chemicals (II).

In another aspect, the invention relates to a method for preparing an aroma chemical composition, comprising incorporating at least one aroma chemical (I) which contains one or more acetate or propionate groups and acetic acid in such an amount that it (= acetic acid) is present in said composition in an amount of from 50 to 600 ppm, relative to the total weight of acetic acid and said aroma chemical(s) (I), into said composition; or mixing at least one aroma chemical (I) which contains one or more acetate or propionate groups and acetic acid in such an amount that it (= acetic acid) is present in the resulting composition in an amount of from 50 to 600 ppm, relative to the total weight of acetic acid and said aroma chemical(s) (I), simultaneously or consecutively with the other components of said composition or with pre-formed mixtures of a part of the other components of said composition. Preferably, the aroma chemical (I) is not 4-tert-butylcyclohexyl acetate if acetic acid is present in the resulting composition in an amount of less than 100 ppm.

In yet another aspect, the invention relates to a method for conferring an aroma, preferably a fragrance, to a composition, comprising incorporating at least one aroma chemical (I) which contains one or more acetate or propionate groups and acetic acid in such an amount that it (= acetic acid) is present in said composition in an amount of from 50 to 600 ppm, relative to the total weight of acetic acid and said aroma chemical(s) (I), into said composition; or comprising mixing at least one aroma chemical (I) which contains one or more acetate or propionate groups and acetic acid in such an amount that it (= acetic acid) is present in the resulting composition in an amount of from 50 to 600 ppm, relative to the total weight of acetic acid and said aroma chemical(s) (I), simultaneously or consecutively with the other components of said composition or with pre-formed mixtures of a part of the other components of said composition. Preferably, the aroma chemical (I) is not 4-tert-butylcyclohexyl acetate if acetic acid is present in the resulting composition in an amount of less than 100 ppm.

### DETAILED DESCRIPTION OF THE INVENTION

### Definitions:

In the context of the present invention, the term "aroma" refers to a sensory property and comprises an odor and/or a flavor.

The term "aroma chemical" denotes a substance which is used to obtain an aroma impression (the term "aroma impression" is used interchangeably herein with the term "note") and comprises its use to obtain an olfactory and/or a flavor impression. The term "olfactory impression" or "odor impression" denotes an odor impression without any positive or negative judgement, while the term "scent impression" or "fragrance impression" (used interchangeably herein) as used herein is connected to an odor impression which is generally felt as pleasant. Thus a "fragrance" or "scent" denotes an aroma chemical which predominately induces a pleasant odor impression. A flavor denotes an aroma chemical which induces a taste impression.

The term "aroma profile" denotes the overall aroma impression of an aroma chemical and is composed of the individual aroma impressions of an aroma chemical.

The term "aroma composition", as used herein, refers to a composition which induces an aroma. The term aroma composition comprises "odor composition" and/or "flavor composition". An odor composition is a composition which predominately induces an odor impression, whereas a flavor composition is a composition which predominantly induces a taste impression.

The term "odor composition" comprises "fragrance composition" or "scent composition" (used interchangeably herein), which predominately induce an odor impression which is generally felt as pleasant.

"Pleasant odor", "pleasant odor impression", "pleasant odiferous properties", "odor impression felt as pleasant" and similar terms are hedonistic expressions which describe the niceness and conciseness of an odor impression conveyed by an aroma chemical. The more general hedonistic expressions "advantageous sensory properties" or "advantageous organoleptic properties" describe the niceness and conciseness of an organoleptic impression conveyed by an aroma chemical. In terms of the present invention, the terms "organoleptic" and "sensory" relate to olfactory or flavor properties. "Niceness" and "conciseness" are terms which are familiar to the person skilled in the art, a perfumer. Niceness generally refers to a spontaneously brought about, positively perceived, pleasant sensory impression. However, "nice" does not have to be synonymous with "sweet". "Nice" can also be the odor of musk or sandalwood. "Conciseness" generally refers to a spontaneously brought about sensory impression which - for the same test panel - brings about a reproducibly identical reminder of something specific. For example, a substance can have an odor which is spontaneously reminiscent of that of an "apple": the odor would then be concisely of "apples". If this apple odor were very pleasant because the odor is reminiscent, for example, of a sweet, fully ripe apple, the odor would be termed "nice". However, the odor of a typical apple tart can also be concise. If both reactions arise upon smelling the substance, in the example thus a nice and concise apple odor, then this substance has particularly advantageous sensory properties.

The term "odor-intensive substances" refers to substances or aroma chemicals exhibiting intense odor impressions. Intense odor impressions are to be understood as meaning those properties of aroma chemicals which permit a striking perception even in very low gas space concentrations.

The intensity can be determined via a threshold value determination. A threshold value is the concentration of a substance in the relevant gas space at which an odor impression can just still be perceived by a representative test panel, although it no longer has to be defined. A substance class which probably belongs to the most odor-intensive known substance classes, i.e. has very low odor threshold values, are thiols, whose threshold value is often in the ppb/m³ range.

In context with intensifying the aroma of the aroma chemicals (I) (alternatively expressed: enhancing the intensity of their aroma) by acetic acid, intensity is however a relative value determined in comparison with the basic aroma of the aroma chemicals (I) without acetic acid (or with the acetic acid amount being below the lower limit of the stated range).

The term "intensifying the aroma of an aroma chemical (I) which contains one or more acetate or propionate groups" is thus to be understood as intensifying the aroma properties of said aroma chemical (I) (i.e. the aroma is felt as stronger, more intense), as compared to said aroma chemical (I) without acetic acid (or with the acetic acid amount being below the lower limit of the stated range). Said intensification may be by 10%, 20%, 30%, 40%, 50% or more compared to said aroma chemical (I) without acetic acid (or with the acetic acid amount being below the lower limit of the stated range). In one embodiment, the intensification is at least 80%, or at least 100%.

The expression "modifying the aroma of an aroma chemical (I) which contains one or more acetate or propionate groups" is to be understood as changing its aroma character or profile, as compared to said aroma chemical (I) without acetic acid (or with the acetic acid amount being below the lower limit of the stated range). The modification is such that the aroma continues to be felt as nice and pleasant. In particular, it is not such that acetic acid is perceived, since this substance is generally felt as disagreeable or even aggressive. Just by way of example, the addition of acetic acid to 1,4-butanediol diacetate in the stated amounts modifies the aroma of the latter from a fruity and sweet type to a more natural and greener character. This aroma modification is for example a benefit for homecare compositions, such as laundry detergents, where a natural, green scent is generally associated with cleanliness.

The more general term "booster", "boosting" or "boost" is used herein to describe the effect of enhancing and/or modifying the aroma of an aroma chemical or of a composition. The term "enhancing" comprises an improvement of the niceness and/or conciseness of an aroma and/or an improvement of the intensity. The term "modifying" comprises the change of an aroma profile.

Booster effects are particularly desired in fragrance composition when top-note-characterized applications are required, in which the odor is to be conveyed particularly quickly and intensively, for example in deodorants, air fresheners or in the taste sector in chewing gums.

The term "tenacity" describes the evaporation behavior over time of an aroma chemical. The tenacity can for example be determined by applying the aroma chemical to a test strip, and by subsequent olfactory evaluation of the odor impression of the test strip. For aroma chemicals with high tenacity the time span after which the panel can still identify an aroma impression is long.

The term "substantivity" describes the interaction of an aroma chemical with a surface, such as for example the skin or a textile, especially after subsequent treatment of the surface, such as for example washing. The substantivity can for example be determined by washing a textile with a textile detergent composition comprising the aroma chemical and subsequent olfactory evaluation of the textile directly after washing (wet textile) as well as evaluation of the dry textile after prolonged storage.

The term "stability" describes the behavior of an aroma chemical upon contact with oxygen, light and/or other substances. An aroma chemical with high stability maintains its aroma profile over a long period in time, preferably in a large variety of compositions and under various storage conditions.

In order to impart a long-lasting aroma impression to a composition or to a surface treated with a composition, the tenacity, the substantivity as well as the stability of the aroma chemical in the compositions should preferably be high.

The terms "compound" and "substance" are used synonymously throughout the invention.

In the context of the present invention, the term "acetate groups" relates to acetic acid ester groups (-O-C(=O)-CH₃) (and not to salts of acetic acid), and the term "propionate groups" relates to propionic acid ester groups (-O-C(=O)-CH₂CH₃) (and not to salts of propionic acid). Aroma chemicals (I) containing one or more acetate or propionate groups thus contain one or more acetylated or propionylated hydroxyl groups.

Unless specified otherwise, where the amounts or concentrations of components are given as "ppm", this corresponds to 1 g of component per 1,000,000 g of reference system (or 1 mg/kg). 1 ppm corresponds thus to 0.0001% by weight, relative to the reference system. 1 ppm of acetic acid corresponds thus to 0.0001% by weight, relative to the total weight of the aroma chemical(s) (I) and acetic acid.

Unless stated otherwise or apparent from the context, the remarks made below concerning preferred embodiments of various features, such as the amount of acetic acid, the aroma chemicals containing acetate or propionate groups and the like apply both in context with the uses and methods of the invention as well as with the compositions of the invention.

### Embodiments of the invention

Acetic acid is used in an amount of preferably from 70 to 600 ppm, more preferably from 70 to 550 ppm, even more preferably from 80 to 500 ppm, e.g. from 80 to 150 ppm or from 100 to 550 ppm or from 100 to 500 ppm or from 150 to 500 ppm or from 150 to 400 ppm, or 200 to 300 ppm, relative to the total weight of acetic acid and said aroma chemical(s) (I), for intensifying and/or modifying the aroma, preferably the fragrance, of an aroma chemical (I) which contains one or more acetate or propionate groups or for intensifying and/or modifying the aroma, preferably the fragrance, of a mixture of different aroma chemicals (I).

Analogously, in the composition used as an aroma composition, preferably as a fragrance, and containing acetic acid and at least one aroma chemical (I) which contains one or more acetate or propionate groups, acetic acid is contained in an amount of preferably from 50 to 600 ppm, preferably 70 to 600 ppm, more preferably from 70 to 550 ppm, even more preferably from 80 to 500 ppm, e.g. from 80 to 150 ppm or from 100 to 550 ppm or from 100 to 500 ppm or from 150 to 500 ppm or from 150 to 400 ppm, or 200 to 300 ppm, relative to the total weight of acetic acid and said aroma chemical(s) (I).

As already explained above, the term "acetate groups" relates to acetic acid ester groups (-O-C(=O)-CH₃) (and not to salts of acetic acid), and the term "propionate groups" relates to propionic acid ester groups (-O-C(=O)-CH₂CH₃) (and not to salts of propionic acid). Thus, the aroma chemicals (I) which contain one or more acetate or propionate groups are esters or acetic acid or propionic acid.

Since compositions comprising aroma chemicals (I) which contain one or more acetate groups and acetic acid in the given concentration can be conveniently obtained by adjusting the preparation/purification/isolation process of the respective aroma chemical if this is obtained by acetylating one or more hydroxyl groups of the precursor of said aroma chemical with acetic acid or a suitable derivative thereof, preference is given to aroma chemicals (I) which contain one or more acetate groups. However, the aroma of propionates (I) is also intensified and/or modified by acetic acid.

The aroma chemical (I) which contains one or more acetate or propionate groups is preferably selected from the group consisting of 1,4-butanediol diacetate, 1,4-butanediol monoacetate, 1,4-butanediol dipropionate, 1,4-butanediol monopropionate, neopentylglycol diacetate, neopentylglycol monoacetate, isopentyl acetate, linalyl acetate, 2-tert-butylcyclohexyl acetate, 4-tert-butylcyclohexyl acetate, benzyl acetate, citronellyl acetate, geranyl acetate, neryl acetate, tetrahydrogeranyl acetate, hexyl acetate, 2-octyl acetate, 2-phenylethyl acetate, α-terpinyl acetate, verdyl acetate, bornyl acetate, isobornyl acetate, leaf alcohol acetate (cis-3-hexenyl acetate), and mixtures of two or more of these aroma chemicals.

1,4-Butanediol diacetate is a compound of the formula (I)

R¹-O-(CH₂)₄-O-C(=O)-R² (I)

wherein R¹ is CH₃-C(=O)- and R² is CH₃.
1,4-Butanediol monoacetate is a compound of the formula (I), wherein R¹ is H and R² is CH₃.
1,4-Butanediol dipropionate is a compound of the formula (I), wherein R¹ is CH₃CH₂-C(=O)- and R² is CH₂CH₃.
1,4-Butanediol monopropionate is a compound of the formula (I), wherein R¹ is H and R² is CH₂CH₃.

Neopentylglycol diacetate is a compound of the formula (II)

R¹-O-CH₂-C(CH₃)₂-CH₂-O-C(=O)-R² (II)

wherein R¹ is CH₃-C(=O)- and R² is CH₃.

Neopentylglycol monoacetate is a compound of the formula (II), wherein R¹ is H and R² is CH₃.

Isopentyl acetate (synonymous: isoamyl acetate) is a compound of the formula CH(CH₃)₂-(CH₂)₂-O-C(=O)-CH₃.

Linalyl acetate is a compound of the formula

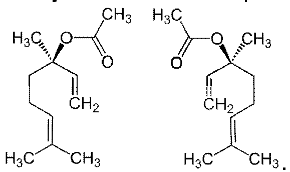

It can be used as the racemate (i.e. the 1:1 mixture of the two enantiomers shown above), as one of the pure enantiomers or as non-racemic mixtures of the enantiomers.

2-tert-Butylcyclohexyl acetate is a compound of the formula

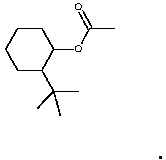

It can be used as a mixture of two or more of the four possible stereoisomers, as one of the pure enantiomers or as one of the diastereomers (Z- or E-isomers) 4-tert-Butylcyclohexyl acetate is a compound of the formula

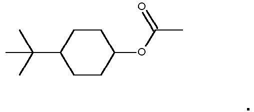

It can be used as a mixture of the possible stereoisomers as one of the pure stereoisomers (Z- or E-isomers).
Benzyl acetate is C₆H₅-CH₂-O-C(=O)-CH₃.
Citronellyl acetate is a compound of the formula

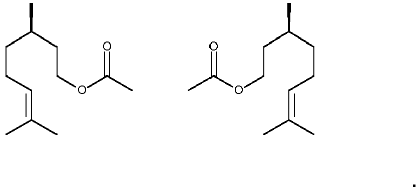

It can be used as the racemate (i.e. the 1:1 mixture of the two enantiomers shown above), as one of the pure enantiomers or as non-racemic mixtures of the enantiomers.

Geranyl acetate is a compound of the formula

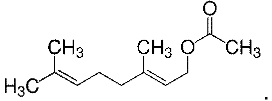

Neryl acetate is a compound of the formula

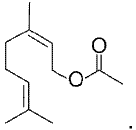

Tetrahydrogeranyl acetate is a compound of the formula CH(CH₃)₂-(CH₂)₃-CH(CH₃)-CH₂CH₂-O-C(=O)-CH₃. It can be used as the racemate, as one of the pure enantiomers or as non-racemic mixtures of the enantiomers.

Hexyl acetate is a compound of the formula CH₃-(CH₂)₅-O-C(=O)-CH₃.

2-Octyl acetate is a compound of the formula CH₃-(CH₂)₅-CH(CH₃)-O-C(=O)-CH₃. It can be used as the racemate, as one of the pure enantiomers or as non-racemic mixtures of the enantiomers.

2-Phenylethyl acetate is a compound of the formula

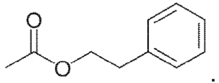

α-Terpinyl acetate is a compound of the formula

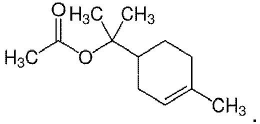

It can be used as the racemate, as one of the pure enantiomers or as non-racemic mixtures of the enantiomers. Commercial α-terpinyl acetate may contain minor amounts of one or more of its other isomers β-, γ- and/or δ-terpinyl acetate. These are generally contained in at most 10% by weight, relative to the total weight of all terpinyl acetate isomers. The term α-terpinyl acetate, as used herein, also encompasses such mixtures of α-terpinyl acetate with minor amounts of one or more of its other isomers β-, γ- and/or δ-terpinyl acetate.

Verdyl acetate (Jasmacyclene^{®}; Cyclacet^{®}) is 3a,4,5,6,7,7a-hexahydro-4,7-methanoinden-6-yl acetate of the formula

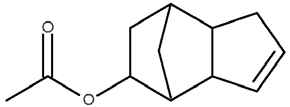

Bornyl acetate is a compound of the formula

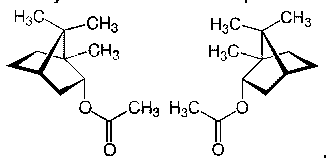

It can be used as the racemate (i.e. the 1:1 mixture of the two enantiomers shown above), as one of the pure enantiomers or as non-racemic mixtures of the enantiomers.

Isobornyl acetate is a compound of the formula

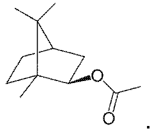

Leaf alcohol acetate (cis-3-hexenyl acetate) is a compound of the formula

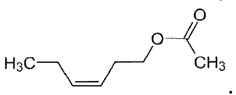

The aroma chemical (I) which contains one or more acetate or propionate groups is more preferably selected from the group consisting of 1,4-butanediol diacetate, 1,4-butanediol monoacetate, neopentylglycol diacetate, neopentylglycol monoacetate, isopentyl acetate, linalyl acetate, 2-tert-butylcyclohexyl acetate, 4-tert-butylcyclohexyl acetate, benzyl acetate, citronellyl acetate, geranyl acetate, neryl acetate, tetrahydrogeranyl acetate, hexyl acetate, 2-octyl acetate, 2-phenylethyl acetate, α-terpinyl acetate, verdyl acetate, bornyl acetate, isobornyl acetate, cis-3-hexenyl acetate, and mixtures of two or more of these aroma chemicals.

The aroma chemical (I) which contains one or more acetate or propionate groups is even more preferably selected from the group consisting of 1,4-butanediol diacetate, mixtures of 1,4-butanediol diacetate and 1,4-butanediol monoacetate, neopentylglycol diacetate, mixtures of neopentylglycol diacetate and neopentylglycol monoacetate, isopentyl acetate, linalyl acetate, 4-tert-butylcyclohexyl acetate, benzyl acetate, citronellyl acetate, geranyl acetate, hexyl acetate, 2-octyl acetate, 2-phenylethyl acetate, α-terpinyl acetate, isobornyl acetate, cis-3-hexenyl acetate, and mixtures of two or more of these aroma chemicals.

In one embodiment, the aroma chemical (I) is not 4-tert-butylcyclohexyl acetate if acetic acid is contained in an amount of less than 100 ppm. In another embodiment, the aroma chemical (I) is not 4-tert-butylcyclohexyl acetate, irrespective of the amount of acetic acid.

The aroma chemical (I) which contains one or more acetate or propionate groups is in particular selected from the group consisting of 1,4-butanediol diacetate, mixtures of 1,4-butanediol diacetate and 1,4-butanediol monoacetate, neopentylglycol diacetate, mixtures of neopentylglycol diacetate and neopentylglycol monoacetate, isopentyl acetate, linalyl acetate, benzyl acetate, citronellyl acetate, α-terpinyl acetate, hexyl acetate, 2-phenylethyl acetate and mixtures of two or more of these aroma chemicals; more particularly from 1,4-butanediol diacetate, neopentylglycol diacetate, isopentyl acetate, linalyl acetate, benzyl acetate, citronellyl acetate, α-terpinyl acetate, hexyl acetate, 2-phenylethyl acetate and mixtures of two or more of these aroma chemicals, and is specifically 1,4-butanediol diacetate.

In an alternatively preferred embodiment, the aroma chemical (I) which contains one or more acetate or propionate groups is preferably selected from the group consisting of 1,4-butanediol diacetate, 1,4-butanediol monoacetate, 1,4-butanediol dipropionate, 1,4-butanediol monopropionate, neopentylglycol diacetate, neopentylglycol monoacetate, isopentyl acetate, citronellyl acetate, α-terpinyl acetate, hexyl acetate, 2-phenylethyl acetate, and mixtures of two or more of these aroma chemicals;
more preferably from 1,4-butanediol diacetate, 1,4-butanediol monoacetate, neopentylglycol diacetate, neopentylglycol monoacetate, isopentyl acetate, citronellyl acetate, α-terpinyl acetate, and mixtures of two or more of these aroma chemicals;
even more preferably from 1,4-butanediol diacetate, mixtures of 1,4-butanediol diacetate and 1,4-butanediol monoacetate, neopentylglycol diacetate, mixtures of neopentylglycol diacetate and neopentylglycol monoacetate, isopentyl acetate, citronellyl acetate, α-terpinyl acetate, and mixtures of two or more of these aroma chemicals;
in particular from 1,4-butanediol diacetate, neopentylglycol diacetate, isopentyl acetate, citronellyl acetate, α-terpinyl acetate and mixtures of two or more of these aroma chemicals;
more particularly from 1,4-butanediol diacetate, neopentylglycol diacetate, and mixtures of two or more of these aroma chemicals, and is specifically 1,4-butanediol diacetate.

In a preferred embodiment, the aroma chemical (I) which contains one or more acetate or propionate groups is selected from the group consisting of 1,4-butanediol diacetate, mixtures of 1,4-butanediol diacetate and 1,4-butanediol monoacetate, isopentyl acetate and mixtures of two or more of these aroma chemicals, and acetic acid is used or contained in an amount of from 70 to 550 ppm, preferably from 80 to 500 ppm, specifically from 80 to 150 ppm, e.g. ca. 100 ppm, relative to the total weight of acetic acid and said aroma chemical(s);
or the aroma chemical (I) which contains one or more acetate or propionate groups is selected from the group consisting of neopentylglycol diacetate, mixtures of neopentylglycol diacetate and neopentylglycol monoacetate, linalyl acetate, benzyl acetate, citronellyl acetate, α-terpinyl acetate and mixtures of two or more of these aroma chemicals, and acetic acid is used or contained in an amount of from 100 to 550 ppm, preferably from 150 to 500 ppm, more preferably from 150 to 400 ppm, in particular from 200 to 300 ppm, e.g. ca. 200 ppm, relative to the total weight of acetic acid and said aroma chemical(s);
or the aroma chemical (I) which contains one or more acetate or propionate groups is selected from the group consisting of hexyl acetate, 2-phenylethyl acetate and mixtures thereof, and acetic acid is used or contained in an amount of from 70 to 550 ppm, preferably from 80 to 500 ppm, more preferably from 80 to 300 ppm, in particular from 100 to 200 ppm, e.g. ca. 200 ppm, relative to the total weight of acetic acid and said aroma chemical(s).

"Circa" (ca.) in this context is intended to include deviations from the exact value caused, for example, by weighing or determination errors. Such errors are in general below 10%, mostly below 5%.

The aroma chemicals (I) which contain one or more acetate or propionate groups are known substances and are either commercially available or can be prepared by standard proceedings, e.g. by esterification of the respective alcohol group(s) with acetic acid or propionic acid or suitable derivatives thereof. The basic alcohols are also known compounds.

Just by way of example, 1,4-butanediol mono- and diacetates or mono- and dipropio-nates can be prepared by standard proceedings, e.g. by esterification of 1,4-butanediol with acetic acid or propionic acid or with more active derivatives thereof, such as the acid halides (e.g. the chloride or bromide), anhydrides or active esters thereof under typical esterification conditions, if desired under removal of the reaction water formed (if the acid as such is used) or of the acid formed (if an anhydride is used) to enhance the reaction rate, or under neutralization (if an acid halide is used). The esterification can be carried out in the presence of an esterification catalyst; this is especially indicated if the carboxylic acid as such is used. Suitable esterification catalysts are well known in the art and are for example metal based catalysts, e.g. iron, cadmium, cobalt, lead, zinc, antimony, magnesium, titanium and tin catalysts in the form of metals, metal oxides or metal salts, such as metal alkoxylates; or acids, e.g. mineral acids, such as sulfuric acid, hydrochloric acid or phosphoric acid; organic sulfonic acids, such as methane sulfonic acid or para-toluene sulfonic acid or strongly acidic cation exchange resins. After completion of the reaction, the esterification product can be isolated by usual means, if necessary after neutralization (especially if an acid halide or anhydride was used as starting material or if the acid was used in excess) and/or removal of the catalyst (especially if this is solid, e.g. one of the above-mentioned metal based catalysts or ionic exchange resins), for example by distillative, extractive or chromatographic methods. If desired, the isolated product can subsequently be further purified. Purification can for example be carried out distillatively. If the acetate has been prepared, distillation can be carried out thus that a composition containing the desired ester and acetic acid in the desired amount is obtained.

Preferably, the composition containing acetic acid in the given concentration and one or more aroma chemical (I) which contain(s) one or more acetate or propionate groups is used for imparting an olfactory impression. In particular, the composition is used as a fragrance.

Specifically, a mixture of 1,4-butanediol diacetate and 50 to 600 ppm or 70 to 600 ppm or 70 to 550 ppm or 80 to 500 ppm or 80 to 150 ppm, e.g. 50 ppm or 100 ppm or 200 ppm or 500 ppm of acetic acid, relative to the total weight of 1,4-butanediol diacetate and acetic acid, is used for imparting one of the following olfactory notes: raspberry, sweet, blueberry; or any combination thereof.

Specifically, a mixture of 1,4-butanediol diacetate, 0.1 to 20% by weight, e.g. 0.1 to 10% by weight or 0.1 to 5% by weight or 0.1 to 2% by weight or 0.1 to 1% by weight or 0.1 to 0.5% by weight 1,4-butanediol monoacetate, relative to the overall weight of 1,4-butanediol diacetate and 1,4-butanediol monoacetate, and 50 to 600 ppm or 70 to 600 ppm or 70 to 550 ppm or 80 to 500 ppm or 80 to 150 ppm, e.g. 50 ppm or 100 ppm or 200 ppm or 500 ppm acetic acid, relative to the total weight of the mixture of 1,4-butanediol diacetate, 1,4-butanediol monoacetate and acetic acid, is used for imparting one of the following olfactory notes: raspberry, sweet, blueberry; or any combination thereof.

Specifically, a mixture of neopentylglycol diacetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of neopentylglycol diacetate and acetic acid, is used for imparting one of the following olfactory notes: sweet, fresh, fruity, resinous, myrrh-like, with notes of fattiness; or any combination thereof.

Specifically, a mixture of isopentyl acetate and 50 to 600 ppm or 70 to 600 ppm or 70 to 550 ppm or 80 to 500 ppm or 80 to 150 ppm, e.g. 50 ppm or 100 ppm or 200 ppm or 500 ppm of acetic acid, relative to the total weight of isopentyl acetate and acetic acid, is used for imparting one of the following olfactory notes: pear, banana; or any combination thereof.

Specifically, a mixture of linalyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of linalyl acetate and acetic acid, is used for imparting one of the following olfactory notes: floral, sweet, citric, with notes of minty and caraway; or any combination thereof.

Specifically, a mixture of benzyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of benzyl acetate and acetic acid, is used for imparting one of the following olfactory notes: sweet, flowery, fresh and slightly fruity, reminiscent of jasmine; or any combination thereof.

Specifically, a mixture of citronellyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of citronellyl acetate and acetic acid, is used for imparting one of the following olfactory notes: floral-rosy, fruity, slightly citrus; or any combination thereof. Specifically, a mixture of α-terpinyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of α-terpinyl acetate and acetic acid, is used for imparting one of the following olfactory notes: herbal, bergamot, lavender, lime, citrus; or any combination thereof. Specifically, a mixture of hexyl acetate and 50 to 600 ppm or 70 to 600 ppm or 70 to 550 ppm or 80 to 500 ppm or 80 to 300 ppm or 100 to 200 ppm, e.g. 50 ppm or 100 ppm or 200 ppm or 500 ppm of acetic acid, relative to the total weight of hexyl acetate and acetic acid, is used for imparting one of the following olfactory notes: fruity, green, apple, banana, sweet ; or any combination thereof.

Specifically, a mixture of 2-phenylethyl acetate and 50 to 600 ppm or 70 to 600 ppm or 70 to 550 ppm or 80 to 500 ppm or 80 to 300 ppm or 100 to 200 ppm, e.g. 50 ppm or 100 ppm or 200 ppm or 500 ppm of acetic acid, relative to the total weight of 2-phenylethyl acetate and acetic acid, is used for imparting one of the following olfactory notes: floral, rose, sweet, honey, fruity, tropical; or any combination thereof.

The composition containing acetic acid in the given concentration and an aroma chemical (I) which contains one or more acetate or propionate groups is generally used in a ready-to-use composition, in particular in a fragranced ready-to-use composition. "Fragranced ready-to-use composition", as used herein, refers to a ready-to-use composition which predominately induces a pleasant odor impression.

The composition containing acetic acid in the given concentration and an aroma chemical (I) which contains one or more acetate or propionate groups is also used for modifying and/or enhancing the aroma of a composition; in particular for modifying and/or enhancing the fragrance impression of a composition; specifically for modifying the scent character of a fragranced ready-to-use composition.

Fragranced ready-to-use compositions are for example compositions used in personal care, in homecare, in industrial applications as well as compositions used in other applications, such as pharmaceutical compositions or crop protection compositions. Among these, preference is given to personal care and homecare compositions.

Personal care compositions encompass body care compositions, products for oral or dental hygiene and hygiene articles for use on the human body.

Personal care compositions are used for cleaning, washing, disinfecting, nurturing, grooming, protecting or embellishing the human body (and thus also include cosmetics). Examples are creams, lotions, ointments, other o/w or w/o emulsions, liquid or gel-like soaps, shower gels, shampoos, make-up and other decorative cosmetics, and compositions for wet wipes (e.g. for cleaning the nappy area). More specific examples are skin-washing and cleansing preparations in the form of soaps, syndets, washing gels, soapless detergents or washing pastes, bath preparations, e.g. foam baths, milks, oils, shower preparations; skin-care preparations, e.g. skin emulsions, multi-emulsions, powders, sprays or skin oils; cosmetic preparations, e.g. facial make-up in the form of day creams or powder creams, face powder (loose or pressed), rouge or cream make-up, eye-care preparations, e.g. eyeshadow preparations, mascara, eyeliner, eye creams or eye-fix creams; lip-care preparations, e.g. lipsticks, lip gloss, lip contour pencils, nail-care preparations, such as nail varnish, nail varnish removers, nail hardeners or cuticle removers; foot-care preparations, e.g. foot baths, foot powders, foot creams or foot balsams, special deodorants and antiperspirants or callus-removing preparations; light-protective preparations, such as sun milks, lotions, creams or oils, sunblocks or tropicals, pre-tanning preparations or after-sun preparations; skin-tanning preparations, e.g. self-tanning creams; depigmenting preparations, e.g. preparations for bleaching the skin or skin-lightening preparations; insect-repellents, e.g. insect-repellent oils, lotions, sprays or sticks; deodorants, such as deodorant sprays, deodorant aerosols, pump-action sprays, deodorant gels, sticks or roll-ons, also water-free deodorant aerosols or sticks; antiperspirants, e.g. antiperspirant sticks, creams or roll-ons; preparations for cleansing and caring for blemished skin, e.g. synthetic detergents (solid or liquid), peeling or scrub preparations or peeling masks; hair-removal preparations in chemical form (depilation), e.g. liquid hair-removing preparations, cream- or paste-form hair-removing preparations, hair-removing preparations in gel form or aerosol foams; shaving preparations, e.g. shaving soap, foaming shaving creams, non-foaming shaving creams, foams and gels, pre-shave preparations for dry shaving, aftershaves or aftershave lotions; fragrance preparations, e.g. fragrances (eau de Cologne, eau de toilette, eau de parfum, parfum de toilette, perfume), perfume oils or perfume creams; cosmetic hair-treatment preparations, e.g. hair-washing preparations in the form of shampoos and conditioners, hair-care preparations, e.g. pre-treatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hairsetting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colorants, preparations containing self-oxidising dyes, or natural hair colorants, such as henna or camomile; antidandruff preparations in the form of shampoos, conditioners, hair tonics, styling creams or gels or treatments packs; oral care preparations such as (tooth) pastes, gels, mouth washes and sprays; disinfectants for mouth or skin. Further examples are given below.

Examples for homecare compositions are dishwashing compositions, laundry compositions (for example laundry detergents, fabric softeners, rinsing compositions, bleacher compositions, stain remover compositions and the like), surface cleaning compositions (also termed hard surface cleaners; for example glass, floor, counter, bath(room), toilet bowl, sink, kitchen, appliance and furniture cleaning compositions; all-purpose cleaners; sanitary cleaners), non-cosmetic deodorants (e.g. air and/or surface deodorants), disinfectants (for example spray air disinfectants, and spray, liquid and paste/gel surface disinfectants), surface protecting and/or polishing compositions, rug shampoos, descaling agents, and compositions for wet wipes (e.g. for cleaning the floor, furniture, bath room surfaces etc.).

Compositions in industrial applications are for example compositions for cleaning or disinfecting on an industrial scale (also called industrial and institutional cleaning or I&I cleaning) or clean-in-place (CIP) composition (CIP is a method of automated cleaning the interior surfaces of pipes, vessels, equipments, filters and associated fittings and the like without major disassembly).

Crop protection compositions, which are often also termed plant protection compositions, are compositions which are effective against various harmful microorganisms, harmful invertebrate pests or undesired plants relevant for agriculture, e.g. harmful fungi, harmful invertebrate pests, such as harmful insects, arachnids, nematodes or molluscs, and weeds, which cause damage to agricultural plants, plant propagation materials, such as seeds, or harvested crops. Examples for crop protection compositions are fungicidal, insecticidal, acaricidal, nematicidal, moluscicidal or herbicidal compositions. The term encompasses also plant growth regulating compositions. Plant growth regulators are plant protection products used to influence plant growth and are used, for example, for increasing the stability of cereals by shortening the stalk length, thus reducing or preventing lodging, for improving the rooting of cuttings, reducing plant height in horticulture, preventing the germination of potatoes and the like. The term encompasses moreover compositions used in material protection for combating various harmful microorganisms and invertebrate pests, such as compositions for the treatment of lumber or the surroundings of lumber material against termites or compositions for the treatment of mosquito nets against harmful insects, such as Anopheles mosquitoes, and the like.

Preferably, the composition containing acetic acid in the given concentration and an aroma chemical (I) which contains one or more acetate or propionate groups is used in a composition selected from the group consisting of perfume compositions, body care compositions (including cosmetic compositions), products for oral and dental hygiene, hygiene articles, cleaning compositions (including dishwashing compositions), textile detergent compositions, compositions for scent dispensers, foods, food supplements, pharmaceutical compositions and crop protection compositions.

Details to the above-listed compositions are given below.

Specifically, a mixture of 1,4-butanediol diacetate and 50 to 600 ppm or 70 to 600 ppm or 70 to 550 ppm or 80 to 500 ppm or 80 to 150 ppm, e.g. 50 ppm or 100 ppm or 200 ppm or 500 ppm of acetic acid, relative to the total weight of 1,4-butanediol diacetate and acetic acid, is used to impart one of the following olfactory notes: raspberry, sweet blueberry; or any combination thereof to the above-listed compositions.

Specifically, a mixture of 1,4-butanediol diacetate, 0.1 to 20% by weight, e.g. 0.1 to 10% by weight or 0.1 to 5% by weight or 0.1 to 2% by weight or 0.1 to 1% by weight or 0.1 to 0.5% by weight 1,4-butanediol monoacetate, relative to the overall weight of 1,4-butanediol diacetate and 1,4-butanediol monoacetate, and 50 to 600 ppm or 70 to 600 ppm or 70 to 550 ppm or 80 to 500 ppm or 80 to 150 ppm, e.g. 50 ppm or 100 ppm or 200 ppm or 500 ppm acetic acid, relative to the total weight of the mixture of 1,4-butanediol diacetate, 1,4-butanediol monoacetate and acetic acid, is used to impart one of the following olfactory notes: raspberry, sweet, blueberry; or any combination thereof to the above-listed compositions.

Specifically, a mixture of neopentylglycol diacetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of neopentylglycol diacetate and acetic acid, is used to impart one of the following olfactory notes: sweet, fresh, fruity, resinous, myrrh-like, with notes of fattiness; or any combination thereof to the above-listed compositions.

Specifically, a mixture of isopentyl acetate and 50 to 600 ppm or 70 to 600 ppm or 70 to 550 ppm or 80 to 500 ppm or 80 to 150 ppm, e.g. 50 ppm or 100 ppm or 200 ppm or 500 ppm of acetic acid, relative to the total weight of isopentyl acetate and acetic acid, is to for impart one of the following olfactory notes: pear, banana; or any combination thereof to the above-listed compositions.

Specifically, a mixture of linalyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of linalyl acetate and acetic acid, is used to impart one of the following olfactory notes: floral, sweet, citric, with notes of minty and caraway; or any combination thereof to the above-listed compositions.

Specifically, a mixture of benzyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of benzyl acetate and acetic acid, is used to impart one of the following olfactory notes: sweet, flowery, fresh and slightly fruity, reminiscent of jasmine; or any combination thereof to the above-listed compositions.

Specifically, a mixture of citronellyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of citronellyl acetate and acetic acid, is used to impart one of the following olfactory notes: floral-rosy, fruity, slightly citrus; or any combination thereof to the above-listed compositions.

Specifically, a mixture of α-terpinyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of α-terpinyl acetate and acetic acid, is used to impart one of the following olfactory notes: herbal, bergamot, lavender, lime, citrus; or any combination thereof to the above-listed compositions.

Specifically, a mixture of hexyl acetate and 50 to 600 ppm or 70 to 600 ppm or 70 to 550 ppm or 80 to 500 ppm or 80 to 300 ppm or 100 to 200 ppm, e.g. 50 ppm or 100 ppm or 200 ppm or 500 ppm of acetic acid, relative to the total weight of hexyl acetate and acetic acid, is used to impart one of the following olfactory notes; fruity, green, apple, banana, sweet; or any combination thereof to the above-listed compositions. Specifically, a mixture of 2-phenylethyl acetate and 50 to 600 ppm or 70 to 600 ppm or 70 to 550 ppm or 80 to 500 ppm or 80 to 300 ppm or 100 to 200 ppm, e.g. 50 ppm or 100 ppm or 200 ppm or 500 ppm of acetic acid, relative to the total weight of 2-phenylethyl acetate and acetic acid, is used to impart one of the following olfactory notes: floral, rose, sweet, honey, fruity, tropical; or any combination thereof to the above-listed compositions.

In addition to the olfactory properties, the combination of acetic acid in the given concentrations and the at least one aroma chemical (I) which contains one or more acetate or propionate groups exhibits advantageous secondary properties.

For example, it can provide better sensory profiles as a result of synergistic effects with other fragrances, which means that it can provide a booster effect for other fragrances. It is therefore suitable as boosters for other fragrances.

Accordingly, another aspect of the invention relates to the use of a combination (generally a mixture) of acetic acid in the given concentration and at least one aroma chemical (I) which contains one or more acetate or propionate groups for modifying the scent character of a fragranced composition; and specifically to the use as a booster for other fragrances.

Booster effect means that the substances enhance and intensify in perfumery formulations the overall impression of the mixture. In the mint range, for example, it is known that menthyl methyl ether intensifies the perfumery or taste mixtures of peppermint oils and particularly in top notes brings about a considerably more intensive and more complex perception although the ether itself, being a pure substance, develops no particular intensive odor at all. In fragrance applications, Hedione^{®} (methyl dihydrojasmonate), which as a pure substance only exhibits a light floral jasmin-note, reinforces diffusion, freshness and volume of a perfume composition as an odor booster. Booster effects are particularly desired when top-note-characterized applications are required, in which the odor impression is to be conveyed particularly quickly and intensively, for example in deodorants, air fresheners or in the taste sector in chewing gums.

To achieve such a booster effect, the composition containing acetic acid in the given concentration and an aroma chemical (I) which contains one or more acetate or propionate groups generally used in an amount of 0.1 to 20% by weight, preferably in an amount of 0.5 to 10% by weight, in particular in an amount of from 0.6 to 5% by weight of the aroma chemical (I) which contains one or more acetate or propionate groups, based on the total weight of the fragrance mixture.

Furthermore, the combination of acetic acid in the given concentration and the at least one aroma chemical (I) which contains one or more acetate or propionate groups can have further positive effects on the composition in which it is used. For example, it can enhance the overall performance of the composition into which it is incorporated, such as the stability, e.g. the formulation stability, the extendability or the staying power of the composition.

The invention relates moreover to a method for intensifying and/or modifying the aroma, preferably the fragrance, of an aroma chemical (I) which contains one or more acetate or propionate groups or of a mixture of two or more different aroma chemicals (I), comprising providing a composition containing said at least one aroma chemical (I) and from 50 to 600 ppm, preferably from 70 to 600 ppm, more preferably from 70 to 550, even more preferably from 80 to 500 ppm, e.g. from 80 to 150 ppm or from 100 to 500 ppm or from 150 to 500 ppm or from 150 to 400 ppm or from 200 to 300 ppm of acetic acid, relative to the total weight of acetic acid and said aroma chemical (I). Suitable and preferred aroma chemicals are those listed above in context with the use according to the invention.

The method preferably comprises adding to said aroma chemical(s) (I) acetic acid in such an amount that a composition comprising said aroma chemical(s) (I) and from 50 to 600 ppm, preferably from 70 to 600 ppm, more preferably from 70 to 550, even more preferably from 80 to 500 ppm, e.g. from 80 to 150 ppm or from 100 to 500 ppm or from 150 to 500 ppm or from 150 to 400 ppm or from 200 to 300 ppm of acetic acid, relative to the total weight of acetic acid and said aroma chemical(s) (I), is obtained; or, in case that the aroma chemical(s) (I) contain(s) one or more acetate groups which are prepared using acetic acid or a derivative thereof, adjusting the reaction, purification and/or isolation conditions in the preparation process of said aroma chemical(s) (I) thus that a composition containing said aroma chemical(s) (I) and from 50 to 600 ppm, preferably from 70 to 600 ppm, more preferably from 70 to 550, even more preferably from 80 to 500 ppm, e.g. from 80 to 150 ppm or from 100 to 500 ppm or from 150 to 500 ppm or from 150 to 400 ppm or from 200 to 300 ppm of acetic acid, relative to the total weight of acetic acid and said aroma chemical(s) (I) is obtained.

Most conveniently, the conditions of the final purification step are adjusted thus that a composition containing said aroma chemical(s) and acetic acid in the desired concentration is obtained.

A further embodiment of the invention is directed to a method of preparing an aroma chemical composition, in particular a fragranced composition, especially a fragranced ready-to-use composition, comprising incorporating at least one aroma chemical (I) which contains one or more acetate or propionate groups and acetic acid in such an amount that it (= acetic acid) is present in said composition in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 500 or from 150 to 400 or from 200 to 300 ppm, relative to the total weight of acetic acid and said aroma chemical (I), into the target composition, e.g. a ready-to-use composition, resulting in an aroma chemical composition, in particular in a fragranced composition, especially in a fragranced ready-to-use composition. Alternatively, the invention is directed to a method of preparing an aroma chemical composition, in particular a fragranced composition, especially a fragranced ready-to-use composition, comprising mixing at least one aroma chemical (I) which contains one or more acetate or propionate groups and acetic acid in such an amount that it (= acetic acid) is present in said composition in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 500 or from 150 to 400 or from 200 to 300 ppm, relative to the total weight of acetic acid and said aroma chemical (I), with at least one aroma chemical different from at least one aroma chemical (I) and/or with at least one non-aroma chemical carrier and/or with at least one antioxidant and/or with at least one deodorant-active agent. Suitable and preferred aroma chemicals different from said aroma chemicals, non-aroma chemical carriers, antioxidants and deodorant-active agents are described below. Alternatively, the invention is directed to a method of preparing an aroma chemical composition, in particular a fragranced composition, especially a fragranced ready-to-use composition, comprising mixing at least one aroma chemical (I) which contains one or more acetate or propionate groups and acetic acid in such an amount that it (= acetic acid) is present in said composition in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 500 or from 150 to 400 or from 200 to 300 ppm, relative to the total weight of acetic acid and said aroma chemical (I), simultaneously or consecutively with the other components of said composition or with pre-formed mixtures of a part of the other components of said composition.

For example, the method can be carried out by mixing at least one aroma chemical (I) which contains one or more acetate or propionate groups, acetic acid in the given amounts and at least one further component selected from the group consisting of aroma chemicals different from compounds (I), non-aroma chemical carriers, antioxidants and deodorant-active agents.

The invention is also directed to a method for modifying the scent character of an aroma chemical composition, in particular of a fragranced composition, especially of a fragranced ready-to-use composition, comprising incorporating at least one aroma chemical (I) which contains one or more acetate or propionate groups and acetic acid in such an amount that it (= acetic acid) is present in said composition in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 500 or from 150 to 400 or from 200 to 300 ppm, relative to the total weight of acetic acid and said aroma chemical (I), into an aroma chemical composition, in particular into a fragranced composition, especially into a fragranced ready-to-use composition. Alternatively, the invention is directed to a method for modifying the scent character of an aroma chemical composition, in particular of a fragranced composition, especially of a fragranced ready-to-use composition, comprising mixing at least one aroma chemical (I) which contains one or more acetate or propionate groups and acetic acid in such an amount that it (= acetic acid) is present in said composition in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 500 or from 150 to 400 or from 200 to 300 ppm, relative to the total weight of acetic acid and said aroma chemical (I), simultaneously or consecutively with the other components of said composition or with pre-formed mixtures of a part of the other components of said composition.

In one embodiment of the methods of the invention, the aroma chemical (I) is not 4-tert-butylcyclohexyl acetate if acetic acid is present in an amount of less than 100 ppm. In a specific embodiment, the aroma chemical (I) is not 4-tert-butylcyclohexyl acetate, irrespective of the amount of acetic acid.

In particular, the invention is directed to a method of preparing a perfume composition, body care composition, product for oral and dental hygiene, hygiene article, cleaning composition, textile detergent composition, composition for scent dispensers, food, food supplement, pharmaceutical composition or crop protection composition, comprising including at least one aroma chemical (I) which contains one or more acetate or propionate groups and acetic acid in such an amount that it (= acetic acid) is present in said composition in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 500 or from 150 to 400 or from 200 to 300 ppm, relative to the total weight of acetic acid and said aroma chemical (I),in a perfume composition, body care composition, product for oral and dental hygiene, hygiene article, cleaning composition, textile detergent composition, composition for scent dispensers, food, food supplement, pharmaceutical composition or crop protection composition. Alternatively, the invention is directed to a method of preparing a perfume composition, body care composition, product for oral and dental hygiene, hygiene article, cleaning composition, textile detergent composition, composition for scent dispensers, food, food supplement, pharmaceutical composition or crop protection composition, comprising mixing at least one aroma chemical (I) which contains one or more acetate or propionate groups and acetic acid in such an amount that it (= acetic acid) is present in said composition in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 500 or from 150 to 400 or from 200 to 300 ppm, relative to the total weight of acetic acid and said aroma chemical (I), simultaneously or consecutively with the other components of said composition or with pre-formed mixtures of a part of the other components of said composition.

In a particular embodiment the invention is directed to a method for imparting one of the olfactory notes: raspberry, sweet, blueberry; or any combination thereof to a perfume composition, body care composition, product for oral and dental hygiene, hygiene article, cleaning composition, textile detergent composition, composition for scent dispensers, food, food supplement, pharmaceutical composition or crop protection composition, which comprises including 1,4-butanediol diacetate and 50 to 600 ppm or 70 to 600 ppm or 70 to 550 ppm or 80 to 500 ppm or 80 to 150 ppm, e.g. 50 ppm or 100 ppm or 200 ppm or 500 ppm of acetic acid, relative to the total weight of 1,4-butanediol diacetate and acetic acid, in a perfume composition, body care composition, product for oral and dental hygiene, hygiene article, cleaning composition, textile detergent composition, composition for scent dispensers, food, food supplement, pharmaceutical composition or crop protection composition.

In another particular embodiment the invention is directed to a method for imparting one of the olfactory notes: raspberry, sweet, blueberry; or any combination thereof to a perfume composition, body care composition, product for oral and dental hygiene, hygiene article, cleaning composition, textile detergent composition, composition for scent dispensers, food, food supplement, pharmaceutical composition or crop protection composition, which comprises including 1,4-butanediol diacetate, 0.1 to 20% by weight, e.g. 0.1 to 10% by weight or 0.1 to 5% by weight or 0.1 to 2% by weight or 0.1 to 1% by weight or 0.1 to 0.5% by weight, of 1,4-butanediol monoacetate, relative to the overall weight of 1,4-butanediol diacetate and 1,4-butanediol monoacetate, and 50 to 600 ppm or 70 to 600 ppm or 70 to 550 ppm or 80 to 500 ppm or 80 to 150 ppm, e.g. 50 ppm or 100 ppm or 200 ppm or 500 ppm acetic acid, relative to the total weight of the mixture of 1,4-butanediol diacetate, 1,4-butanediol monoacetate and acetic acid, in a perfume composition, body care composition, product for oral and dental hygiene, hygiene article, cleaning composition, textile detergent composition, composition for scent dispensers, food, food supplement, pharmaceutical composition or crop protection composition. The fruity impression increases with increasing monoacetate content. The fruity impression increases with increasing monoacetate content.

In another particular embodiment the invention is directed to a method for imparting one of the olfactory notes: pear, banana; or any combination thereof to a perfume composition, body care composition, product for oral and dental hygiene, hygiene article, cleaning composition, textile detergent composition, composition for scent dispensers, food, food supplement, pharmaceutical composition or crop protection composition, which comprises including isopentyl acetate and 50 to 600 ppm or 70 to 600 ppm or 70 to 550 ppm or 80 to 500 ppm or 80 to 150 ppm, e.g. 50 ppm or 100 ppm or 200 ppm or 500 ppm of acetic acid, relative to the total weight of isopentyl acetate and acetic acid, in a perfume composition, body care composition, product for oral and dental hygiene, hygiene article, cleaning composition, textile detergent composition, composition for scent dispensers, food, food supplement, pharmaceutical composition or crop protection composition.

In another particular embodiment the invention is directed to a method for imparting one of the olfactory notes: sweet, fresh, fruity, resinous, myrrh-like, with notes of fattiness; or any combination thereof to a perfume composition, body care composition, product for oral and dental hygiene, hygiene article, cleaning composition, textile detergent composition, composition for scent dispensers, food, food supplement, pharmaceutical composition or crop protection composition, which comprises including neopentylglycol diacetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of neopentylglycol diacetate and acetic acid, in a perfume composition, body care composition, product for oral and dental hygiene, hygiene article, cleaning composition, textile detergent composition, composition for scent dispensers, food, food supplement, pharmaceutical composition or crop protection composition.

In another particular embodiment the invention is directed to a method for imparting one of the olfactory notes: floral, sweet, citric, with notes of minty and caraway; or any combination thereof to a perfume composition, body care composition, product for oral and dental hygiene, hygiene article, cleaning composition, textile detergent composition, composition for scent dispensers, food, food supplement, pharmaceutical composition or crop protection composition, which comprises including linalyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of linalyl acetate and acetic acid, in a perfume composition, body care composition, product for oral and dental hygiene, hygiene article, cleaning composition, textile detergent composition, composition for scent dispensers, food, food supplement, pharmaceutical composition or crop protection composition.

In another particular embodiment the invention is directed to a method for imparting one of the olfactory notes: sweet, flowery, fresh and slightly fruity, reminiscent of jasmine; or any combination thereof to a perfume composition, body care composition, product for oral and dental hygiene, hygiene article, cleaning composition, textile detergent composition, composition for scent dispensers, food, food supplement, pharmaceutical composition or crop protection composition, which comprises including benzyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of benzyl acetate and acetic acid, in a perfume composition, body care composition, product for oral and dental hygiene, hygiene article, cleaning composition, textile detergent composition, composition for scent dispensers, food, food supplement, pharmaceutical composition or crop protection composition.

In another particular embodiment the invention is directed to a method for imparting one of the olfactory notes: floral-rosy, fruity, slightly citrus; or any combination thereof to a perfume composition, body care composition, product for oral and dental hygiene, hygiene article, cleaning composition, textile detergent composition, composition for scent dispensers, food, food supplement, pharmaceutical composition or crop protection composition, which comprises including citronellyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of citronellyl acetate and acetic acid, in a perfume composition, body care composition, product for oral and dental hygiene, hygiene article, cleaning composition, textile detergent composition, composition for scent dispensers, food, food supplement, pharmaceutical composition or crop protection composition.

In another particular embodiment the invention is directed to a method for imparting one of the olfactory notes: herbal, bergamot, lavender, lime, citrus; or any combination thereof to a perfume composition, body care composition, product for oral and dental hygiene, hygiene article, cleaning composition, textile detergent composition, composition for scent dispensers, food, food supplement, pharmaceutical composition or crop protection composition, which comprises including α-terpinyl acetate and 50 to 600 ppm or 70 to 600 ppm or 70 to 550 ppm or 80 to 500 ppm or 150 to 500 ppm or 150 to 400 ppm or 200 to 300 ppm, e.g. 50 ppm or 100 ppm or 200 ppm or 500 ppm of acetic acid, relative to the total weight of α-terpinyl acetate and acetic acid, in a perfume composition, body care composition, product for oral and dental hygiene, hygiene article, cleaning composition, textile detergent composition, composition for scent dispensers, food, food supplement, pharmaceutical composition or crop protection composition.

In another particular embodiment the invention is directed to a method for imparting one of the olfactory notes: fruity, green, apple, banana, sweet; or any combination thereof to a perfume composition, body care composition, product for oral and dental hygiene, hygiene article, cleaning composition, textile detergent composition, composition for scent dispensers, food, food supplement, pharmaceutical composition or crop protection composition, which comprises including hexyl acetate and 50 to 600 ppm or 70 to 600 ppm or 70 to 550 ppm or 80 to 500 ppm or 80 to 300 ppm or 100 to 200 ppm, e.g. 50 ppm or 100 ppm or 200 ppm or 500 ppm of acetic acid, relative to the total weight of hexyl acetate and acetic acid, in a perfume composition, body care composition, product for oral and dental hygiene, hygiene article, cleaning composition, textile detergent composition, composition for scent dispensers, food, food supplement, pharmaceutical composition or crop protection composition.

In another particular embodiment the invention is directed to a method for imparting one of the olfactory notes: floral, rose, sweet, honey, fruity, tropical; or any combination thereof to a perfume composition, body care composition, product for oral and dental hygiene, hygiene article, cleaning composition, textile detergent composition, composition for scent dispensers, food, food supplement, pharmaceutical composition or crop protection composition, which comprises including 2-phenylethyl acetate and 50 to 600 ppm or 70 to 600 ppm or 70 to 550 ppm or 80 to 500 ppm or 80 to 300 ppm or 100 to 200 ppm, e.g. 50 ppm or 100 ppm or 200 ppm or 500 ppm of acetic acid, relative to the total weight of 2-phenylethyl acetate and acetic acid, in a perfume composition, body care composition, product for oral and dental hygiene, hygiene article, cleaning composition, textile detergent composition, composition for scent dispensers, food, food supplement, pharmaceutical composition or crop protection composition.

In a particular embodiment of the methods of the invention, the aroma chemical (I) which contains one or more acetate or propionate groups is selected from the group consisting of 1,4-butanediol diacetate, mixtures of 1,4-butanediol diacetate and 1,4-butanediol monoacetate, isopentyl acetate and mixtures of two or more of these aroma chemicals, and acetic acid is used or contained in an amount of from 70 to 550 ppm, preferably from 80 to 500 ppm, specifically from 80 to 150 ppm, e.g. ca. 100 ppm, relative to the total weight of acetic acid and said aroma chemical(s); or the aroma chemical (I) which contains one or more acetate or propionate groups is selected from the group consisting of neopentylglycol diacetate, mixtures of neopentylglycol diacetate and neopentylglycol monoacetate, linalyl acetate, benzyl acetate, citronellyl acetate, α-terpinyl acetate and mixtures of two or more of these aroma chemicals, and acetic acid is used or contained in an amount of from 100 to 550 ppm, preferably from 150 to 500 ppm, more preferably from 150 to 400 ppm, in particular from 200 to 300 ppm, e.g. ca. 200 ppm, relative to the total weight of acetic acid and said aroma chemical(s); or the aroma chemical (I) which contains one or more acetate or propionate groups is selected from the group consisting of hexyl acetate, 2-phenylethyl acetate and mixtures thereof, and acetic acid is used or contained in an amount of from 70 to 550 ppm, preferably from 80 to 500 ppm, more preferably from 80 to 300 ppm, in particular from 100 to 200 ppm, e.g. ca. 200 ppm, relative to the total weight of acetic acid and said aroma chemical(s).

"ca." in this context is intended to include deviations from the exact value caused, for example, by weighing or determination errors. Such errors are in general below 10%, mostly below 5%.

### Compositions

The invention relates moreover to a composition comprising
- at least one aroma chemical (I) which contains one or more acetate or propionate groups as defined above,
- acetic acid in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s) (I), and
- at least one further component selected from the group consisting of aroma chemicals (II) different from said aroma chemicals which contain one or more acetate or propionate groups, non-aroma chemical carriers, anti-oxidants and deodorant-active agents; and in particular from the group consisting of aroma chemicals different from said aroma chemicals which contain one or more acetate or propionate groups, surfactants, oil components, solvents, anti-oxidants and deodorant-active agents.

Suitable and preferred aroma chemicals (I) which contains one or more acetate or propionate groups are as defined above in context with the use according to the invention.

In one embodiment of the composition of the invention, the aroma chemical (I) is not 4-tert-butylcyclohexyl acetate if acetic acid is contained in an amount of less than 100 ppm. In a specific embodiment, the aroma chemical (I) is not 4-tert-butylcyclohexyl acetate, irrespective of the amount of acetic acid.

The non-aroma chemical carriers, anti-oxidants and deodorant-active agents are of course also different from said aroma chemicals which contain one or more acetate or propionate groups.

The non-aroma chemical carrier is in particular selected from the group consisting of surfactants, oil components (emollients) and solvents.

Thus, in a preferred embodiment, the composition comprises one or more of said aroma chemicals (I) which contain one or more acetate or propionate groups as defined above, acetic acid in the concentrations given above and at least one further component selected from the group consisting of aroma chemicals different from said aroma chemicals which contain one or more acetate or propionate groups, surfactants, oil components, solvents, anti-oxidants and deodorant-active agents.

The composition of the invention is preferably an aroma chemical composition, more preferably an odor composition and in particular a fragrance composition.

One embodiment of the invention is directed to a composition comprising
- at least one aroma chemical (I) which contains one or more acetate or propionate groups as defined above,
- acetic acid in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s) (I), and
- at least one further component selected from the group consisting of oil components, solvents, anti-oxidants and deodorant-active agents.

One embodiment of the invention is directed to a composition comprising
- at least one aroma chemical (I) which contains one or more acetate or propionate groups as defined above,
- acetic acid in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s) (I), and
- at least one aroma chemical (II) different from the above aroma chemical.

One embodiment of the invention is directed to a composition comprising
- at least one aroma chemical (I) which contains one or more acetate or propionate groups as defined above,
- acetic acid in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s) (I), and
- at least one non-aroma chemical carrier.

One embodiment of the invention is directed to a composition comprising
- at least one aroma chemical (I) which contains one or more acetate or propionate groups as defined above,
- acetic acid in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s) (I), and
- at least one surfactant.

One embodiment of the invention is directed to a composition comprising
- at least one aroma chemical (I) which contains one or more acetate or propionate groups as defined above,
- acetic acid in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s) (I), and
- at least one oil component.

One embodiment of the invention is directed to a composition comprising
- at least one aroma chemical (I) which contains one or more acetate or propionate groups as defined above,
- acetic acid in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s) (I), and
- at least one solvent.

One embodiment of the invention is directed to a composition comprising
- at least one aroma chemical (I) which contains one or more acetate or propionate groups as defined above,
- acetic acid in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s) (I), and
- at least one anti-oxidant.

One embodiment of the invention is directed to a composition comprising
- at least one aroma chemical (I) which contains one or more acetate or propionate groups as defined above,
- acetic acid in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s) (I), and
- at least one deodorant-active agent.

The composition according to the invention can be selected from, but is not limited to, the group consisting of perfume compositions, body care compositions (including cosmetic compositions), products for oral and dental hygiene, hygiene articles, cleaning compositions (including dishwashing compositions), textile detergent compositions, compositions for scent dispensers, foods, food supplements, pharmaceutical compositions and crop protection compositions.

### Aroma chemicals (II) (different from aroma chemicals which contain one or more acetate or propionate groups)

By virtue of the physical properties of the compositions containing aroma chemicals (I) which contain one or more acetate or propionate groups and acetic acid in the given concentrations, combinations of said compositions have particularly good, virtually universal solvent properties for and in aroma chemicals (II) and other customary ingredients in aroma compositions such as, in particular, fragrance compositions. Therefore, said compositions are well combinable with aroma chemicals (II) which are different from said aroma chemicals (I) which contain one or more acetate or propionate groups, allowing, in particular, the creation of aroma compositions (preferably fragrance compositions) having novel advantageous sensory profiles. Especially, as already explained above, the combinations can boost the sensory profile of aroma chemicals (II) (such as for example of fragrances) wherein said aroma chemicals (II) are different from said aroma chemicals (I) which contain one or more acetate or propionate groups.

The compositions of the invention can comprise at least one aroma chemical (II) that is different from said aroma chemicals (I) which contain one or more acetate or propionate groups. Said at least one different aroma chemical (II) can for example be 1, 2, 3, 4, 5, 6, 7, 8 or more aroma chemicals, selected from the group consisting of:
Geranyl acetate (3,7-dimethyl-2,6 octadien-1yl acetate), alpha-hexylcinnamaldehyde, 2-phenoxyethyl isobutyrate (Phenirat¹), dihydromyrcenol (2,6-dimethyl-7-octen-2-ol), methyl dihydrojasmonate (preferably with a content of cis isomer of more than 60 wt.%) (Hedione⁹, Hedione HC⁹), 4,6,6,7,8,8-hexamethyl-1,3,4,6,7,8-hexahydro-cyclopenta[g]benzopyran (Galaxolid³), tetrahydrolinalool (3,7-dimethyloctan-3-ol), ethyllinalool, benzyl salicylate, 2-methyl-3-(4-tert-butylphenyl)propanal (Lysmeral²), cinnamyl alcohol, 4,7-methano-3a,4,5,6,7,7a-hexahydro-5-indenyl acetate and/or 4,7-methano-3a,4,5,6,7,7a-hexahydro-6-indenyl acetate (Herbaflorat¹), citronellol, citronellyl acetate, tetrahydrogeraniol, vanillin, linalyl acetate, styrolyl acetate (1-phenylethyl acetate), octahydro-2,3,8,8-tetramethyl-2-acetonaphthone and/or 2-acetyl-1,2,3,4,6,7,8-octahydro-2,3,8,8-tetramethylnaphthalene (Iso E Super³), hexyl salicylate, 4-tert-butylcyclohexyl acetate (Oryclone¹), 2-tert-butylcyclohexyl acetate (Agrumex HC¹), alpha-ionone (4-(2,2,6-trimethyl-2-cyclohexen-1-yl)-3-buten-2-one), n-alpha-methylionone, alpha-isomethylionone, coumarin, terpinyl acetate, 2-phenylethyl alcohol, 4-(4-hydroxy-4-methylpentyl)-3-cyclohexenecarboxaldehyde (Lyral³), alpha-amylcinnamaldehyde, ethylene brassylate, I- and/or (Z)-3-methyl-cyclopentadec-5-enone (Muscenon⁹), 15-pentadec-11-enolide and/or 15-pentadec-12-enolide (Globalide¹), 15-cyclopentadecanolide (Macrolide¹), 1-(5,6,7,8-tetrahydro-3,5,5,6,8,8-hexamethyl-2-naphthalenyl)ethanone (Tonalid¹⁰), 2-isobutyl-4-methyl-tetrahydro-2H-pyran-4-ol (Florol⁹), 2-ethyl-4-(2,2,3-trimethyl-3-cyclopenten-1-yl)-2-buten-1-ol (Sandolen¹), cis-3-hexenyl acetate, trans-3-hexenyl acetate, trans-2/cis-6-nonadienol, 2,4-dimethyl-3-cyclohexenecarboxaldehyde (Vertocitral¹), 2,4,4,7-tetra-methyloct-6-en-3-one (Claritone¹), 2,6-dimethyl-5-hepten-1-al (Melonal²), borneol, 3-(3-isopropylphenyl)butanal (Florhydral²), 2-methyl-3-(3,4-methylenedioxyphenyl)-propanal (Helional³), 3-(4-ethylphenyl)-2,2-dimethylpropanal (Florazon¹), 7-methyl-2H-1,5-benzodioxepin-3(4H)-one (Calone), 3,3,5-trimethylcyclohexyl acetate (preferably with a content of cis isomers of 70 wt.%) or more, 2,5,5-trimethyl-1,2,3,4,4a,5,6,7-octahydronaphthalen-2-ol (Ambrinol S¹), 3-(4-*tert*-butylphenyl)-propanal (Bourgeonal⁴), ethyl 2-methylpentanoate (Manzanate⁴), ethoxymethoxycyclododecane (Amberwood¹), 2,4-dimethyl-4,4a,5,9b-tetrahydroindeno[1,2-d][1,3]dioxine (Magnolan'), (2-tert-butylcyclohexyl) acetate (Verdox³) and 3-[5,5,6-trimethylbicyclo[2.2.1]hept-2-yl]cyclohexan-1-ol (Sandela⁴). Within the context of the present invention, the aforementioned aroma chemical(s) are accordingly preferably combined with a compound of formula (I), or a mixture of different compounds (I), as described above.

Where trade names are given above, these refer to the following sources:
¹ trade name of Symrise GmbH, Germany;
² trade name of BASF SE;
³ trade name of International Flavors & Fragrances Inc., USA;
⁴ Givaudan AG, Switzerland;
⁹ trade name of Firmenich S.A., Switzerland;
¹⁰ trade name of PFW Aroma Chemicals B.V., the Netherlands.

A preferred embodiment of the invention relates to a composition comprising at least one aroma chemical (I) which contains one or more acetate or propionate groups, acetic acid in the given concentrations and at least one aroma chemical (II) selected from the group consisting of methyl benzoate, benzyl acetate, geranyl acetate, 2-isobutyl-4-methyltetrahydro-2H-pyran-4-ol and linalool.

A further embodiment of the invention relates to a composition at least one aroma chemical (I) which contains one or more acetate or propionate groups, acetic acid in the given concentrations and 2-isobutyl-4-methyltetrahydro-2H-pyran-4-ol.

A further embodiment of the invention relates to a composition comprising at least one aroma chemical (I) which contains one or more acetate or propionate groups, acetic acid in the given concentrations, and methyl benzoate.

A preferred embodiment of the invention relates to a composition comprising at least one aroma chemical (I) which contains one or more acetate or propionate groups, acetic acid in the given concentrations, and at least one further aroma chemical (II) selected from the group consisting of ethylvanillin, vanillin, 2,5-dimethyl-4-hydroxy-2H-furan-3-one (furaneol) or 3-hydroxy-2-methyl-4H-pyran-4-one (maltol).

A preferred embodiment of the invention relates to a composition comprising at least one aroma chemical (I) which contains one or more acetate or propionate groups, acetic acid in the given concentrations, and at least one further aroma chemical (II) selected from the group consisting of geranyl acetate, alpha-hexylcinnamaldehyde, 2-phenoxyethyl isobutyrate, dihydromyrcenol, methyl dihydrojasmonate (preferably with a content of cis isomer of more than 60 wt.%), 4,6,6,7,8,8-hexamethyl-1,3,4,6,7,8-hexahydrocyclopenta[g]benzopyran, tetrahydrolinalool, ethyllinalool, benzyl salicylate, 2-methyl-3-(4-tert-butylphenyl)propanal, cinnamyl alcohol, 4,7-methano-3a,4,5,6,7,7a-hexahydro-5-indenyl acetate, 4,7-methano-3a,4,5,6,7,7a-hexahydro-6-indenyl acetate, citronellyl acetate, tetrahydrogeraniol, linalyl acetate, styrolyl acetate, octahydro-2,3,8,8-tetramethyl-2-acetonaphthone, 2-acetyl-1,2,3,4,6,7,8-octahydro-2,3,8,8-tetramethylnaphthalene, hexyl salicylate, 4-tert-butylcyclohexyl acetate, 2-tert-butylcyclohexyl acetate, alpha-ionone, n-alpha-methylionone, alpha-isomethylionone, coumarin, terpinyl acetate, 4-(4-hydroxy-4-methylpentyl)-3-cyclohexenecarboxaldehyde, alpha-amylcinnamaldehyde, ethylene brassylate, (E)-3-methylcyclopentadec-5-enone, (Z)-3-methylcyclopentadec-5-enone, 15-pentadec-11-enolide, 15-pentadec-12-enolide, 15-cyclopentadecanolide, 1-(5,6,7,8-tetrahydro-3,5,5,6,8,8-hexamethyl-2-naphthalenyl)ethanone, 2-isobutyl-4-methyltetrahydro-2H-pyran-4-ol, 2-ethyl-4-(2,2,3-trimethyl-3-cyclopenten-1-yl)-2-buten-1-ol, cis-3-hexenyl acetate, trans-3-hexenyl acetate, trans-2/cis-6-nonadienol, 2,4-dimethyl-3-cyclohexenecarboxaldehyde, 2,4,4,7-tetramethyloct-6-en-3-one, 2,6-dimethyl-5-hepten-1-al, borneol, 3-(3-isopropylphenyl)butanal, 2-methyl-3-(3,4-methylenedioxyphenyl)propanal, 3-(4-ethylphenyl)-2,2-dimethylpropanal, 7-methyl-2H-1,5-benzodioxepin-3(4H)-one, 3,3,5-trimethylcyclohexyl acetate (preferably with a content of cis isomers of 70 wt.% or more), 2,5,5-trimethyl-1,2,3,4,4a,5,6,7-octahydronaphthalen-2-ol, 3-(4-*tert*-butylphenyl)-propanal, ethyl 2-methylpentanoate, ethoxymethoxycyclododecane, 2,4-dimethyl-4,4a,5,9b-tetrahydroindeno[1,2-d][1,3]dioxine, (2-tert-butylcyclohexyl) acetate, 3-[5,5,6-trimethylbicyclo[2.2.1]hept-2-yl]cyclohexan-1-ol, menthone, isomenthone, carvone, beta-n-methylionone, beta-isomethylionone, alpha-irone, alpha-damascone, beta-damascone, delta-damascone, acetylated cedar wood oil, and mixtures thereof.

Further aroma chemicals (II) with which the at least one aroma chemical (I) which contains one or more acetate or propionate groups and acetic acid in the given concentrations can be combined to give a composition according to the invention can be found, e.g., in S. Arctander, Perfume and Flavor Chemicals, Vol. I and II, Montclair, N. J., 1969, self-published or K. Bauer, D. Garbe and H. Surburg, Common Fragrance and Flavor Materials, 4th Ed., Wiley- VCH, Weinheim 2001. Specifically, mention may be made of:
extracts from natural raw materials such as essential oils, concretes, absolutes, resins, resinoids, balsams, tinctures such as e.g.
ambergris tincture; amyris oil; angelica seed oil; angelica root oil; aniseed oil; valerian oil; basil oil; tree moss absolute; bay oil; mugwort oil; benzoin resin; bergamot oil; beeswax absolute; birch tar oil; bitter almond oil; savory oil; buchu leaf oil; cabreuva oil; cade oil; calmus oil; camphor oil; cananga oil; cardamom oil; cascarilla oil; cassia oil; cassia absolute; castoreum absolute; cedar leaf oil; cedar wood oil; cistus oil; citronella oil; lemon oil; copaiba balsam; copaiba balsam oil; coriander oil; costus root oil; cumin oil; cypress oil; davana oil; dill weed oil; dill seed oil; Eau de brouts absolute; oak moss absolute; elemi oil; tarragon oil; eucalyptus citriodora oil; eucalyptus oil; fennel oil; pine needle oil; galbanum oil; galbanum resin; geranium oil; grapefruit oil; guaiacwood oil; gurjun balsam; gurjun balsam oil; helichrysum absolute; helichrysum oil; ginger oil; iris root absolute; iris root oil; jasmine absolute; calmus oil; camomile oil blue; roman camomile oil; carrot seed oil; cascarilla oil; pine needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil pressed; linalool oil; litsea cubeba oil; laurel leaf oil; mace oil; marjoram oil; mandarin oil; mas-soia bark oil; mimosa absolute; musk seed oil; musk tincture; clary sage oil; nutmeg oil; myrrh absolute; myrrh oil; myrtle oil; clove leaf oil; clove flower oil; neroli oil; olibanum absolute; olibanum oil; opopanax oil; orange blossom absolute; orange oil; origanum oil; palmarosa oil; patchouli oil; perilla oil; peru balsam oil; parsley leaf oil; parsley seed oil; petitgrain oil; peppermint oil; pepper oil; pimento oil; pine oil; pennyroyal oil; rose absolute; rose wood oil; rose oil; rosemary oil; Dalmatian sage oil; Spanish sage oil; sandalwood oil; celery seed oil; spike-lavender oil; star anise oil; styrax oil; tagetes oil; fir needle oil; tea tree oil; turpentine oil; thyme oil; tolubalsam; tonka absolute; tuberose absolute; vanilla extract; violet leaf absolute; verbena oil; vetiver oil; juniper berry oil; wine lees oil; wormwood oil; winter green oil; hyssop oil; civet absolute; cinnamon leaf oil; cinnamon bark oil, and fractions thereof, or ingredients isolated therefrom;
individual fragrances from the group of hydrocarbons, such as e.g. 3-carene; alpha-pinene; beta-pinene; alpha-terpinene; gamma-terpinene; p-cymene; bisabolene; camphene; caryophyllene; cedrene; farnesene; limonene; longifolene; myrcene; ocimene; valencene; (E,Z)-1,3,5-undecatriene; styrene; diphenylmethane;
the aliphatic alcohols such as e.g. hexanol; octanol; 3-octanol; 2,6-dimethylheptanol; 2-methyl-2-heptanol; 2-methyl-2-octanol; (E)-2-hexenol; (E)- and (Z)-3-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
the aliphatic aldehydes and acetals thereof such as e.g. hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-9-undecenal; 2,6,10-trimethyl-5,9-undecadienal; heptanal diethylacetal; 1,1-dimethoxy-2,2,5-trimethyl-4-hexene; citronellyloxyacetaldehyde; (E/Z)-1-(1-methoxypropoxy)-hex-3-ene; the aliphatic ketones and oximes thereof such as e.g. 2-heptanone; 2-octanone; 3-octanone; 2-nonanone; 5-methyl-3-heptanone; 5-methyl-3-heptanone oxime; 2,4,4,7-tetramethyl-6-octen-3-one; 6-methyl-5-hepten-2-one;
the aliphatic sulfur-containing compounds such as e.g. 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
the aliphatic nitriles such as e.g. 2-nonenenitrile; 2-undecenenitrile; 2-tridecenenitrile; 3,12-tridecadienenitrile; 3,7-dimethyl-2,6-octadienenitrile; 3,7-dimethyl-6-octenenitrile;
the esters of aliphatic carboxylic acids such as e.g. (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octinate; methyl 2-noninate; allyl 2-isoamyloxy acetate; methyl-3,7-dimethyl-2,6-octadienoate; 4-methyl-2-pentyl crotonate;
the acyclic terpene alcohols such as e.g. geraniol; nerol; linalool; lavandulol; nerolidol; farnesol; tetrahydrolinalool; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-1-ol; and the formates, acetates, propionates, isobutyrates, butyrates, isovalerates, pentanoates, hexanoates, crotonates, tiglinates and 3-methyl-2-butenoates thereof;
the acyclic terpene aldehydes and ketones such as e.g. geranial; neral; citronellal; 7-hydroxy-3,7-dimethyloctanal; 7-methoxy-3,7-dimethyloctanal; 2,6,10-trimethyl-9-undecenal; geranyl acetone; as well as the dimethyl- and diethylacetals of geranial, neral, 7-hydroxy-3,7-dimethyloctanal; the cyclic terpene alcohols such as e.g. menthol; isopulegol; alpha-terpineol; terpine-4-ol; menthan-8-ol; menthan-1-ol; menthan-7-ol; borneol; isoborneol; linalool oxide; nopol; cedrol; ambrinol; vetiverol; guajol; and the formates, acetates, propionates, isobutyrates, butyrates, isovalerates, pentanoates, hexanoates, crotonates, tiglinates and 3-methyl-2-butenoates thereof;
the cyclic terpene aldehydes and ketones such as e.g. menthone; isomenthone; 8-mercaptomenthan-3-one; carvone; camphor; fenchone; alpha-ionone; beta-ionone; alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methano-naphthalene-8(5H)-one; 2-methyl-4-(2,6,6-trimethyl-1-cyclohexen-1-yl)-2-butenal; noot-katone; dihydronootkatone; 4,6,8-megastigmatrien-3-one; alpha-sinensal; beta-sinensal; acetylated cedar wood oil (methyl cedryl ketone);
the cyclic alcohols such as e.g. 4-tert-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
the cycloaliphatic alcohols such as e.g. alpha-3,3-trimethylcyclohexylmethanol; 1-(4-isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 2-ethyl-4-(2,2,3-trimethyl-3-cyclopent-1-yl)-2-buten-1-ol; 3-methyl-5-(2,2,3-trimethyl-3-cyclopent-1-yl)pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
the cyclic and cycloaliphatic ethers such as e.g. cineol; cedryl methyl ether; cyclododecyl methyl ether; 1,1-dimethoxycyclododecane; (ethoxymethoxy)cyclododecane; alpha-cedrene epoxide; 3a,6,6,9a-tetramethyldodecahydronaphtho[2,1-b]furan; 3a-ethyl-6,6,9a-trimethyldodecahydronaphtho[2,1-b]furan; 1,5,9-trimethyl-13-oxabicyclo-[10.1.0]trideca-4,8-diene; rose oxide; 2-(2,4-dimethyl-3-cyclohexen-1-yl)-5-methyl-5-(1-methylpropyl)-1,3-dioxane;
the cyclic and macrocyclic ketones such as e.g. 4-tert-butylcyclohexanone; 2,2,5-trimethyl-5-pentylcyclopentanone; 2-heptylcyclopentanone; 2-pentylcyclo-pentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopenta-decenone; 3-methyl-5-cyclopentadecenone; 3-methylcyclopentadecanone; 4-(1-ethoxyvinyl)-3,3,5,5-tetramethylcyclohexanone; 4-tert-pentylcyclohexanone; 5-cyclohexadecen-1-one; 6,7-dihydro-1,1,2,3,3-pentamethyl-4(5H)-indanone; 8-cyclo-hexadecen-1-one; 7-cyclohexadecen-1-one; (7/8)-cyclohexadecen-1-one; 9-cyclo-heptadecen-1-one; cyclopentadecanone; cyclohexadecanone;
the cycloaliphatic aldehydes such as e.g. 2,4-dimethyl-3-cyclohexenecarbaldehyde; 2-methyl-4-(2,2,6-trimethylcyclohexen-1-yl)-2-butenal; 4-(4-hydroxy-4-methylpentyl)-3-cyclohexene carbaldehyde; 4-(4-methyl-3-penten-1-yl)-3-cyclohexenecarbaldehyde;
the cycloaliphatic ketones such as e.g. 1-(3,3-dimethylcyclohexyl)-4-penten-1-one; 2,2-dimethyl-1-(2,4-dimethyl-3-cyclohexen-1-yl)-1-propanone; 1-(5,5-dimethyl-1-cyclohexen-1-yl)-4-penten-1-one; 2,3,8,8-tetramethyl-1,2,3,4,5,6,7,8-octahydro-2-naphthalenyl methyl ketone; methyl 2,6,10-trimethyl-2,5,9-cyclododecatrienyl ketone; tert-butyl (2,4-dimethyl-3-cyclohexen-1-yl) ketone;
the esters of cyclic alcohols such as e.g. 2-tert-butylcyclohexyl acetate; 4-tert-butylcyclohexyl acetate; 2-tert-pentylcyclohexyl acetate; 4-tert-pentylcyclohexyl acetate; 3,3,5-trimethylcyclohexyl acetate; decahydro-2-naphthyl acetate; 2-cyclopentylcyclopentyl crotonate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5 or 6-indenyl acetate; 4,7-methano-3a,4,5,6,7,7a-hexahydro-5 or 6-indenyl propionate; 4,7-methano-3a,4,5,6,7,7a-hexahydro-5 or 6-indenyl isobutyrate; 4,7-methanooctahydro-5 or 6-indenyl acetate;
the esters of cycloaliphatic alcohols such as e.g. 1-cyclohexylethyl crotonate;
the esters of cycloaliphatic carboxylic acids such as e.g. allyl 3-cyclohexylpropionate; allyl cyclohexyloxyacetate; cis- and trans-methyl dihydrojasmonate; cis- and trans-methyl jasmonate; methyl 2-hexyl-3-oxocyclopentanecarboxylate; ethyl 2-ethyl-6,6-dimethyl-2-cyclohexenecarboxylate; ethyl 2,3,6,6-tetramethyl-2-cyclohexenecarboxylate; ethyl 2-methyl-1,3-dioxolane-2-acetate;
the araliphatic alcohols such as e.g. benzyl alcohol; 1-phenylethyl alcohol, 2-phenylethyl alcohol, 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol;
the esters of araliphatic alcohols and aliphatic carboxylic acids such as e.g. benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; alpha-trichloromethylbenzyl acetate; alpha,alpha-dimethyl-phenylethyl acetate; alpha,alpha-dimethylphenylethyl butyrate; cinnamyl acetate; 2-phenoxyethyl isobutyrate; 4-methoxybenzyl acetate;
the araliphatic ethers such as e.g. 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxine; 4,4a,5,9b-tetrahydro-2,4-dimethylindeno[1,2-d]-m-dioxine;
the aromatic and araliphatic aldehydes such as e.g. benzaldehyde; phenylacetaldehyde; 3-phenylpropanal; hydratropaaldehyde; 4-methylbenzaldehyde; 4-methylphenylacetaldehyde; 3-(4-ethylphenyl)-2,2-dimethylpropanal; 2-methyl-3-(4-isopropylphenyl)propanal; 2-methyl-3-(4-tert-butylphenyl)propanal; 2-methyl-3-(4-isobutylphenyl)propanal; 3-(4-tert-butylphenyl)propanal; cinnamaldehyde; alpha-butylcinnamaldehyde; alpha-amylcinnamaldehyde; alpha-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
the aromatic and araliphatic ketones such as e.g. acetophenone; 4-methylacetophenone; 4-methoxyacetophenone; 4-tert-butyl-2,6-dimethylaceto-phenone; 4-phenyl-2-butanone; 4-(4-hydroxyphenyl)-2-butanone; 1-(2-naphthalenyl)-ethanone; 2-benzofuranylethanone; (3-methyl-2-benzofuranyl)ethanone; benzophenone; 1,1,2,3,3,6-hexamethyl-5-indanyl methyl ketone; 6-tert-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;
the aromatic and araliphatic carboxylic acids and esters thereof such as e.g. benzoic acid; phenylacetic acid; methyl benzoate; ethyl benzoate; hexyl benzoate; benzyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycidate; ethyl 3-methyl-3-phenylglycidate;
the nitrogen-containing aromatic compounds such as e.g. 2,4,6-trinitro-1,3-dimethyl-5-tert-butylbenzene; 3,5-dinitro-2,6-dimethyl-4-tert-butylacetophenone; cinnamonitrile; 3-methyl-5-phenyl-2-pentenonitrile; 3-methyl-5-phenylpentanonitrile; methyl anthranilate; methyl-N-methylanthranilate; Schiff bases of methyl anthranilate with 7-hydroxy-3,7-dimethyloctanal, 2-methyl-3-(4-tert-butylphenyl)propanal or 2,4-dimethyl-3-cyclohexenecarbaldehyde; 6-isopropylquinoline; 6-isobutylquinoline; 6-sec-butylquinoline; 2-(3-phenylpropyl)pyridine; indole; skatole; 2-methoxy-3-isopropyl-pyrazine; 2-isobutyl-3-methoxypyrazine;
the phenols, phenyl ethers and phenyl esters such as e.g. estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; p-cresyl phenylacetate;
the heterocyclic compounds such as e.g. 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;
the lactones such as e.g. 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 4-methyl-1,4-decanolide; 1,15-pentadecanolide; cis- and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin.

Preferably, the aroma chemicals different from said aroma chemicals which contain one or more acetate or propionate groups are selected from the group consisting of geranyl acetate, alpha-hexylcinnamaldehyde, 2-phenoxyethyl isobutyrate, dihydromyrcenol, methyl dihydrojasmonate (preferably with a content of cis isomer of more than 60 wt.%), 4,6,6,7,8,8-hexamethyl-1,3,4,6,7,8-hexahydrocyclopenta[g]benzopyran, tetrahydrolinalool, ethyllinalool, benzyl salicylate, 2-methyl-3-(4-tert-butylphenyl)propanal, cinnamyl alcohol, 4,7-methano-3a,4,5,6,7,7a-hexahydro-5-indenyl acetate, 4,7-methano-3a,4,5,6,7,7a-hexahydro-6-indenyl acetate, citronellol, citronellyl acetate, tetrahydrogeraniol, vanillin, linalyl acetate, styrolyl acetate, octahydro-2,3,8,8-tetramethyl-2-acetonaphthone, 2-acetyl-1,2,3,4,6,7,8-octahydro-2,3,8,8-tetramethyl-naphthalene, hexyl salicylate, 4-tert-butylcyclohexyl acetate, 2-tert-butylcyclohexyl acetate, alpha-ionone, n-alpha-methylionone, alpha-isomethylionone, coumarin, terpinyl acetate, 2-phenylethyl alcohol, 4-(4-hydroxy-4-methylpentyl)-3-cyclohexenecarboxaldehyde, alpha-amylcinnamaldehyde, ethylene brassylate, (E)-3-methylcyclopentadec-5-enone, (Z)-3-methylcyclopentadec-5-enone, 15-pentadec-11-enolide, 15-pentadec-12-enolide, 15-cyclopentadecanolide, 1-(5,6,7,8-tetrahydro-3,5,5,6,8,8-hexamethyl-2-naphthalenyl)ethanone, 2-isobutyl-4-methyltetrahydro-2H-pyran-4-ol, 2-ethyl-4-(2,2,3-trimethyl-3-cyclopenten-1-yl)-2-buten-1-ol, cis-3-hexenyl acetate, trans-3-hexenyl acetate, trans-2/cis-6-nonadienol, 2,4-dimethyl-3-cyclohexenecarboxaldehyde, 2,4,4,7-tetramethyloct-6-en-3-one, 2,6-dimethyl-5-hepten-1-al, borneol, 3-(3-isopropylphenyl)butanal, 2-methyl-3-(3,4-methylenedioxyphenyl)propanal, 3-(4-ethylphenyl)-2,2-dimethylpropanal, 7-methyl-2H-1,5-benzodioxepin-3(4H)-one, 3,3,5-trimethylcyclohexyl acetate (preferably with a content of cis isomers of 70 wt.% or more), 2,5,5-trimethyl-1,2,3,4,4a,5,6,7-octahydronaphthalen-2-ol, 3-(4-*tert*-butylphenyl)-propanal, ethyl 2-methylpentanoate, ethoxymethoxycyclododecane, 2,4-dimethyl-4,4a,5,9b-tetrahydroindeno[1,2-d][1,3]dioxine, (2-tert-butylcyclohexyl) acetate, 3-[5,5,6-trimethylbicyclo[2.2.1]hept-2-yl]cyclohexan-1-ol, menthone, isomenthone, carvone, camphor, beta-ionone, beta-n-methylionone, beta-isomethylionone, alpha-irone, alpha-damascone, beta-damascone, beta-damascenone, delta-damascone, acetylated cedar wood oil, and mixtures thereof.

### Non-aroma chemical carriers

A further embodiment of the invention is directed to a composition comprising at least one aroma chemical (I) which contains one or more acetate or propionate groups as defined above, acetic acid in the stated amount, and at least one non-aroma chemical carrier.

The at least one non-aroma chemical carrier can be a compound, a mixture of compounds or other additives, which has/have no or no noteworthy sensory properties. The non-aroma chemical carrier can serve for the dilution and/or the fixing of the aroma chemical (I) which contains one or more acetate or propionate groups, acetic acid and optionally the at least one aroma chemical different therefrom, as defined above, or any other component, if comprised in the composition.

A further embodiment of the invention is directed to a composition comprising at least one aroma chemical (I) which contains one or more acetate or propionate groups as defined above, acetic acid in the stated amounts, and at least one non-aroma chemical carrier selected from the group consisting of solvents, surfactants and oil components.

According to preferred embodiments of the present invention, said non-aroma chemical carrier(s) is/are selected from the solvents, surfactants and oil components listed below.

One embodiment of the invention is directed to a composition comprising at least one aroma chemical (I) which contains one or more acetate or propionate groups as defined above, acetic acid in the stated amounts and at least one solvent.

In the context of the present invention, a "solvent" serves for the dilution of the aroma chemical(s) (I) to be used according to the invention and/or any further component of the composition without having its own aroma.

The one or more solvent(s) can be present in the composition in amount of 0.01 to 99 wt.% based on the composition. In a preferred embodiment of the invention, the composition comprises 0.1 to 90 wt.%, preferably 0.5 to 80 wt.% of solvent(s) based on the total weight of the composition. The amount of solvent(s) can be chosen depending on the composition. In one embodiment of the invention, the composition comprises 0.05 to 10 wt.%, preferably 0.1 to 5 wt.%, more preferably 0.2 to 3 wt.% based on the total weight of the composition. In one embodiment of the invention, the composition comprises 20 to 70 wt.%, preferably 25 to 50 wt.% of solvent(s) based on the total weight of the composition.

Preferred solvents are ethanol, isopropanol, dipropylene glycol (DPG), propylene glycol, 1,2-butylene glycol, glycerol, diethylene glycol monoethyl ether, diethyl phthalate (DEP), isopropyl myristate (IPM), triethyl citrate (TEC), and benzyl benzoate (BB).

Especially preferred solvents are selected from the group consisting of ethanol, propylene glycol, dipropylene glycol, triethyl citrate, benzyl benzoate and isopropyl myristate.

In a preferred embodiment of the invention, the solvent is selected from the group consisting of ethanol, isopropanol, diethylene glycol monoethyl ether, glycerol, propylene glycol, 1,2-butylene glycol, dipropylene glycol, triethyl citrate and isopropyl myristate.

In a particular embodiment of the invention, the solvent is selected from the group consisting of isopropanol, dipropylene glycol (DPG), propylene glycol, 1,2-butylene glycol, diethylene glycol monoethyl ether, diethyl phthalate (DEP), isopropyl myristate (IPM), triethyl citrate (TEC), benzyl benzoate and mixtures thereof. More particularly, the solvent is selected from the group consisting of isopropanol, isopropyl myristate (IPM), triethyl citrate (TEC), benzyl benzoate and mixtures thereof.

Very typically, the solvent is ethanol or triethyl citrate.

According to a further embodiment, the at least one aroma chemical (I) in combination with acetic acid in the given amounts is used according to the present invention in surfactant-containing compositions. Due to its characteristic fragrance property and its substantivity, tenacity as well as stability, the combination of the at least one aroma chemical (I) and acetic acid in the stated amounts can especially be used to provide an odor, preferably a fragrance impression to surfactant-containing compositions such as, for example, cleaners (in particular laundry care products and all-purpose cleaners). It can preferably be used to impart a long-lasting clean note to a surfactant comprising composition.

One embodiment of the invention is therefore directed to a composition at least one aroma chemical (I) which contains one or more acetate or propionate groups as defined above, acetic acid in the stated amounts, and at least one surfactant.

The surfactant(s) may be selected from anionic, non-ionic, cationic and/or amphoteric or zwitterionic surfactants. Surfactant-containing compositions, such as for example shower gels, foam baths, shampoos, etc., preferably contain at least one anionic surfactant.

The compositions according to the invention usually contain the surfactant(s), in the aggregate, in an amount of 0 to 40 wt.%, preferably 0 to 20 wt.%, more preferably 0.1 to 15 wt.%, and particularly 0.1 to 10 wt.%, based on the total weight of the composition. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partly oxidized alk(en)yl oligoglycosides or glucuronic acid derivatives, fatty acid-N-alkyl glucamides, protein hydrolyzates (particularly wheat-based vegetable products), polyol fatty acid esters, sugar esters, sorbitan esters, polysorbates and amine oxides. If the nonionic surfactants contain polyglycol ether chains, they may have a conventional homolog distribution, although they preferably have a narrow-range homolog distribution.

Zwitterionic surfactants are surface-active compounds which contain at least one quaternary ammonium group and at least one -COO⁽⁻⁾ or -SO₃⁽⁻⁾ group in the molecule. Particularly suitable zwitterionic surfactants are the so-called betaines, such as the N-alkyl-N,N-dimethyl ammonium glycinates, for example, cocoalkyl dimethyl ammonium glycinate, N-acylaminopropyl-N,N-dimethyl ammonium glycinates, for example, co-coacylaminopropyl dimethyl ammonium glycinate, and 2-alkyl-3-carboxymethyl-3-hydroxyethyl imidazolines, containing 8 to 18 carbon atoms in the alkyl or acyl group, and cocoacylaminoethyl hydroxyethyl carboxymethyl glycinate. The fatty acid amide derivative known under the CTFA name of Cocamidopropyl Betaine is particularly preferred.

Ampholytic surfactants are also suitable, particularly as co-surfactants. Ampholytic surfactants are surface-active compounds which, in addition to a C₈ to C₁₈ alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and acyl sarcosine.

Anionic surfactants are characterized by a water-solubilizing anionic group such as, for example, a carboxylate, sulfate, sulfonate or phosphate group and a lipophilic group. Dermatologically safe anionic surfactants are known to the practitioner in large numbers from relevant textbooks and are commercially available. They are, in particular, alkyl sulfates in the form of their alkali metal, ammonium or alkanolammonium salts, alkylether sulfates, alkylether carboxylates, acyl isethionates, acyl sarcosinates, acyl taurines containing linear C₁₂-C₁₈ alkyl or acyl groups and sulfosuccinates and acyl glutamates in the form of their alkali metal or ammonium salts.

Particularly suitable cationic surfactants are quaternary ammonium compounds, preferably ammonium halides, more especially chlorides and bromides, such as alkyl trimethyl ammonium chlorides, dialkyl dimethyl ammonium chlorides and trialkyl methyl ammonium chlorides, for example, cetyl trimethyl ammonium chloride, stearyl trim ethyl ammonium chloride, distearyl dimethyl ammonium chloride, lauryl dimethyl ammonium chloride, lauryl dimethyl benzyl ammonium chloride and tricetyl methyl ammonium chloride. In addition, the readily biodegradable quaternary ester compounds, such as, for example, the dialkyl ammonium methosulfates and methyl hydroxyalkyl dialkoyloxyalkyl ammonium methosulfates marketed under the name of Stepantexe and the corresponding products of the Dehyquart^{®} series, may be used as cationic surfactants. "Esterquats" are generally understood to be quaternized fatty acid triethanolamine ester salts. They can provide the compositions with particular softness. They are known substances which are prepared by the relevant methods of organic chemistry. Other cationic surfactants suitable for use in accordance with the invention are the quaternized protein hydrolysates.

One embodiment of the invention is directed to a composition comprising at least one aroma chemical (I) which contains one or more acetate or propionate groups as defined above, acetic acid in the stated amounts, and at least one oil component.

The oil components are typically present in an amount of 0.1 to 80 wt.%, preferably 0.5 to 70 wt.%, more preferably 1 to 60 wt.%, even more preferably 1 to 50 wt.%, in particular 1 to 40 wt.%, more particularly 5 to 25 wt.% and specifically 5 to 15 wt.% based on the total weight of the composition.

The oil components may be selected, for example, from Guerbet alcohols based on fatty alcohols containing 6 to 18, preferably 8 to 10, carbon atoms and other additional esters, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of C₁₈-C₃₈ alkyl-hydroxycarboxylic acids with linear or branched C₆-C₂₂ fatty alcohols, more especially dioctyl malate, esters of linear and/or branched fatty acids with polyhydric alcohols (for example propylene glycol, dimer dial or trimer triol), triglycerides based on C₆-C₁₀ fatty acids, liquid mono-, di- and triglyceride mixtures based on C₆-C₁₈ fatty acids, esters of C₆-C₂₂ fatty alcohols and/or Guerbet alcohols with aromatic carboxylic acids, more particularly benzoic acid, esters of dicarboxylic acids with polyols containing 2 to 10 car- bon atoms and 2 to 6 hydroxyl groups, vegetable oils, branched primary alcohols, substituted cyclohexanes, linear and branched C₆-C₂₂ fatty alcohol carbonates such as, for example, dicaprylyl carbonate (Cetiol^{®} CC), Guerbet carbonates based on fatty alcohols containing 6 to 18, preferably 8 to 10, carbon atoms, esters of benzoic acid with linear and/or branched C₆ to C₂₂ alcohols (for example Finsolv^{®} TN), linear or branched, symmetrical or nonsymmetrical dialkyl ethers containing 6 to 22 carbon atoms per alkyl group such as, for example, dicaprylyl ether (Cetiol^{®} OE), ring opening products of epoxidized fatty acid esters with polyols and hydrocarbons or mixtures thereof.

### Anti-oxidants

One embodiment of the invention is directed to a composition comprising at least one aroma chemical (I) which contains one or more acetate or propionate groups as defined above, acetic acid in the stated amounts, and at least one anti-oxidant.

Anti-oxidants are able to inhibit or prevent the undesired changes in the compositions to be protected caused by oxygen effects and other oxidative processes. The effect of the anti-oxidants consists in most cases in them acting as free-radical scavengers for the free radicals which arise during autoxidation.

Anti-oxidants can for example be selected from the group consisting of
- amino acids (for example glycine, alanine, arginine, serine, threonine, histidine, tyrosine, tryptophan) and derivatives thereof,
- imidazoles (e.g. urocanic acid) and derivatives thereof,
- peptides, such as D,L-carnosine, D-carnosine, L-carnosine (=*β* -Alanyl-L-histidin) and derivatives thereof
- carotenoids, carotenes (e.g. alpha-carotene, beta-carotene, lycopene, lutein) or derivatives thereof,
- chlorogenic acid and derivatives thereof,
- lipoic acid and derivatives thereof (for example dihydrolipoic acid),
- auro-thioglucose, propylthiouracil and other thiols (for example thioredoxin, glutathione, cysteine, cystine, cystamine and the glycosyl, N-acetyl, methyl, ethyl, propyl, amyl, butyl and lauryl, palmitoyl, oleyl, gamma-linoleyl, cholesteryl and glyceryl esters thereof) and salts thereof,
- dilauryl thiodipropionate, distearyl thiodipropionate, thiodipropionic acid and derivatives thereof (esters, ethers, peptides, lipids, nucleotides, nucleosides and salts),
- sulfoximine compounds (for example buthionine sulfoximines, homocysteine sulfoximine, buthionine sulfones, penta-, hexa-, heptathionine sulfoximine)
- (metal) chelating agents (e.g. alpha-hydroxy fatty acids, palmitic acid, phytic acid, lactoferrin),
- alpha-hydroxy acids (for example citric acid, lactic acid, malic acid),
- humic acid, bile acid, bile extracts, bilirubin, biliverdin, boldin (= alkaloid from the plant Peumus boldus, boldo extract,
- EDTA, EGTA and derivatives thereof,
- unsaturated fatty acids and derivatives thereof (e.g. gamma-linolenic acid, linoleic acid, oleic acid),
- folic acid and derivatives thereof,
- ubiquinone and ubiquinol and derivatives thereof,
- vitamin C and derivatives (for example ascorbyl palmitate, Mg ascorbyl phosphate, ascorbyl acetate),
- tocopherols and derivatives (for example vitamin E acetate),
- vitamin A and derivatives (for example vitamin A palmitate),
- coniferyl benzoate of gum benzoin, rutic acid and derivatives thereof, alpha-glycosylrutin, ferulic acid, furfurylideneglucitol,
- butylhydroxytoluene (BHT), butylhydroxyanisole (BHA)
- nordihydroguaiacic acid, nordihydroguaiaretic acid, trihydroxybutyrophenone, uric acid and derivatives thereof, mannose and derivatives thereof,
- superoxide dismutase
- zinc and derivatives thereof (for example ZnO, ZnSO4),
- selenium and derivatives thereof (for example selenomethionine) and
- stilbenes and derivatives thereof (e.g. stilbene oxide, trans-stilbene oxide)

In a preferred embodiment, the anti-oxidant is selected from the group consisting of pentaerythrityl, tetra-di-t-butyl hydroxyhydrocinnamate, nordihydroguaiaretic acid, ferulic acid, resveratrol, propyl gallate, butylhydroxytoluene (BHT), butylhydroxyanisole (BHA), ascorbyl palmitate and tocopherol.

The compositions according to the invention can comprise the anti-oxidants in an amount of 0.001 to 25 wt.-%, preferably 0.005 to 10 wt.-%, preferably 0.01 to 8 wt.-%, preferably 0.025 to 7 wt.-%, preferably 0.05 to 5 wt.-%, based on the total weight of the composition.

### Deodorant-active agents

One embodiment of the invention is directed to a composition comprising at least one aroma chemical (I) which contains one or more acetate or propionate groups as defined above, acetic acid in the stated amounts, and at least one deodorant-active agent.

The at least one aroma chemical (I) which contains one or more acetate or propionate groups as defined above together with acetic acid in the stated amounts can be used to impart a clean, long-lasting note to deodorizing compositions as well as to the skin treated with such compositions.

Deodorizing compositions (deodorants and antiperspirants) counteract, mask or eliminate body odors. Body odors are formed through the action of skin bacteria on apocrine perspiration which results in the formation of unpleasant-smelling degradation products.

In a preferred embodiment of the invention, the at least one deodorant-active agent is selected from the groups consisting of anti-perspirants, esterase inhibitors and antibacterial agents.

Suitable antiperspirants can be selected from the group consisting of salts of aluminium, zirconium or zinc. Examples are aluminium chloride, aluminium chlorohydrate, aluminium dichlorohydrate, aluminium sesquichlorohydrate and complex compounds thereof, for example with 1,2-propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, for example with amino acids, such as glycine. Aluminium chlorohydrate, aluminium zirconium tetrachlorohydrate, aluminium zirconium pentachlorohydrate and complex compounds thereof are preferably used.

In a preferred embodiment of the invention the compositions comprise at least one antiperspirant selected from the group consisting aluminium chloride, aluminium chlorohydrate, aluminium dichlorohydrate, aluminium sesquichlorohydrate, aluminium hydroxyallantoinate, aluminium chloride tartrate, aluminium zirconium trichlorohydrate, aluminium zirconium tetrachlorohydrate and aluminium zirconium pentachlorohydrate

The compositions according to the invention can comprise the antiperspirants in an amount of 1 to 50, preferably 5 to 30 and more particularly 10 to 25 wt.-%, based on the solids content of the composition.

Where perspiration is present in the underarm region, extracellular enzymes-esterases, mainly proteases and/or lipases are formed by bacteria and split the esters present in the perspiration, releasing odors in the process. Suitable esterase inhibitors are for example trialkyl citrates, such as trimethyl citrate, tripropyl citrate, triisopropyl citrate, tributyl citrate and, in particular, triethyl citrate. Esterase inhibitors inhibit enzyme activity and thus reduce odor formation. The free acid is probably released by the cleavage of the citric acid ester and reduces the pH value of the skin to such an extent that the enzymes are inactivated by acylation. Other esterase inhibitors are sterol sulfates or phosphates such as, for example, lanosterol, cholesterol, campesterol, stigmasterol and sitosterol sulfate or phosphate, dicarboxylic acids and esters thereof, for example glutaric acid, glutaric acid monoethyl ester, glutaric acid diethyl ester, adipic acid, adipic acid monoethyl ester, adipic acid diethyl ester, malonic acid and malonic acid diethyl ester, hydroxycarboxylic acids and esters thereof, for example citric acid, malic acid, tartaric acid or tartaric acid diethyl ester, and zinc glycinate.

In a preferred embodiment of the invention the compositions comprise at least one esterase inhibitor selected from the group consisting of trimethyl citrate, tripropyl citrate, triisopropyl citrate, tributyl citrate triethyl citrate, lanosterol, cholesterol, campesterol, stigmasterol, sitosterol sulfate, sitosterol phosphate, glutaric acid, glutaric acid monoethyl ester, glutaric acid diethyl ester, adipic acid, adipic acid monoethyl ester, adipic acid diethyl ester, malonic acid, malonic acid diethyl ester, citric acid, malic acid, tartaric acid, tartaric acid diethyl ester and zinc glycinate.

The compositions according to the invention can comprise the esterase inhibitors in amounts of 0.01 to 20, preferably 0.1 to 10 and more particularly 0.5 to 5 wt.-%, based on the solids content of the composition.

The term "anti-bacterial agents" as used herein encompasses substances which have bactericidal and/or bacteriostatic properties. Typically these substances act against gram-positive bacteria such as, for example, 4-hydroxybenzoic acid and salts and esters thereof, N-(4-chlorophenyl)-N'-(3,4-dichlorophenyl)-urea, 2,4,4'-trichloro-2'-hydroxydiphenylether (triclosan), 4-chloro-3,5-dimethylphenol, 2,2'-methylene-bis-(6-bromo-4-chlorophenol), 3-methyl-4-(1-methylethyl)-phenol, 2-benzyl-4-chlorophenol, 3-(4-chlorophenoxy)-propane-1,2-diol, 3-iodo-2-propinyl butyl carbamate, chlorhexidine, 3,4,4'-trichlorocarbanilide (TTC), phenoxyethanol, glycerol monocaprate, glycerol monocaprylate, glycerol monolaurate (GML), diglycerol monocaprate (DMC), salicylic acid-N-alkylamides such as, for example, salicylic acid-n-octyl amide or salicylic acid-n-decyl amide.

In a preferred embodiment the antibacterial agent is selected from the group consisting of chitosan, phenoxyethanol, 5-chloro-2-(2,4-dichlorophenoxy)-phenol, 4-hydroxybenzoic acid and salts and esters thereof, N-(4-chlorophenyl)-N'-(3,4-dichlorophenyl)-urea, 2,4,4'-trichloro-2'-hydroxydiphenylether (triclosan), 4-chloro-3,5-dimethylphenol, 2,2'-methylene-bis-(6-bromo-4-chlorophenol), 3-methyl-4-(1-methylethyl)-phenol, 2-benzyl-4-chlorophenol, 3-(4-chlorophenoxy)-propane-1,2-diol, 3-iodo-2-propinyl butyl carbamate, chlorhexidine, 3,4,4'-trichlorocarbanilide (TTC), phenoxyethanol, glycerol monocaprate, glycerol monocaprylate, glycerol monolaurate (GML), diglycerol monocaprate (DMC), salicylic acid-N-alkylamides.

The compositions according to the invention can comprise the antibacterial agents in amounts of 0.01 to 5 wt.% and preferably 0.1 to 2 wt.-%, based on the solids content of the composition.

The compositions according to the invention can further comprise one or more substances, such as, for example: preservatives, abrasives, anti-acne agents, agents to combat skin aging, anti-cellulite agents, antidandruff agents, anti-inflammatory agents, irritation-preventing agents, irritation-alleviating agents, astringents, sweat-inhibiting agents, antiseptics, anti-statics, binders, buffers, carrier materials, chelating agents, cell stimulants, care agents, hair removal agents, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant substances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-calming agents, skin-cleansing agents, skin care agents, skin-healing agents, skin lightening agents, skin-protective agents, skin-softening agents, cooling agents, skin-cooling agents, warming agents, skin-warming agents, stabilizers, UV-absorbent agents, UV filters, fabric softeners, suspending agents, skin-tanning agents, thickeners, vitamins, waxes, fats, phospholipids, saturated fatty acids, mono- or polyunsaturated fatty acids, α-hydroxy acids, polyhydroxy fatty acids, liquefiers, dyes, color-protection agents, pigments, anti-corrosives, polyols, electrolytes, or silicone derivatives.

The at least one aroma chemical (I) which contains one or more acetate or propionate groups as defined above together with acetic acid in the stated amounts can be used in a wide range of compositions, preferably in aroma compositions, more preferably in fragrance compositions. The olfactory properties and the substance properties (such as solubility in customary solvents and compatibility with further customary constituents of such compositions) of the compounds underline the particular suitability of the combinations for the stated use purposes and compositions.

Suitable compositions are for example perfume compositions, body care compositions (including cosmetic compositions), products for oral and dental hygiene, hygiene articles, cleaning compositions (including dishwashing compositions), textile detergent compositions, compositions for scent dispensers, foods, food supplements, pharmaceutical compositions and crop protection compositions.

Perfume compositions can be selected from fine fragrances, air fresheners in liquid form, gel-like form or a form applied to a solid carrier, aerosol sprays, scented cleaners, perfume candles and oils, such as lamp oils or oils for massage.

Examples for fine fragrances are perfume extracts, Eau de Parfums, Eau de Toilettes, Eau de Colognes, Eau de Solide and Extrait Parfum.

Body care compositions include cosmetic compositions, and can be selected from after-shaves, pre-shave products, splash colognes, solid and liquid soaps, shower gels, shampoos, shaving soaps, shaving foams, bath oils, cosmetic emulsions of the oil-in-water type, of the water-in-oil type and of the water-in-oil-in-water type, such as e.g. skin creams and lotions, face creams and lotions, sunscreen creams and lotions, after-sun creams and lotions, hand creams and lotions, foot creams and lotions, hair removal creams and lotions, after-shave creams and lotions, tanning creams and lotions, hair care products such as e.g. hairsprays, hair gels, setting hair lotions, hair conditioners, hair shampoo, permanent and semi-permanent hair colorants, hair shaping compositions such as cold waves and hair smoothing compositions, hair tonics, hair creams and hair lotions, deodorants and antiperspirants such as e.g. underarm sprays, roll-ons, deodorant sticks and deodorant creams, products of decorative cosmetics such as e.g. eye-liners, eye-shadows, nail varnishes, make-ups, lipsticks and mascara. Further examples are given above.

Products for oral and dental hygiene can be selected from toothpaste, dental floss, mouth wash, breath fresheners, dental foam, dental gels and dental strips.

Hygiene articles can be selected from joss sticks, insecticides, repellents, propellants, rust removers, perfumed freshening wipes, armpit pads, baby diapers, sanitary towels, toilet paper, cosmetic wipes, pocket tissues, dishwasher and deodorizer.

Cleaning compositions, such as e.g. cleaners for solid surfaces, can be selected from perfumed acidic, alkaline and neutral cleaners, such as e.g. floor cleaners, window cleaners, dishwashing compositions both for handwashing and machine washing use, bath and sanitary cleaners, scouring milk, solid and liquid toilet cleaners, powder and foam carpet cleaners, waxes and polishes such as furniture polishes, floor waxes, shoe creams, disinfectants, surface disinfectants and sanitary cleaners, brake cleaners, pipe cleaners, limescale removers, grill and oven cleaners, algae and moss removers, mold removers, facade cleaners.

Textile detergent compositions can be selected from liquid detergents, powder detergents, laundry pretreatments such as bleaches, soaking agents and stain removers, fabric softeners, washing soaps, washing tablets.

Food means a raw, cooked, or processed edible substance, ice, beverage or ingredient used or intended for use in whole or in part for human consumption, or chewing gum, gummies, jellies, and confectionaries.

A food supplement is a product intended for ingestion that contains a dietary ingredient intended to add further nutritional value to the diet. A dietary ingredient may be one, or any combination, of the following substances: a vitamin, a mineral, an herb or other botanical, an amino acid, a dietary substance for use by people to supplement the diet by increasing the total dietary intake, a concentrate, metabolite, constituent, or extract. Food supplements may be found in many forms such as tablets, capsules, softgels, gelcaps, liquids, or powders.

Pharmaceutical compositions comprise compositions which are intended for use in the diagnosis, cure, mitigation, treatment, or prevention of disease as well as articles (other than food) intended to affect the structure or any function of the body of man or other animals.

Crop protection compositions comprise compositions which are intended for the managing of plant diseases, weeds and other pests (both vertebrate and invertebrate) that damage agricultural crops and forestry. A more detailed definition is given above.

Preferably, the composition of the invention is a perfume composition, a body care composition (including cosmetic compositions), a product for oral and dental hygiene, a hygiene article, a cleaning composition (including dishwashing compositions), a textile detergent composition, a composition for scent dispensers, or a crop protection composition.

In particular, the composition of the invention is a personal care or a homecare composition, such as a body care composition (including cosmetic compositions), a cleaning composition (including dishwashing compositions) or a textile detergent composition.

The compositions according to the invention can further comprise one or more substances, such as, for example: preservatives, abrasives, anti-acne agents, agents to combat skin aging, antibacterial agents, anti-cellulite agents, antidandruff agents, anti-inflammatory agents, irritation-preventing agents, irritation-alleviating agents, antimicrobial agents, antioxidants, astringents, sweat-inhibiting agents, antiseptics, anti-statics, binders, buffers, carrier materials, chelating agents, cell stimulants, cleaning agents, care agents, hair removal agents, surface-active substances, deodorizing agents, antiperspirants, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant substances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-calming agents, skin-cleansing agents, skin care agents, skin-healing agents, skin lightening agents, skin-protective agents, skin-softening agents, cooling agents, skin-cooling agents, warming agents, skin-warming agents, stabilizers, UV-absorbent agents, UV filters, fabric softeners, suspending agents, skin-tanning agents, thickeners, vitamins, waxes, fats, phospholipids, saturated fatty acids, mono- or polyunsaturated fatty acids, α-hydroxy acids, polyhydroxy fatty acids, liquefiers, dyes, color-protection agents, pigments, anti-corrosives, polyols, electrolytes, or silicone derivatives.

In one preferred embodiment, the composition of the invention is a homecare composition, in particular a dishwashing composition, laundry composition or a surface cleaning composition. Such compositions comprise at least one surfactant and often at least one of following components: Sequestrants, enzymes, foamers, foam stabilizers, antimicrobials, pH adjusting agents (acid or base), and if the composition is liquid or gel like also water and optionally also an organic solvent.

Just by way of example, such a composition can comprise
(a) 0.1 to 40% by weight, relative to the total weight of the composition, of one or more surfactants;
(b.1) 0.01 to 5% by weight of one or more aroma chemicals (I) which contain one or more acetate or propionate groups as defined above;
(b.2) acetic acid in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and the aroma chemical(s) (I) of component (b.1);
(c) 0 to 15% by weight, relative to the total weight of the composition, of at least one organic solvent;
(d) 0 to 6 by weight, relative to the total weight of the composition, of at least one enzyme;
(e) 0 to 20% by weight, relative to the total weight of the composition, of at least one sequestrant;
(f) 0 to 8% by weight, relative to the total weight of the composition, of at least one defoamer and/or foam stabilizer;
(g) optionally a pH adjusting agent; amount adapted to obtain the desired pH;
(h) 0 to 50% by weight, relative to the total weight of the composition, of a further additive;
(i) optionally water ad 100%.

Suitable surfactants of component (a) correspond to those listed above. They can be non-ionic, anionic, cationic, zwitterionic or amphoteric. Generally, mixtures of two or more surfactants are used.

Suitable solvents of component (c) are for example C₂-C₃-alkanols (e.g. ethanol, n-propanol, isopropanol), C₂-C₃-alkanediols (e.g. ethyleneglycol, propylene glycol), C₁-C₄-alkylmonoethers of C₂-C₃-alkanediols (e.g. ethylene glycol monomethyl ether, ethylene glycol monoethyl ether, ethylene glycol monopropyl ether, ethylene glycol mono-n-butyl ether (also termed butylglyol), propylene glycol monomethyl ether, propylene glycol monoethyl ether, propylene glycol monopropyl ether and propylene glycol mono-n-butyl ether), polyetherpolyols (e.g. polyethylene glycol, polypropylene glycol), C₁-C₃-alkylmonoethers of polyetherpolyols (e.g. polyethylene glycol monomethyl ether, polyethylene glycol monoethyl ether, polyethylene glycol mono-n-propyl ether, polyethylene glycol mono-n-butyl ether), 5-, 6- or 7-membered lactones which may be substituted by one or more C₁-C₁₂-alkyl groups (e.g. γ-butyrolactone, γ-valerolactone, γ-octalactone, γ-nonalactone, δ-valerolactone, δ-decanolactone, δ-dodecanolactone and ε-caprolactone); 5-, 6- or 7-membered cyclic carbonates which may be substituted by one or more C₁-C₁₂-alkyl groups (e.g. ethylene carbonate, propylene carbonate and butylene carbonate which may carry one or more C₁-C₁₂-alkyl substituents); aliphatic esters (e.g. ethyl acetate, propyl acetate, butyl acetate, methylpropionate and ethyl propionate), carboxamides (e.g. N,N-dimethylformamide, N,N-diethylformamide and N,N-dimethylacetamide) or mixtures thereof.

Suitable enzymes of component (d) are those typically used in laundry, dishwashing or surface cleaning compositions, such as hydrolases, e.g. proteases, esterases, gluco-sidases, lipases, DNAses, amylases, cellulases, mannanases, other glycosylhydrolases and mixtures of the aforementioned enzymes. All these hydrolases contribute to dissolution and removal of soil from protein-, grease- or starch-containing stains/residues. For bleaching, it is also possible to use oxidoreductases.

Sequestrants of component (e), also termed builders, structural substances, framework substances, complexing agents, chelators, chelating agents or softeners, bind alkaline earth metals and other water-soluble metal salts without precipitating. They help to break up soil, disperse soil components, help to detach soil and in some cases themselves have a washing effect. Many sequenstrants are multi-functional, meaning that the substances have additional functions, such as a dispersing activity or anti-greying properties. Suitable sequestrants may be either organic or inorganic in nature. Examples are aluminosilicates, carbonates, phosphates and polyphosphates, polycarboxylic acids, polycarboxylates, hydroxycarboxylic acids, phosphonic acids, e.g. hydroxyalkylphosphonic acids, phosphonates, aminopolycarboxylic acids and salts thereof, and polymeric compounds containing carboxylic acid groups and salts thereof. More specific examples for inorganic sequestrants are crystalline or amorphous aluminosilicates with ion-exchanging properties, such as zeolites; disilicates or sheet silicates; carbonates and hydrogencarbonates, generally in the form of their alkali metal, alkaline earth metal or ammonium salts; alkali metal orthophosphates and/or polyphosphates, for example pentasodium triphosphate. More specific examples for organic sequestrants are C₄-C₃₀-di-, -tri- and -tetracarboxylic acids, e.g. succinic acid, propanetricarboxylic acid, butanetetracarboxylic acid, cyclopentanetetracarboxylic acid, and alkyl- and alkenylsuccinic acids with C₂-C₂₀-alkyl or -alkenyl radicals; hydroxycarboxylic acids and polyhydroxycarboxylic acids (sugar acids), e.g. malic acid, tartaric acid, glutonic acid, mucic acid, lactic acid, glutaric acid, citric acid, tartronic acid, glucoheptonic acid, lactobionic acid, and sucrosemono-, -di- and -tricarboxylic acid; phosphonic acids, e.g. hydroxyalkylphosphonic acids or aminophosphonic acids, and the salts thereof; poly-asparatic acids and their salts; polymeric compounds containing carboxylic acid groups, such as acrylic acid homopolymers; oligomaleic acids, etc..

Defoamer and/or foam stabilizer of component (f) are for example soaps, paraffins and silicone oils.

pH adjusting agents of component (g) are acids, bases and buffering systems. The acids can be inorganic or organic. Suitable inorganic acids are for example sulfuric acid, hydrochloric acid and phosphoric acid. Suitable organic acids are for example aliphatic, saturated non-substituted C₁-C₆-mono-, di- and tri-carboxylic acids such as formic acid, acetic acid, propanoic acid, oxalic acid, succinic acid and glutaric acid; aliphatic, saturated C₁-C₆-mono-, di- and tri-carboxylic acids carrying one or more OH groups, such as lactic acid, tartric acid and citric acid; aliphatic, unsaturated C₁-C₆-mono-, di- and tri-carboxylic acids such as sorbic acid; aromatic carboxylic acids, such as benzoic acid, salicylic acid and mandelic acid; and sulfonic acids, such as methanesulfonic acid or toluenesulfonic acid. The organic acids mainly serve for adapting the pH of the composition, but some of them, e.g. the di-and tricarboxylic acids, can also act as sequestrants. Suitable bases are in particular inorganic bases, such as the carbonates mentioned in context with the sequestrant, e.g. sodium or potassium carbonate; further alkali metal and earth alkaline metal hydroxides, such as NaOH or KOH. Suitable buffering agents are the typical systems, such as hydrogenphosphate/dihydrogenphosphate buffer, carbonate/hydrogencarbonate buffer, acetic acid/acetate buffer or Tris buffer. Moreover, most of the above acids which are weak and the anion of which is not a strong salt also have buffering capacity.

Examples for further additives (h) are antimicrobials, including preservatives, fragrances different from (b), hydrotropic agents, enzyme stabilizers, bleaching agents, corrosion inhibitors, dyes, thickeners, activity enhancers and inorganic salts.

If the composition is liquid or gel-like, it mandatorily contains water, generally in an amount of 10 to 99.89% by weight, relative to the total weight of the composition. In liquid ready-to-use compositions (ready-to-use-compositions are compositions which are used as such and do not need any further dilution or addition of further substances), the amount of water is generally in the range of 30 to 99.89% by weight, often 40 to 99% by weight, relative to the total weight of the composition.

In a particular embodiment, the homecare composition is a refill concentrate. Refill concentrates contain all ingredients of the final ready-to-use product, but for water which is either essentially absent ("essentially" taking account of the fact that some of the ingredients may contain some residual water) or contained in amounts far below those of the final ready-to-use product. The end user has just to fill the refill concentrate into a suitable container and add the indicated amount of water.

Such refill concentrates preferably comprise:
(a) 1 to 60% by weight, relative to the total weight of the composition, of one or more surfactants;
(b.1) 0.01 to 5% by weight of one or more aroma chemicals (I) which contain one or more acetate or propionate groups as defined above;
(b.2) acetic acid in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and the aroma chemical(s) (I) of component (b.1);
(c) 0 to 30% by weight, relative to the total weight of the composition, of at least one organic solvent;
(d) 0 to 10% by weight, relative to the total weight of the composition, of at least one enzyme;
(e) 0 to 10% by weight, relative to the total weight of the composition, of at least one sequestrant;
(f) 0 to 10% by weight, relative to the total weight of the composition, of at least one defoamer and/or foam stabilizer;
(g) optionally a pH adjusting agent; amount adapted to obtain the desired pH;
(h) 0 to 50% by weight, relative to the total weight of the composition, of a further additive; and
(i) 0 to 20% by weight, relative to the total weight of the composition, of water;
where components (a) to (i) add to 100% by weight.

In another preferred embodiment, the composition of the invention is a personal care composition. Personal care compositions can be such for hygienic or cosmetic use. Examples for suitable personal care compositions are given above.

Suitable personal care compositions may exist in a wide variety of forms, for example in the form of liquid preparations as a W/O, O/W, O/W/O, W/O/W or PIT emulsion and all kinds of microemulsions; in the form of a non-emulsified, water-based liquid, in the form of a gel, in the form of an oil, a cream, milk or lotion, in the form of a spray (spray with propellant gas or pump-action spray) or an aerosol, in the form of a foam, in the form of a paste, in the form of a wet wipe (such as for cleaning the nappy area). The personal care compositions may be, for example, creams, gels, lotions, alcoholic and aqueous/alcoholic solutions, emulsions, wax/fat compositions, or ointments. Ingredients typically present in such personal care compositions and their amounts vary according to the specific formulation. Examples for such ingredients are solvents, surfactants, emulsifiers, rheology modifiers (generally thickeners), conditioners, emollients, skin caring ingredients, lubricants, fillers, antioxidants, dermatologically active ingredients, fragrances and water.

Preparations intended mainly for cleaning, such as soaps, shower gels and shampoos, contain at least one or more surfactants, often of the anionic type, optionally in combination with such of the zwitterionic type; and water. Furthermore, they generally contain at least one of following components: emulsifier, sequestrant, fragrance, pH modifier (generally an organic acid and/or an inorganic base), inorganic salt (mostly NaCl), dye, pigment, preservative, pearlescer, opacifier, viscosity modifier, conditioning agent, natural hair nutrients, dermatologically active ingredient.

Of special interest are hair-washing preparations in the form of shampoos. Of special interest are moreover shower gels. Shampoos as well as shower gels generally contain water and at least one anionic surfactant.

Suitable anionic surfactants are principally all those described above which are cosmetically acceptable. The overall amount of anionic surfactant in shampoo and shower gel compositions generally ranges from 0.5 to 45% by weight, e.g. from 1.5 to 35% by weight, preferably from 2 to 20% by weight, based on the total weight of the composition. In shampoos and in shower gels, it is useful to use surfactants which are good foam-formers. The shampoo or shower gel composition can moreover include non-ionic, amphoteric and/or cationic co-surfactants, which help impart aesthetic, haptic, combing, physical or cleansing properties to the composition. Suitable non-ionic, amphoteric/zwitterionic and cationic surfactants are those described above. The overall amount of non-ionic surfactants in the shampoo or shower gel compositions generally ranges from 0.5 to 15% by weight, preferably from 2 to 10% by weight, the overall amount of amphoteric or zwitterionic surfactant in the shampoo or shower gel compositions generally ranges from 0.5 to 15% by weight, preferably from 1 to 10% by weight, and the overall amount of cationic surfactant in the shampoo or shower gel compositions generally ranges from 0.05 to 1% by weight, more preferably from 0.08 to 0.5% by weight, based on the total weight of the composition. The total amount of surfactant (including any co-surfactant and/or any emulsifier) in a shampoo or shower gel composition is generally from 1 to 50% by weight, preferably from 2 to 40% by weight, more preferably from 10 to 25% by weight, based on the total weight of the composition.

In a shampoo or shower gel composition the at least one aroma chemical (I) which contain one or more acetate or propionate groups as defined above is generally present in an amount of from 0.01 to 5% by weight, preferably from 0.01 to 2% by weight, in particular from 0.01 to 1% by weight, based on the total weight of the composition.

If the shampoo or shower gel composition is liquid or gel-like, this contains water; generally in an amount of from 30 to 98% by weight, preferably from 50 to 90% by weight, relative to the total weight of the composition.

Other components which may be present in the shampoo or shower gel are for example suspending agents, such as polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives, the latter being preferably selected from ethylene glycol stearate, alkanolamides of fatty acids having from 16 to 22 carbon atoms and mixtures thereof; fragrances different from 1,4-butanediol mono- and diacetate, dyes and pigments, pH adjusting agents, preservatives, pearlescers or opacifiers, viscosity modifiers, and salts, such as as NaCl; in shampoos moreover conditioning agents, natural hair nutrients or dermatologically active ingredients, such as botanicals, fruit extracts, caffeine, panthenol, sugar derivatives, amino acids, such as hydrolized keratine or glycine, vegetable or hydrogenated vegetable oils.

The aroma chemical (I) which contains one or more acetate or propionate groups and acetic acid may be worked into in compositions simply by directly mixing them with the basic composition lacking only this/these compound(s). Alternatively, the aroma chemical (I) which contains one or more acetate or propionate groups and acetic acid may be mixed simultaneously or consecutively with the other components of the composition or with pre-formed mixtures of a part of the other components.

The aroma chemical (I) which contains one or more acetate or propionate groups and acetic acid may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, or may be chemically bonded to substrates, which are adapted to release the compounds (I) upon application of an external stimulus such as light, enzyme, or the like, and then mixed with the composition.

The aroma chemical (I) which contains one or more acetate or propionate groups and acetic acid or compositions comprising these compounds according to the present invention can also be in microencapsulated form, spray-dried form, in the form of inclusion complexes or in the form of extrusion products. The properties can be further optimized by so-called "coating" with suitable materials with regard to a more targeted release of the scent, for which purpose preferably waxy synthetic substances such as e.g. polyvinyl alcohol are used.

The microencapsulation can take place for example by the so-called coacervation method with the help of capsule materials, e.g. made of polyurethane-like substances or soft gelatin. The spray-dried perfume oils can be produced for example by spray-drying an emulsion or dispersion comprising the aroma chemical (I) which contains one or more acetate or propionate groups and acetic acid, or a composition of the present invention described herein, wherein carrier substances that can be used are modified starches, proteins, dextrin and vegetable gums. Inclusion complexes can be prepared e.g. by introducing dispersions of fragrance compositions and cyclodextrins or urea derivatives into a suitable solvent, e.g. water. Extrusion products can be produced by melting an aroma chemical (I) which contains one or more acetate or propionate groups, or a composition of the present invention described herein with a suitable wax-like substance and by extrusion with subsequent solidification, optionally in a suitable solvent, e.g. isopropanol.

Generally, the total amount of the one or more aroma chemicals which contain one or more acetate or propionate groups in the compositions according to the present invention is typically adapted to the particular intended use or the intended application and can, thus, vary over a wide range. As a rule, the customary standard commercial amounts for scents are used.

The compositions according to the invention can comprise the aroma chemical (I) which contains one or more acetate or propionate groups in an overall amount of from 0.001 to 99.9% by weight, preferably from 0.01 to 90% by weight, more preferably from 0.01 to 80%, in particular from 0.01 to 60% by weight, more particularly from 0.01 to 40% by weight, e.g. from 0.01 to 10% by weight or 0.01 to 15% by weight or 0.01 to 5% by weight or 0.05 to 5% by weight, based on the total weight of the composition.

In one embodiment of the invention, the compositions comprise the aroma chemical (I) which contains one or more acetate or propionate groups in an overall amount of from 0.001 to 10 weight% or from 0.001 to 5 weight%, preferably from 0.01 to 2 weight% or from 0.05 to 5 weight%, based on the total weight of the composition.

The invention relates moreover to a method for preparing an aroma chemical composition, in particular a fragranced composition, specifically a fragranced ready-to-use composition, comprising incorporating at least one aroma chemical (I) which contains one or more acetate or propionate groups as defined above, and acetic acid in such an amount that it is present in said composition in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s), into said composition; or mixing at least one aroma chemical (I) which contains one or more acetate or propionate groups as defined above and acetic acid in such an amount that it is present in the resulting composition in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s), simultaneously or consecutively with the other components of said composition or with pre-formed mixtures of a part of the other components of said composition.

The invention relates also to a method for conferring an aroma, preferably a fragrance, to a composition, comprising incorporating at least one aroma chemical (I) which contains one or more acetate or propionate groups as defined above, and acetic acid in such an amount that it is present in said composition in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s), into said composition; or comprising mixing at least one aroma chemical (I) which contains one or more acetate or propionate groups as defined above and acetic acid in such an amount that it is present in the resulting composition in an amount of from 50 to 600 ppm, preferably from 70 to 600 ppm, more preferably from 70 to 550 ppm, even more preferably from 80 to 500 ppm, e.g. from 80 to 150 or from 100 to 500 or from 150 to 400 or from 200 to 300 ppm, relative to the total weight of acetic acid and said aroma chemical(s), simultaneously or consecutively with the other components of said composition or with pre-formed mixtures of a part of the other components of said composition.

Acetic acid in the stated concentrations/amounts intensifies the aroma, especially the fragrance, of aroma chemicals (I) which contain one or more acetate or propionate groups. In some instances, acetic acid also modifies the aroma character, especially the scent character, which in particular becomes fresher and greener. Acetic acid is a bulk chemical of abundant availability, can be obtained from renewable sources, is very economic, and environmentally and toxicologically harmless.

The following examples serve as further illustration of the invention.

### EXAMPLES

### Abbreviations:

- HOAc: acetic acid

### Tested aroma chemicals:

Following aroma chemicals (I) were tested:
- 1,4-butanediol diacetate
- neopentylglycol diacetate
- isopentyl acetate
- linalyl acetate
- benzyl acetate
- citronellyl acetate
- α-terpinyl acetate
- hexyl acetate
- 2-phenyethyl acetate

1,4-Butanediol diacetate and neopentylglycol diacetate were prepared by esterification of the respective glycols with acetic acid anhydride, as described below. The other aroma chemicals (I) were purchased from Sigma Aldrich.

### Preparation of 1,4-butanediol diacetate

In a 2.5 L jacketed tank equipped with a reflux condenser and impeller stirrer, acetic anhydride (1277 g, 12.5 mol, 2.05 equiv.) was added and heated to 120°C. 1,4-Butanediol (550 g, 6.10 mol, 1.00 equiv.) was added within 2.75 hours at such rate that the reaction temperature did not exceed 136°C. The reaction mixture was stirred at 135°C for an additional 2 hours, resulting in full conversion of 1,4-butanediol to 1,4-butanediol diacetate (yield: 99.4%). The reaction mixture was distilled in several steps to remove unreacted acetic anhydride and the major part of acetic acid to give the title product with an acetic acid content of 48 ppm, relative to the total weight of 1,4-butanediol diacetate and acetic acid (acetic acid content determined as describe below by determination of the acid number via potentiometric titration).

### Preparation of neopentylglycol diacetate

Neopentylglycol diacetate was prepared analogously to 1,4-butanediol diacetate.

### Preparation of the test samples

Prior to preparing the mixtures with acetic acid, commercial products (I) were subjected to a purification step (generally washing with water) to avoid disturbances in the aroma perception by impurities. Mixtures of the aroma chemicals (I) and acetic acid in a total amount of 50 ppm, 100 ppm, 200 ppm or 500 ppm, relative to the total weight of acetic acid and said aroma chemical (I) were prepared. To this end, the amount of acetic acid in the aroma chemicals (I) was determined and then acetic acid was added up to the indicated concentration.

The amount of acetic acid was determined via the determination of the acid number. The acid number was in turn determined via potentiometric titration: The samples were dissolved in isopropanol and titrated with a standardized tetrabutylammonium hydroxide (TBAH) solution in toluene/methanol (0.1 mol/l).

### Olfactory evaluation

10 wt.-% solutions of the aroma chemical/acetic acid mixtures in triethyl citrate (TEC) were prepared and evaluated from the headspace of the sample vial and fresh on blotter (= scent strip) by trained perfumers. Evaluation of each sample set was done under blinded conditions; i.e. the perfumers did not know the composition of the samples. The intensity was ranked from lowest (= 1) to highest (= 4) intensity within each series.

The results are compiled in the following table.

**Table 1: Fragrance intensity of aroma chemical (I)/acetic acid mixtures**

| Ex. | Aroma chemical (I) | 50 ppm HOAc (ex. 1.x.1) | 100 ppm HOAc (ex. 1.x.2) | 200 ppm HOAc (ex. 1.x.3) | 500 ppm HOAc (ex. 1.x.4) |
|---|---|---|---|---|---|
| 1.1.1-1.1.4 | 1,4-butanediol diacetate | 1^{a} | 4 | 2 | 3 |
| 1.2.1-1.2.4 | neopentylglycol diacetate | 1^{b} | 2 | 4 | 3 |
| 1.3.1-1.3.4 | isopentyl acetate | 1^{c} | 4 | 3 | 2 |
| 1.4.1-1.4.4 | linalyl acetate | 1^{d} | 2 | 4 | 3 |
| 1.5.1-1.5.4 | benzyl acetate | 1^{e} | 2 | 4 | 3 |
| 1.6.1-1.6.4 | citronellyl acetate | 1^{f} | 2 | 3 | 4 |
| 1.7.1-1.7.4 | α-terpinyl acetate | 29 | 3 | 4 | 1 |
| 1.8.1-1.8.4 | hexyl acetate* | 1^{h} | 3 | 4 | 2 |
| 1.9.1-1.9.4 | 2-phenylethyl acetate | 1ⁱ | 3 | 4 | 2 |

- ^{a}: scent description: raspberry, sweet, blueberry
- ^{b}: scent description: sweet, fresh, fruity, resinous, myrrh-like, with notes of fattiness
- ^{c}: scent description: pear, banana
- ^{d}: scent description: floral, sweet, citric, with notes of minty and caraway
- ^{e}: scent description: sweet, flowery, fresh and slightly fruity, reminiscent of jasmine
- ^{f}: scent description: floral-rosy, fruity, slightly citrus
- ^{g}: scent description: herbal, bergamot, lavender, lime, citrus
- ^{h}: scent description: fruity, green, apple, banana, sweet
- ⁱ: scent description: floral, rose, sweet, honey, fruity, tropical
- *: evaluated after 30 min on blotter

Acetic acid was not perceptible on the scent strips at any of the above concentrations. Only in the headspace of the vials of the samples containing 500 ppm of acetic acid, a weak vinegar note was faintly perceptible (except for hexyl acetate, where no acetic acid/vinegar note was perceptible).

With increasing acetic acid concentrations, the 1,4-butanediol diacetate/acetic acid samples were perceived as greener and more natural.

### Sales products and advantageous fragrance compositions

"Solution A" is non-diluted 1,4-butanediol diacetate containing 100 ppm of acetic acid, relative to the total weight of 1,4-butanediol diacetate and acetic acid (ex. 1.1.2).

"Solution B" is non-diluted neopentylglycol diacetate containing 200 ppm of acetic acid, relative to the total weight of neopentylglycol diacetate and acetic acid (ex. 1.2.3).

"Solution C" is non-diluted isopentyl acetate containing 100 ppm of acetic acid, relative to the total weight of isopentyl acetate and acetic acid (ex. 1.3.2).

"Solution D" is non-diluted linalyl acetate containing 200 ppm of acetic acid, relative to the total weight of linalyl acetate and acetic acid (ex. 1.4.3).

"Solution E" is non-diluted benzyl acetate containing 200 ppm of acetic acid, relative to the total weight of benzyl acetate and acetic acid (ex. 1.5.3).

"Solution F" is non-diluted citronellyl acetate containing 200 ppm of acetic acid, relative to the total weight of citronellyl acetate and acetic acid (ex. 1.6.3).

"Solution G" is non-diluted α-terpinyl acetate containing 200 ppm of acetic acid, relative to the total weight of α-terpinyl acetate and acetic acid (ex. 1.7.3).

"Solution H" is non-diluted hexyl acetate containing 200 ppm of acetic acid, relative to the total weight of hexyl acetate and acetic acid (ex. 1.8.3).

"Solution I" is non-diluted 2-phenylethyl acetate containing 200 ppm of acetic acid, relative to the total weight of 2-phenylethyl acetate and acetic acid (ex. 1.9.3).

### I. Advantageous fragrance compositions:

Solution A as described above was formulated in the compositions according to table

### 2. The amounts given in table 2 are weight units in grams.

**Table 2: Fragrance compositions 1A and 1B**

| | 1A | 1B |
|---|---|---|
| Lactone C10 gamma (5-hexyloxolan-2-one) | 2 | 2 |
| Bourgeonal (3-(4-tert-butylphenyl)propanal) | 2 | 2 |
| Citronellol | 3 | 3 |
| Aldehyde C-14 (5-heptyloxolan-2-one) | 3 | 3 |
| Allyl heptylate | 4 | 4 |
| Amber core (1-(2-tert-butylcyclohexyl)oxybutan-2-ol) | 4 | 4 |
| Ethyl-2-methyl butyrate | 4 | 4 |
| Geranyl acetate | 5 | 5 |
| Helional^{®} (3-(1,3-benzodioxol-5-yl)-2-methylpropanal) | 10 | 10 |
| Manzanate (ethyl 2-methylpentanoate) | 10 | 10 |
| Amberwood^{®} (ethoxymethoxycyclododecane) | 10 | 10 |
| Hexyl acetate | 11 | 11 |
| Benzyl salicylate | 12 | 12 |
| Magnolan^{®} (2,4-dimethyl-4,4a,5,9b-tetrahydroindeno[1,2-d][1,3]dioxine) | 15 | 15 |
| Verdox^{®} (2-tert-butylcyclohexyl) acetate) | 25 | 25 |
| Bergamot oil bergaptene free | 25 | 25 |
| Linalol | 30 | 30 |
| Dipropylene glycol | 45 | 45 |
| Iso E Super^{®} (Tetramethyl acetyloctahydronaphthalenes) | 110 | 110 |
| Pyranol (4-methyl-2-(2-methylpropyl)oxan-4-ol) | 170 | 170 |
| Hedione^{®} (methyl 3-oxo-2-pentylcyclopentaneacetate) | 200 | 200 |
| Galaxolide^{®} 50% IPM (1,3,4,6,7,8-hexahydro-4,6,6,7,8,8-hexamethylcyclopenta(g)-2-benzopyran 50% in isopropyl myristate) | 300 | 300 |
| Solution A | 25 | 50 |
| | 1025 | 1050 |

Solution A as described above is formulated in the compositions according to table 3.

The amounts given in table 3 are weight units in grams.

**Table 3: Fragrance compositions 2A and 2B**

| | 2A | 2B |
|---|---|---|
| Raspberry ketone (4-(4-hydroxyphenyl)butan-2-one) | 4 | 4 |
| Vanitrope^{®} (2-ethoxy-5-prop-1-enylphenol) | 6 | 6 |
| Cyclamen aldehyde (at least 90% 2-methyl-3-(p-isopropylphenyl)-propionaldehyde; secondary component: 5% 3-(p-cumenyl)-2-methylpropionic acid) | 10 | 10 |
| Bicyclononalactone (3,4,4a,5,6,7,8,8a-octahydrochromen-2-one) | 10 | 10 |
| Aldehyde C-14 (5-heptyloxolan-2-one) | 14 | 14 |
| Ethylvanillin (3-ethoxy-4-hydroxybenzaldehyde) | 16 | 16 |
| Heliotropine (1,3-benzodioxole-5-carbaldehyde) | 20 | 20 |
| Iso E Super^{®} (tetramethyl acetyloctahydronaphthalenes) | 20 | 20 |
| Sandela^{®} (3-[5,5,6-trimethylbicyclo[2.2.1]hept-2-yl]cyclohexan-1-ol) | 30 | 30 |
| Vanillin isobutyrate ((4-formyl-2-methoxyphenyl) 2-methylpropanoate) | 40 | 40 |
| Aldehyde C-18 (5-pentyloxolan-2-one) | 50 | 50 |
| Benzyl salicylate | 60 | 60 |
| Hexyl cinnamic aldehyde (2-(phenylmethylidene)octanal) | 70 | 70 |
| Hedione^{®} (methyl 3-oxo-2-pentylcyclopentaneacetate) | 130 | 130 |
| Pyranol (4-methyl-2-(2-methylpropyl)oxan-4-ol) | 150 | 150 |
| Ethylene brassylate (1,4-dioxacycloheptadecane-5,17-dione) | 170 | 170 |
| Galaxolide^{®} 50% IPM (1,3,4,6,7,8-hexahydro-4,6,6,7,8,8-hexamethylcyclopenta(g)-2-benzopyran 50% in isopropyl myristate) | 200 | 200 |
| Solution A | 10 | 20 |
| | 1010 | 1020 |

**Fragrance composition 3** corresponds to fragrance composition 1A, where Solution A is replaced by the same amount of Solution B. **Fragrance composition 4** corresponds to fragrance composition 1B, where Solution A is replaced by the same amount of Solution B. **Fragrance composition 5** corresponds to fragrance composition 2A, where Solution A is replaced by the same amount of Solution B. **Fragrance composition 6** corresponds to fragrance composition 2B, where Solution A is replaced by the same amount of Solution B.

**Fragrance composition 7** corresponds to fragrance composition 1A, where Solution A is replaced by the same amount of Solution C. **Fragrance composition 8** corresponds to fragrance composition 1B, where Solution A is replaced by the same amount of Solution C. **Fragrance composition 9** corresponds to fragrance composition 2A, where Solution A is replaced by the same amount of Solution C. **Fragrance composition 10** corresponds to fragrance composition 2B, where Solution A is replaced by the same amount of Solution C.

**Fragrance composition 11** corresponds to fragrance composition 1A, where Solution A is replaced by the same amount of Solution D. **Fragrance composition 12** corresponds to fragrance composition 1B, where Solution A is replaced by the same amount of Solution D. **Fragrance composition 13** corresponds to fragrance composition 2A, where Solution A is replaced by the same amount of Solution D. **Fragrance composition 14** corresponds to fragrance composition 2B, where Solution A is replaced by the same amount of Solution D.

**Fragrance composition 15** corresponds to fragrance composition 1A, where Solution A is replaced by the same amount of Solution E. **Fragrance composition 16** corresponds to fragrance composition 1B, where Solution A is replaced by the same amount of Solution E. **Fragrance composition 17** corresponds to fragrance composition 2A, where Solution A is replaced by the same amount of Solution E. **Fragrance composition 18** corresponds to fragrance composition 2B, where Solution A is replaced by the same amount of Solution E.

**Fragrance composition 19** corresponds to fragrance composition 1A, where Solution A is replaced by the same amount of Solution F. **Fragrance composition 20** corresponds to fragrance composition 1B, where Solution A is replaced by the same amount of Solution F. **Fragrance composition 21** corresponds to fragrance composition 2A, where Solution A is replaced by the same amount of Solution F. **Fragrance composition 22** corresponds to fragrance composition 2B, where Solution A is replaced by the same amount of Solution F.

**Fragrance composition 23** corresponds to fragrance composition 1A, where Solution A is replaced by the same amount of Solution G. **Fragrance composition 24** corresponds to fragrance composition 1B, where Solution A is replaced by the same amount of Solution G. **Fragrance composition 25** corresponds to fragrance composition 2A, where Solution A is replaced by the same amount of Solution G. **Fragrance composition 26** corresponds to fragrance composition 2B, where Solution A is replaced by the same amount of Solution G.

**Fragrance composition 27** corresponds to fragrance composition 1A, where Solution A is replaced by the same amount of Solution H. **Fragrance composition 28** corresponds to fragrance composition 1B, where Solution A is replaced by the same amount of Solution H. **Fragrance composition 29** corresponds to fragrance composition 2A, where Solution A is replaced by the same amount of Solution H. **Fragrance composition 30** corresponds to fragrance composition 2B, where Solution A is replaced by the same amount of Solution H.

**Fragrance composition 31** corresponds to fragrance composition 1A, where Solution A is replaced by the same amount of Solution I. **Fragrance composition 32** corresponds to fragrance composition 1B, where Solution A is replaced by the same amount of Solution I. **Fragrance composition 33** corresponds to fragrance composition 2A, where Solution A is replaced by the same amount of Solution I. **Fragrance composition 34** corresponds to fragrance composition 2B, where Solution A is replaced by the same amount of Solution I.

The compositions according to tables 2 and table 3, namely 1A, 1B, 2A and 2B as well as fragrance compositions 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33 and 34 could be included in various compositions selected from the group consisting of deo pump spray, clean hair-conditioner, face wash gel, foam bath concentrate, hair gel, self-foaming bodywash, sprayable sun care emulsion, sprayable sun protection emulsion, emollient facial gel, 2-phases oil foam bath, shampoos, shower bath, hydro-alcoholic AP/deo pump spray, aerosol, aqueous/alcoholic AP/deo roll-on, styling gel type "Out of Bed", shaving foam, sensitive skin baby shampoo, body wash for sensitive skin, gloss enhancing shampoo for sensitive scalp, deo stick, baby wipe, after shave balm, face gel, face day care cream, face cleanser, body lotion, sun care SPF50+, sprayable lotion, hand dish cleaner - regular, hand dish cleaner - concentrate, sanitary cleaner - concentrate, all-purpose cleaner, anti-bacterial fabric softener, detergent composition, powder detergent composition and liquid detergent composition.

A person skilled in art may be well versed with the various general formulations for the above-mentioned products.

Compositions 1A, 1B, 2A, 2B, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33 and 34 can for example be formulated in specific formulations as disclosed in IP.com Number: IPCOM000258614D entitled New Aroma Chemicals pages 6 to 46, Table 1 to Table D13, wherein the "Fragrance Composition 1A" is replaced by identical amounts of compositions 1A, 1B, 2A, 2B, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33 and 34.

### II. Advantageous fragrance compositions

### II.1 Perfume formulation for various uses

A base formulation of the composition listed in the following table was prepared. DPG is dipropylene glycol.

| Component | Parts by weight |
|---|---|
| Aldehyde C-09 10% in DPG | 70 |
| Aldehyde C-14 undecalactone | 200 |
| Allyl caproate | 200 |
| Amyl butyrate-N | 100 |
| Anisic aldehyde | 50 |
| Benzyl acetate | 100 |
| Cis-3-Hexenol | 40 |
| Cis-3-Hexenyl acetate | 100 |
| Cyclamen aldehyde | 100 |
| Damascone alpha | 50 |
| Decalactone gamma | 400 |
| Ethyl vanillin | 30 |
| Ethyl-2-methyl butyrate | 50 |
| Ethylene glycol brasylate | 2750 |
| Galbascone | 20 |
| Hedione | 200 |
| Helvetolide | 500 |
| Hexyl acetate | 250 |
| Ionone beta | 40 |
| Linalool | 3000 |
| Manzanate | 200 |
| Styryl acetate | 100 |
| Terpineol | 100 |
| Triplal | 100 |
| Verdox | 1000 |
| | 9750 |

To this base formulation 2.5% by weight of the product of example 1.1.2 is added. The resulting perfume formulation is olfactively evaluated as follows by a panel of trained perfumers:
The product of example 1.1.2 adds an own impression and signature to the base formulation, conferring to the perfume formulation a red fruit note and making it nicer, stronger, more natural, powerful and greener.

### II.2 Perfume formulation for various uses

A base formulation of the composition listed in the following table was prepared. DPG is dipropylene glycol.

| Component | Parts by weight |
|---|---|
| Hexenyl acetate, cis-3 | 1 |
| Rose oxide | 1 |
| Calone 1951 | 2 |
| Ethyl-2-methyl butyrate | 2 |
| Manzanate | 2 |
| Floralozone | 4 |
| Hexenol, cis-3 10% in DPG | 4 |
| Hexyl acetate | 5 |
| Aldehyde C-14 | 10 |
| Allyl heptylate | 10 |
| Ethyl acetoacetate | 10 |
| Lavandin oil Abrialis | 10 |
| Cyclamen aldehyde | 14 |
| Isobornyl acetate | 20 |
| Dimethyl benzyl carbinyl acetate | 30 |
| Benzyl acetate | 40 |
| Dihydromyrcenol | 40 |
| Nopyl acetate | 50 |
| Verdox | 50 |
| Pyranol | 60 |
| Tonalid | 60 |
| Hexyl salicylate | 95 |
| Tetrahydrolinalool | 120 |
| Phenylethyl alcohol | 130 |
| PTBCHA | 130 |
| Dipropylene glycol | 100 |
| | 1000 |

This base formulation had a green, light fruity, slightly tropical sweet, floral of jasmine and rose scent with woody, musky backnotes.

To this base formulation, 100 parts by weight of the product of example 1.1.2 are added. The resulting perfume formulation is characterized by a panel of trained perfumers as more fruity and sweet, more raspberry, moreover the musk note is more pronounced. The product of example 1.1.2 thus also acts as a booster for musk notes.

### II.3 Perfume formulation intended for use in dishwashing formulations

A base formulation of the composition listed in the following table for use in dishwashing formulations was prepared.

| Component | Parts by weight |
|---|---|
| Aldehyde C-08 | 20 |
| Aldehyde C-09 | 20 |
| Aldehyde C-10 | 50 |
| Aldehyde C-14 undecalactone | 200 |
| Allyl amyl glycolate | 100 |
| Anisic aldehyde | 50 |
| Benzaldehyde | 20 |
| Benzyl acetate | 400 |
| Cis-3-Hexenol | 150 |
| Cis-3-Hexenyl isobutyrate | 100 |
| Citral | 200 |
| Citronellal | 40 |
| Citronellol | 600 |
| Citronelyl nitrile | 200 |
| Cyclamen aldehyde | 100 |
| Decalactone gamma | 400 |
| Dihydromyrcenol | 700 |
| Diphenyl oxide | 80 |
| DPG | 270 |
| Geraniol | 600 |
| Geranyl acetate | 100 |
| Habanolide | 500 |
| Hedione | 500 |
| Hexyl cinnamic aldehyde N-alpha | 500 |
| lonone beta | 100 |
| Isocyclocitral | 5 |
| Isobornyl acetate | 300 |
| Isomenthone | 15 |
| Linalool Giv. | 700 |
| Methyl anthranilate | 100 |
| Methyl benzoate | 40 |
| Methyl ionone | 150 |
| Oxane pure | 8 |
| Phenoxyethyl isobutyrate | 450 |
| Prenyl acetate | 100 |
| Styryl acetate | 100 |
| Terpineol | 300 |
| Terpenyl acetate | 500 |
| Tetrahydrolinalool | 230 |
| Trans-2-dodecenal | 2 |
| Pyranol | 500 |
| | 9500 |

To this base formulation 500 parts by weight of the product of example 1.1.2 is added. The resulting perfume formulation is olfactively evaluated as follows by a panel of trained perfumers:
The product of example 1.1.2 adds a red fruit, strawberry, sweet note to the base formulation.

### II.4 Perfume formulation intended for use in reed diffusers

A formulation of the composition listed in the following table for use in reed diffusers (scent dispenser where the scent is dispensed by slow evaporation supported by reeds) is prepared.

| Component | Parts by weight |
|---|---|
| Davana oil | 2 |
| Ethyl vanillin | 2 |
| Damascenone 10% in DPG | 4 |
| Raspberry ketone | 4 |
| Cinnamic aldehyde | 8 |
| Vetivert oil bourbon | 8 |
| Citral | 10 |
| Manzanate | 10 |
| Methyl pamplemousse | 10 |
| Vanillin | 10 |
| Ambroxan | 12 |
| Eugenol | 12 |
| Lemon oil sicilian | 12 |
| Pepper oil black | 12 |
| Ambrocenide 10 10% in DPG | 14 |
| Safranolide 1% in DPG | 14 |
| Cashmeran | 15 |
| Coumarin | 16 |
| Aldehyde C-16 | 20 |
| Cedramber | 20 |
| Ethyl linalool | 20 |
| Linalyl acetate | 25 |
| Sandalore | 25 |
| lonone beta | 30 |
| Pyranol | 30 |
| Patchouli oil deironized | 35 |
| Pyranyl acetate | 50 |
| Galaxolide 50 DEP | 80 |
| Iso E super | 130 |
| Hedione | 160 |
| Dowanol DPM | 200 |
| Product of example 1.1.2 | 200 |
| | 1200 |

The resulting perfume formulation is olfactively evaluated by a panel of trained perfumers as sweeter, more fruity than without the product of example 1.1.2.

### II.5 Perfume formulation intended for use in shower gel

A base formulation of the composition listed in the following table for use in shower gels is prepared.

| Component | Parts by weight |
|---|---|
| Aldehyde C-10 | 2 |
| Evernyl | 3 |
| Thiomenthone 1% in DPG | 4 |
| Indolarome | 5 |
| Methyl anthranilate | 5 |
| Liffarome | 7 |
| Melonal | 8 |
| Calone 1951 | 9 |
| Ligustral | 9 |
| Allyl amyl glycolate | 10 |
| Ethyl-2-methyl butyrate | 16 |
| Florhydral | 18 |
| Oxane 1% in DPG | 20 |
| Styrallyl acetate | 20 |
| Lactone C-12 gamma | 22 |
| Undecavertol | 22 |
| Aldehyde C-14 | 25 |
| Phenyl ethyl alcohol | 25 |
| Allyl cyclohexane propionate | 30 |
| Benzyl salicylate | 30 |
| Geranyl acetate | 30 |
| Benzyl acetate | 35 |
| Methyl ionone gamma | 35 |
| Cyclacet | 40 |
| Hexyl cinnamic aldehyde | 40 |
| Cyclamen aldehyde | 45 |
| Dihydromyrcenol | 50 |
| Habanolide | 55 |
| Pyranol | 80 |
| Linalool | 80 |
| Hedione | 170 |
| Product of example 1.1.2 | 50 |
| | 1000 |

The resulting perfume formulation is olfactively evaluated by a panel of trained perfumers as a tropical fruit blend with multiple fruit notes including watermelon, kiwi, pineapple, passionfruit and grapefruit on a floral, musky base.

An analogous formulation is prepared, containing however 50 parts by weight of DPG instead of the product of example 1.1.2. This is characterized as a bright, fresh tropical fruit blend with obvious notes of watermelon and floral musky undertones.

A comparison of the two blends reveals that the product of example 1.1.2 introduces a more natural effect and multiplies the various tropical notes, especially in the direction of pineapple. Watermelon remains, but it is better outbalanced, other fruits becoming better perceivable. The product of example 1.1.2 thus also acts as a booster for certain notes.

### II.6 Perfume formulation intended for use in shower gel

A base formulation of the composition listed in the following table for use in shower gels is prepared.

| Component | Parts by weight |
|---|---|
| Hexenol cis-3- | 1 |
| Clary Sage Absolute | 2 |
| Ambroxan | 3 |
| Aldehyde C-14 | 4 |
| Grapefruit oil | 6 |
| Coriander oil Russian | 10 |
| Olibanum resinoid 50% | 10 |
| Petitgrain oil Paraguay | 12 |
| Evernyl | 12 |
| Heliotropine | 15 |
| Iso E Super | 15 |
| Tonalid | 15 |
| Mandarin oil green | 25 |
| Cedarwood oil Texas | 30 |
| Patchouli oil deironized | 30 |
| Vertofix Coeur | 30 |
| Bergamot oil | 40 |
| Coumarin | 40 |
| Pyranol | 40 |
| Hedione | 40 |
| Rosemary oil Tunisian | 40 |
| Dihydro myrcenol | 50 |
| Linalool | 50 |
| Galaxolide 50 | 60 |
| Linalyl acetate | 90 |
| Lavender oil Bulgarian | 100 |
| Lavandin oil Abrialis | 180 |
| Product of example 1.1.2 | 50 |
| | 1000 |

The resulting perfume formulation is olfactively evaluated by a panel of trained perfumers as a fresh, bright lavender fragrance with a dominant natural impression in a modern context provided by woody, musky and citrus elements.

An analogous formulation is prepared, containing however 50 parts by weight of DPG instead of the product of example 1.1.2. This is characterized as a classical lavender fragrance modernized with Dihydro myrcenol and Iso E Super and having a fresh citrus accord in the topnotes.

A comparison of the two blends reveals that the product of example 1.1.2 energizes the topnotes and delivers a more natural feel by virtue of its fruity notes which emphasize the ketonic notes of the lavandin and lavender.

### III. Ready-to-use compositions

### III.1 Shampoo or shower gel

A shampoo or shower gel formulation is prepared by mixing the following components in the given proportions:

| Trade name | INCI name /chemical name | Function | Amount [wt.%] |
|---|---|---|---|
| Dehyquart^{®} Guar N | Guar hydroxypropyltrimonium chloride | Conditioning agent | 0.30 |
| Texapon^{®} N 70 | Sodium laureth sulfate | Anionic surfactant | 12.86 |
| Dehyton^{®} PK 45 | Cocoamidopropyl betaine | Zwitterionic surfactant | 8.10 |
| Lamesoft^{®} Balance | Coco glucoside, hydrogenated castor oil | Stabilizer | 1.50 |
| Euperlan^{®} OP White | Glycol distearate, sodium laureth sulfate, cocoamidopropyl betaine, glyceryl oleate | Opacifier | 4.00 |
| | Sodium benzoate | Preservative | 0.50 |
| | Citric acid | pH adjustment | ad pH 6 |
| | Perfume composition from example II.5 containing 5% of the product of example 1.1.2 | Perfume | 1 |
| | Water, demineralized | Solvent | Ad 100% |

The shower/shampoo formulation has a pleasant fruity fragrance of the notes listed in example II.5.

The analogous formulation containing 1% of the perfume composition from example II.6 instead of the perfume composition from example II.5 has a pleasant lavender fragrance of the notes listed in example II.6.

The analogous formulation containing 0.1% of the product of example 1.1.2 instead of the perfume composition from example II.5 covered the unpleasant odor of the base formulation (i.e. above shampoo or shower gel formulation without perfume) well. The formulation containing 0.1% of the product of example 1.1.2 instead of the perfume composition from example II.5 has a distinctive fruity scent.

### III.2 Laundry detergent formulation

A laundry detergent formulation is prepared by mixing the following components in the given proportions:

| Trade name | INCI name /chemical name | Function | Amount [wt.%] |
|---|---|---|---|
| Maranil^{®} DBS/LC | Linear C₁₀-C₁₃-alkyl benzene sulfonate | Anionic surfactant | 5.5 |
| Texapon^{®} N 70 | Sodium laureth sulfate | Anionic surfactant | 5.4 |
| Lutensol^{®} AO 7 | C₁₃-₁₅-Oxoalkoholethoxylate with 7 EO | Non-ionic surfactant | 5.4 |
| Edenor^{®} K 12-18 | Stripped coconut fatty acid | Additive | 2.4 |
| | 1,2-Propanediol | Solvent | 6.0 |
| | Ethanol | Solvent, preservative | 2.0 |
| | Na-citrate dihydrate | Sequestrant | 3.0 |
| | NaOH | pH adjustment | 2.2 |
| Actinide^{®} RS 1500 | 5-chloro-2-methyl-4-isothiazolin-3-one (1.11%) and 2-methyl-4-isothiazolin-3-one (0.37%) | Preservative | 0.5 |
| Rheovis^{®} AT 120 | Methacrylic acid/acrylic acid esters copolymer | Thickener | 3 |
| | Perfume composition from example II.1 containing 2.5% of the product of example 1.1.2 | Perfume | 1 |
| | Water, demineralized | Solvent | Ad 100 |

The resulting clear and slightly yellowish formulation of a pH of 8.1 has a pleasant fruity fragrance of the notes listed in example II.1.

The analogous formulation containing 0.5% of the product of example 1.1.2 instead of the perfume composition from example II.1 covers the unpleasant odor of the base formulation (i.e. above laundry detergent formulation without perfume) well. Increasing the amount of the product of example 1.1.2 (but still below 1%) leads to a distinctive fruity scent.

## Claims

1. The use of acetic acid for intensifying or modifying the aroma, preferably the fragrance, of an aroma chemical which contains one or more acetic acid ester or propionic acid ester groups, where acetic acid is used in an amount of from 50 to 600 ppm, relative to the total weight of acetic acid and said aroma chemical.

2. The use of a composition containing acetic acid and at least one aroma chemical which contains one or more acetic acid ester or propionic acid ester groups, where acetic acid is contained in an amount of from 50 to 600 ppm, relative to the total weight of acetic acid and said at least one aroma chemical, as an aroma composition, preferably as a fragrance.

3. The use according to any of the preceding claims, where acetic acid is used or contained in an amount of from 70 to 600, preferably from 70 to 550 ppm, more preferably from 80 to 500 ppm, e.g. from 80 to 150 ppm or from 100 to 500 ppm or from 150 to 400 ppm or from 200 to 300 ppm, relative to the total weight of acetic acid and said at least one aroma chemical.

4. The use according to any of the preceding claims, where the aroma chemical which contains one or more acetic acid ester or propionic acid ester groups is selected from the group consisting of 1,4-butanediol diacetate, 1,4-butanediol monoacetate, 1,4-butanediol dipropionate, 1,4-butanediol monopropionate, neopentylglycol diacetate, neopentylglycol monoacetate, isopentyl acetate, linalyl acetate, 2-tert-butylcyclohexyl acetate, 4-tert-butylcyclohexyl acetate, benzyl acetate, citronellyl acetate, geranyl acetate, neryl acetate, tetrahydrogeranyl acetate, hexyl acetate, 2-octyl acetate, 2-phenylethyl acetate, α-terpinyl acetate, verdyl acetate, bornyl acetate, isobornyl acetate, leaf alcohol acetate (cis-3-hexenyl acetate), and mixtures of two or more of these aroma chemicals.

5. The use according to any of the preceding claims, where the aroma chemical which contains one or more acetic acid ester or propionic acid ester groups is an aroma chemical which contains one or more acetic acid ester groups.

6. The use according to any of claims 4 or 5, where the aroma chemical which contains one or more acetic acid ester or propionic acid ester groups is selected from the group consisting of 1,4-butanediol diacetate, 1,4-butanediol monoacetate, neopentylglycol diacetate, neopentylglycol monoacetate, isopentyl acetate, linalyl acetate, 2-tert-butylcyclohexyl acetate, 4-tert-butylcyclohexyl acetate, benzyl acetate, citronellyl acetate, geranyl acetate, neryl acetate, tetrahydrogeranyl acetate, hexyl acetate, 2-octyl acetate, 2-phenylethyl acetate, α-terpinyl acetate, verdyl acetate, bornyl acetate, isobornyl acetate, cis-3-hexenyl acetate, and mixtures of two or more of these aroma chemicals;
where the aroma chemical which contains one or more acetic acid ester or propionic acid ester groups is preferably selected from the group consisting of 1,4-butanediol diacetate, neopentylglycol diacetate, isopentyl acetate, linalyl acetate, 4-tert-butylcyclohexyl acetate, benzyl acetate, citronellyl acetate, geranyl acetate, hexyl acetate, 2-octyl acetate, 2-phenylethyl acetate, α-terpinyl acetate, isobornyl acetate, cis-3-hexenyl acetate, and mixtures of two or more of these aroma chemicals.

7. The use according to claim 6, where the aroma chemical which contains one or more acetic acid ester or propionic acid ester groups is selected from the group consisting of 1,4-butanediol diacetate, mixtures of 1,4-butanediol diacetate and 1,4-butanediol monoacetate, neopentylglycol diacetate, mixtures of neopentylglycol diacetate and neopentylglycol monoacetate, isopentyl acetate, linalyl acetate, benzyl acetate, citronellyl acetate, hexyl acetate, 2-phenylethyl acetate, α-terpinyl acetate and mixtures of two or more of these aroma chemicals;
where the aroma chemical which contains one or more acetic acid ester or propionic acid ester groups is preferably selected from the group consisting of 1,4-butanediol diacetate, neopentylglycol diacetate, isopentyl acetate, linalyl acetate, benzyl acetate, citronellyl acetate, hexyl acetate, 2-phenylethyl acetate, α-terpinyl acetate and mixtures of two or more of these aroma chemicals, and is preferably 1,4-butanediol diacetate.

8. A method for intensifying or modifying the aroma, preferably the fragrance, of an aroma chemical which contains one or more acetic acid ester or propionic acid ester groups as defined in any of claims 1 and 4 to 7 or of a mixture of different aroma chemicals which contain one or more acetic acid ester or propionic acid ester groups as defined in any of claims 1 and 4 to 7, comprising providing a composition containing said one or more aroma chemicals and from 50 to 600 ppm, preferably from 70 to 600 ppm, more preferably from 70 to 550 ppm, even more preferably from 80 to 500 ppm, e.g. from 80 to 150 ppm or from 100 to 500 ppm or from 150 to 400 ppm or from 200 to 300 ppm of acetic acid, relative to the total weight of acetic acid and said one or more aroma chemicals;
with the proviso that the aroma chemical which contains one or more acetic acid ester or propionic acid ester groups is not 4-tert-butylcyclohexyl acetate if the composition contains less than 100 ppm of acetic acid; in particular with the proviso that the aroma chemical which contains one or more acetic acid ester or propionic acid ester groups is not 4-tert-butylcyclohexyl acetate.

9. The method according to claim 8, comprising adding to said one or more aroma chemicals acetic acid in such an amount that a composition comprising said one or more aroma chemicals and from 50 to 600 ppm, preferably from 70 to 600 ppm, more preferably from 70 to 550 ppm, even more preferably from 80 to 500 ppm, e.g. from 80 to 150 ppm or from 100 to 500 ppm or from 150 to 400 ppm or from 200 to 300 ppm of acetic acid, relative to the total weight of acetic acid and said one or more aroma chemicals, is obtained; or, in case that the one or more aroma chemicals contain one or more acetic acid ester groups which are prepared using acetic acid or a derivative thereof, adjusting the reaction, purification and/or isolation conditions in the preparation process of said one or more aroma chemicals thus that a composition containing said one or more aroma chemicals and from 50 to 600 ppm, preferably from 70 to 600 ppm, more preferably from 70 to 550 ppm, even more preferably from 80 to 500 ppm, e.g. from 80 to 150 ppm or from 100 to 500 ppm or from 150 to 400 ppm or from 200 to 300 ppm of acetic acid, relative to the total weight of acetic acid and said one or more aroma chemicals is obtained.

10. Composition comprising
- at least one aroma chemical which contains one or more acetic acid ester or propionic acid ester groups as defined in any of claims 1 and 4 to 7,
- acetic acid in an amount of from 50 to 600 ppm, preferably from 70 to 600 ppm, more preferably from 70 to 550 ppm, even more preferably from 80 to 500 ppm, e.g. from 80 to 150 ppm or from 100 to 500 ppm or from 150 to 400 ppm or from 200 to 300 ppm, relative to the total weight of acetic acid and said at least one aroma chemical, and
- at least one further component selected from the group consisting of aroma chemicals different from said aroma chemicals which contain one or more acetic acid ester or propionic acid ester groups, non-aroma chemical carriers, anti-oxidants and deodorant-active agents; and in particular from the group consisting of aroma chemicals different from said aroma chemicals which contain one or more acetic acid ester or propionic acid ester groups, surfactants, oil components, solvents, anti-oxidants and deodorant-active agents with the proviso that the aroma chemical which contains one or more acetic acid ester or propionic acid ester groups is not 4-tert-butylcyclohexyl acetate if the composition contains less than 100 ppm of acetic acid; in particular with the proviso that the aroma chemical which contains one or more acetic acid ester or propionic acid ester groups is not 4-tert-butylcyclohexyl acetate.

11. The composition according to claim 10, where one or both of the following conditions (a) and/or (b) apply:
(a) in case that the non-aroma chemical carriers comprise one or more solvents, these are selected from the group consisting of ethanol, isopropanol, dipropylene glycol (DPG), propylene glycol, 1,2-butylene glycol, glycerol, diethylene glycol monoethyl ether, diethyl phthalate (DEP), isopropyl myristate (IPM), triethyl citrate (TEC), benzyl benzoate and mixtures thereof;
and/or
(b) the aroma chemicals different from said aroma chemicals which contain one or more acetic acid ester or propionic acid ester groups are selected from the group consisting of geranyl acetate, alpha-hexylcinnamaldehyde, 2-phenoxyethyl isobutyrate, dihydromyrcenol, methyl dihydrojasmonate (preferably with a content of cis isomer of more than 60 wt.%), 4,6,6,7,8,8-hexamethyl-1,3,4,6,7,8-hexahydrocyclopenta[g]benzopyran, tetrahydrolinalool, ethyllinalool, benzyl salicylate, 2-methyl-3-(4-tert-butylphenyl)propanal, cinnamyl alcohol, 4,7-methano-3a,4,5,6,7,7a-hexahydro-5-indenyl acetate, 4,7-methano-3a,4,5,6,7,7a-hexahydro-6-indenyl acetate, citronellol, citronellyl acetate, tetrahydrogeraniol, vanillin, linalyl acetate, styrolyl acetate, octahydro-2,3,8,8-tetramethyl-2-acetonaphthone, 2-acetyl-1,2,3,4,6,7,8-octahydro-2,3,8,8-tetramethylnaphthalene, hexyl salicylate, 4-tert-butylcyclohexyl acetate, 2-tert-butylcyclohexyl acetate, alpha-ionone, n-alpha-methylionone, alpha-isomethylionone, coumarin, terpinyl acetate, 2-phenylethyl alcohol, 4-(4-hydroxy-4-methylpentyl)-3-cyclohexenecarboxaldehyde, alpha-amylcinnamaldehyde, ethylene brassylate, (E)-3-methylcyclopentadec-5-enone, (Z)-3-methylcyclopentadec-5-enone, 15-pentadec-11-enolide, 15-pentadec-12-enolide, 15-cyclopentadecanolide, 1-(5,6,7,8-tetrahydro-3,5,5,6,8,8-hexamethyl-2-naphthalenyl)ethanone, 2-isobutyl-4-methyltetrahydro-2H-pyran-4-ol, 2-ethyl-4-(2,2,3-trimethyl-3-cyclopenten-1-yl)-2-buten-1-ol, cis-3-hexenyl acetate, trans-3-hexenyl acetate, trans-2/cis-6-nonadienol, 2,4-dimethyl-3-cyclohexenecarboxaldehyde, 2,4,4,7-tetramethyloct-6-en-3-one, 2,6-dimethyl-5-hepten-1-al, borneol, 3-(3-isopropylphenyl)butanal, 2-methyl-3-(3,4-methylenedioxyphenyl)-propanal, 3-(4-ethylphenyl)-2,2-dimethylpropanal, 7-methyl-2H-1,5-benzodioxepin-3(4H)-one, 3,3,5-trimethylcyclohexyl acetate (preferably with a content of cis isomers of 70 wt.% or more), 2,5,5-trimethyl-1,2,3,4,4a,5,6,7-octahydronaphthalen-2-ol, 3-(4-*tert*-butylphenyl)-pro-panal, ethyl 2-methylpentanoate, ethoxymethoxycyclododecane, 2,4-dimethyl-4,4a,5,9b-tetrahydroindeno[1,2-d][1,3]dioxine, (2-tert-butylcyclohexyl) acetate, 3-[5,5,6-trimethylbicyclo[2.2.1]hept-2-yl]cyclohexan-1-ol,
menthone, isomenthone, carvone, camphor, beta-ionone, beta-n-methylionone, beta-isomethylionone, alpha-irone, alpha-damascone, beta-damascone, beta-damascenone, delta-damascone, acetylated cedar wood oil, and mixtures thereof.

12. The composition according to any of claims 10 or 11, which is selected from the group consisting of perfume compositions, body care compositions, products for oral or dental hygiene, hygiene articles, cleaning compositions, textile detergent compositions, compositions for scent dispensers, and crop protection compositions.

13. A method for preparing an aroma chemical composition, in particular a fragranced composition, specifically a fragranced ready-to-use composition, comprising incorporating at least one aroma chemical which contains one or more acetic acid ester or propionic acid ester groups as defined in any of claims 1 and 4 to 7, and acetic acid in such an amount that it is present in said composition in an amount of from 50 to 600 ppm, preferably from 70 to 600 ppm, more preferably from 70 to 550 ppm, even more preferably from 80 to 500 ppm, e.g. from 80 to 150 ppm or from 100 to 500 ppm or from 150 to 400 ppm or from 200 to 300 ppm, relative to the total weight of acetic acid and said at least one aroma chemical, into said composition; or mixing at least one aroma chemical which contains one or more acetic acid ester or propionic acid ester groups as defined in any of claims 1 and 4 to 7 and acetic acid in such an amount that it is present in the resulting composition in an amount of from 50 to 600 ppm, preferably from 70 to 600 ppm, more preferably from 80 to 550 ppm, even more preferably from 80 to 500 ppm, e.g. from 80 to 150 ppm or from 100 to 500 ppm or from 150 to 400 ppm or from 200 to 300 ppm, relative to the total weight of acetic acid and said at least one aroma chemical, simultaneously or consecutively with the other components of said composition or with pre-formed mixtures of a part of the other components of said composition;
with the proviso that the aroma chemical which contains one or more acetic acid ester or propionic acid ester groups is not 4-tert-butylcyclohexyl acetate if acetic acid is present in said composition in an amount of less than 100 ppm; in particular with the proviso that the aroma chemical which contains one or more acetic acid ester or propionic acid ester groups is not 4-tert-butylcyclohexyl acetate.

14. A method for conferring an aroma, preferably a fragrance, to a composition, comprising incorporating at least one aroma chemical which contains one or more acetic acid ester or propionic acid ester groups as defined in any of claims 1 and 4 to 7, and acetic acid in such an amount that it is present in said composition in an amount of from 50 to 600 ppm, preferably from 70 to 600 ppm, more preferably from 70 to 550 ppm, even more preferably from 80 to 500 ppm, e.g. from 80 to 150 ppm or from 100 to 500 ppm or from 150 to 400 ppm or from 200 to 300 ppm, relative to the total weight of acetic acid and said at least one aroma chemical, into said composition; or comprising mixing at least one aroma chemical which contains one or more acetic acid ester or propionic acid ester groups as defined in any of claims 1 and 4 to 7 and acetic acid in such an amount that it is present in the resulting composition in an amount of from 50 to 600 ppm, preferably from 70 to 600 ppm, more preferably from 70 to 550 ppm, even more preferably from 80 to 500 ppm, e.g. from 80 to 150 ppm or from 100 to 500 ppm or from 150 to 400 ppm or from 200 to 300 ppm, relative to the total weight of acetic acid and said at least one aroma chemical, simultaneously or consecutively with the other components of said composition or with pre-formed mixtures of a part of the other components of said composition;
with the proviso that the aroma chemical which contains one or more acetic acid ester or propionic acid ester groups is not 4-tert-butylcyclohexyl acetate if acetic acid is present in said composition in an amount of less than 100 ppm; in particular with the proviso that the aroma chemical which contains one or more acetic acid ester or propionic acid ester groups is not 4-tert-butylcyclohexyl acetate.

15. The use or the composition or the method according to any of the preceding claims, where the aroma chemical which contains one or more acetic acid ester or propionic acid ester groups is selected from the group consisting of 1,4-butanediol diacetate, neopentylglycol diacetate, isopentyl acetate, linalyl acetate, benzyl acetate, citronellyl acetate, hexyl acetate, 2-phenylethyl acetate, α-terpinyl acetate and mixtures of two or more of these aroma chemicals, and where acetic acid is used or contained or present in an amount of from 50 to 500, preferably from 70 to 500 ppm, more preferably from 80 to 500 ppm, even more preferably from 100 to 500 ppm, particularly preferably from 100 to 300 ppm, relative to the total weight of acetic acid and said at least one aroma chemical.

## Patentansprüche

1. Die Verwendung von Essigsäure zur Intensivierung oder Modifizierung des Aromas, vorzugsweise des Duftes, einer Aromachemikalie, die eine oder mehrere Essigsäureester- oder Propionsäureestergruppen enthält, wobei Essigsäure in einer Menge von 50 bis 600 ppm, bezogen auf das Gesamtgewicht von Essigsäure und der genannten Aromachemikalie, verwendet wird.

2. Die Verwendung einer Zusammensetzung, die Essigsäure und mindestens eine Aromachemikalie enthält,die eine oder mehrere Essigsäureester- oder Propionsäureestergruppen enthält, wobei Essigsäure in einer Menge von 50 bis 600 ppm, bezogen auf das Gesamtgewicht von Essigsäure und der genannten mindestens einen Aromachemikalie, enthalten ist, als Aromazusammensetzung, vorzugsweise als Duftstoff.

3. Die Verwendung nach einem der vorhergehenden Ansprüche, wobei Essigsäure in einer Menge von 70 bis 600, vorzugsweise von 70 bis 550 ppm, besonders bevorzugt von 80 bis 500 ppm, z. B. von 80 bis 150 ppm oder von 100 bis 500 ppm oder von 150 bis 400 ppm oder von 200 bis 300 ppm, bezogen auf das Gesamtgewicht von Essigsäure und der genannten mindestens einen Aromachemikalie, verwendet oder enthalten ist.

4. Die Verwendung nach einem der vorhergehenden Ansprüche, wobei die Aromachemikalie, die eine oder mehrere Essigsäureester- oder Propionsäureestergruppen enthält, ausgewählt ist aus der Gruppe bestehend aus 1,4-Butandioldiacetat, 1,4-Butandiolmonoacetat, 1,4-Butandioldipropionat, 1,4-Butandiolmonopropionat, Neopentylglykoldiacetat, Neopentylglykolmonoacetat, Isopentylacetat, Linalylacetat, 2-tert-Butylcyclohexylacetat, 4-tert-Butylcyclohexylacetat, Benzylacetat, Citronellylacetat, Geranylacetat, Nerylacetat, Tetrahydrogeranylacetat, Hexylacetat, 2-Octylacetat, 2-Phenylethylacetat, α-Terpinylacetat, Verdylacetat, Bornylacetat, Isobornylacetat, Blattalkohol- acetat (cis-3-Hexenylacetat) und Mischungen von zwei oder mehr dieser Aromachemikalien.

5. Die Verwendung nach einem der vorhergehenden Ansprüche, wobei die Aromachemikalie, die eine oder mehrere Essigsäureester- oder Propionsäureestergruppen enthält, eine Aromachemikalie ist, die eine oder mehrere Essigsäureestergruppen enthält.

6. Die Verwendung nach einem der Ansprüche 4 oder 5, wobei die Aromachemikalie, die eine oder mehrere Essigsäureester- oder Propionsäureestergruppen enthält, ausgewählt ist aus der Gruppe bestehend aus 1,4-Butandioldiacetat, 1,4-Butandiol- monoacetat, Neopentylglykoldiacetat, Neopentylglykolmonoacetat, Isopentylacetat, Linalylacetat, 2-tert-Butylcyclohexylacetat, 4-tert-Butylcyclohexylacetat, Benzylacetat, Citronellylacetat, Geranylacetat, Nerylacetat, Tetrahydrogeranylacetat, Hexylacetat, 2-Octylacetat, 2-Phenylethylacetat, α-Terpinylacetat, Verdylacetat, Bornylacetat, Isobornylacetat, cis-3-Hexenylacetat und Mischungen von zwei oder mehr dieser Aromachemikalien;
wobei die Aromachemikalie, die eine oder mehrere Essigsäureester- oder Propionsäureestergruppen enthält, vorzugsweise ausgewählt ist aus der Gruppe bestehend aus 1,4-Butandioldiacetat, Neopentylglykoldiacetat, Isopentylacetat, Linalylacetat, 4-tert-Butylcyclohexylacetat, Benzylacetat, Citronellylacetat, Geranylacetat, Hexylacetat, 2-Octylacetat, 2-Phenylethylacetat, α-Terpinylacetat, Isobornylacetat, cis-3-Hexenylacetat und Mischungen von zwei oder mehr dieser Aromachemikalien.

7. Die Verwendung nach Anspruch 6, wobei die Aromachemikalie, die eine oder mehrere Essigsäureester- oder Propionsäureestergruppen enthält, ausgewählt ist aus der Gruppe bestehend aus 1,4-Butandioldiacetat, Mischungen aus 1,4-Butandioldiacetat und 1,4-Butandiolmonoacetat, Neopentylglykoldiacetat, Mischungen aus Neopentylglykoldiacetat und Neopentylglykolmonoacetat, Isopentylacetat, Linalylacetat, Benzylacetat, Citronellylacetat, Hexylacetat, 2-Phen-ylethylacetat, α-Terpinylacetat und Mischungen von zwei oder mehr dieser Aromachemikalien;
wobei die Aromachemikalie, die eine oder mehrere Essigsäureester- oder Propionsäureestergruppen enthält, vorzugsweise ausgewählt ist aus der Gruppe bestehend aus 1,4-Butandioldiacetat, Neopentylglykoldiacetat, Isopentylacetat, Linalyl-acetat, Benzylacetat, Citronellylacetat, Hexylacetat, 2-Phenylethylacetat, α-Terpinylacetat und Mischungen von zwei oder mehr dieser Aromachemikalien, und vorzugsweise 1,4-Butandioldiacetat ist.

8. Ein Verfahren zur Intensivierung oder Modifizierung des Aromas, vorzugsweise des Duftes, von einer Aromachemikalie, die eine oder mehrere Essigsäureester- oder Propionsäureestergruppen enthält, wie in einem der Ansprüche 1 und 4 bis 7 definiert, oder einer Mischung verschiedener Aromachemikalien, die eine oder mehrere Essigsäureester- oder Propionsäureestergruppen enthalten, wie in einem der Ansprüche 1 und 4 bis 7 definiert, umfassend das Bereitstellen einer Zusammensetzung, die die genannte(n) eine oder mehrere Aromachemikalie(n) und 50 bis 600 ppm, vorzugsweise 70 bis 600 ppm, besonders bevorzugt 70 bis 550 ppm, noch bevorzugter 80 bis 500 ppm, z. B. 80 bis 150 ppm oder 100 bis 500 ppm oder 150 bis 400 ppm oder 200 bis 300 ppm Essigsäure, bezogen auf das Gesamtgewicht von Essigsäure und der genannten einen oder mehreren Aromachemikalien, enthält;
mit der Maßgabe, dass die Aromachemikalie, die eine oder mehrere Essigsäureester- oder Propionsäureestergruppen enthält, nicht 4-tert-Butylcyclohexylacetat ist, wenn die Zusammensetzung weniger als 100 ppm Essigsäure enthält; insbesondere mit der Maßgabe, dass die Aromachemikalie, die eine oder mehrere Essigsäureester- oder Propionsäureestergruppen enthält, nicht 4-tert-Butylcyclohexylacetat ist.

9. Das Verfahren nach Anspruch 8, umfassend das Hinzufügen von Essigsäure zu der genannten einen oder mehreren Aromachemikalie(n) in einer solchen Menge, dass eine Zusammensetzung erhalten wird, die die genannte(n) eine oder mehrere Aromachemikalie(n) und 50 bis 600 ppm, vorzugsweise 70 bis 600 ppm, besonders bevorzugt 70 bis 550 ppm, noch bevorzugter 80 bis 500 ppm, z. B. 80 bis 150 ppm oder 100 bis 500 ppm oder150 bis 400 ppm oder 200 bis 300 ppm Essigsäure, bezogen auf das Gesamtgewicht von Essigsäure und der genannten einen oder mehreren Aromachemikalien, enthält; oder, falls die eine oder mehreren Aromachemikalien eine oder mehrere Essigsäureestergruppen enthalten, die unter Verwendung von Essigsäure oder einem Derivat davon hergestellt werden, Anpassen der Reaktions-, Reinigungs- und/oder Isolierungsbedingungen bei der Herstellung der genannten einen oder mehreren Aromachemikalien derart, dass eine Zusammensetzung erhalten wird, die die genannte(n) eine oder mehrere Aromachemikalie(n) und 50 bis 600 ppm, vorzugsweise 70 bis 600 ppm, besonders bevorzugt 70 bis 550 ppm, noch bevorzugter 80 bis 500 ppm, z. B. 80 bis 150 ppm oder 100 bis 500 ppm oder 150 bis 400 ppm oder 200 bis 300 ppm Essigsäure, bezogen auf das Gesamtgewicht von Essigsäure und der genannten einen oder mehreren Aromachemikalien, enthält.

10. Zusammensetzung umfassend
- mindestens eine Aromachemikalie, die eine oder mehrere Essigsäureester- oder Propionsäureestergruppen enthält, wie in einem der Ansprüche 1 und 4 bis 7 definiert,
- Essigsäure in einer Menge von 50 bis 600 ppm, vorzugsweise 70 bis 600 ppm, besonders bevorzugt 70 bis 550 ppm, noch bevorzugter 80 bis 500 ppm, z. B. 80 bis 150 ppm oder 100 bis 500 ppm oder 150 bis 400 ppm oder 200 bis 300 ppm, bezogen auf das Gesamtgewicht von Essigsäure und
der genannten mindestens einen Aromachemikalie, und
- mindestens eine weitere Komponente, ausgewählt aus der Gruppe bestehend aus Aromachemikalien, die sich von den genannten Aromachemikalien unterscheiden, die eine oder mehrere Essigsäureester- oder Propionsäureestergruppen enthalten, nicht-aromatischen chemischen Trägern, Antioxidantien und desodorierend wirksamen Mitteln; und insbesondere aus der Gruppe bestehend aus Aromachemikalien, die sich von den genannten Aromachemikalien unterscheiden, die eine oder mehrere Essigsäureester- oder Propionsäureestergruppen enthalten, Tensiden, Ölkomponenten, Lösungsmitteln, Antioxidantien und desodorierend wirksamen Mitteln, mit der Maßgabe, dass die Aromachemikalie, die eine oder mehrere Essigsäureester- oder Propionsäureestergruppen enthält, nicht 4-tert-Butylcyclohexylacetat ist, wenn die Zusammensetzung weniger als 100 ppm Essigsäure enthält; insbesondere mit der Maßgabe, dass die Aromachemikalie, die eine oder mehrere Essigsäureester- oder Propionsäureestergruppen enthält, nicht 4-tert-Butylcyclohexylacetat ist.

11. Die Zusammensetzung nach Anspruch 10, wobei eine oder beide der folgenden Bedingungen (a) und/oder (b) zutreffen:
(a) falls die nicht-aromatischen chemischen Träger ein oder mehrere Lösungsmittel umfassen, sind diese ausgewählt aus der Gruppe bestehend aus Ethanol, Isopropanol, Dipropylenglykol (DPG), Propylenglykol, 1,2-Butylenglykol, Glycerol, Diethylenglykolmonoethylether, Diethylphthalat (DEP), Isopropylmyristat (IPM), Triethylcitrat (TEC), Benzylbenzoat und Mischungen davon;
und/oder
(b) die Aromachemikalien, die sich von den genannten Aromachemikalien unterscheiden, die eine oder mehrere Essigsäureester- oder Propionsäureestergruppen enthalten, sind ausgewählt aus der Gruppe bestehend aus Geranylacetat, alpha-Hexylzimtaldehyd-2-Phenoxyethylisobutyrat, Dihydromyrcenol, Methyldihydrojasmonat (vorzugsweise mit einem Gehalt an cis-Isomer von mehr als 60 Gew.-%), 4,6,6,7,8,8-Hexamethyl-1,3,4,6,7,8 hexahydrocyclopenta[g]benzopyran, Tetrahydrolinalool, Ethyllinalool, Benzylsalicylat, 2-Methyl-3-(4-tert-butylphenyl)propanal, Zimtalkohol, 4,7-Methano-3a,4,5,6,7,7a-hexahydro-5-indenylacetat, 4,7-Methano-3a,4,5,6,7,7a-hexahydro-6-indenylacetat, Citronellol, Citronellylacetat, Tetrahydrogeraniol, Vanillin, Linalylacetat, Styrolylacetat, Octahydro-2,3,8,8-tetramethyl-2-acetonaphthon, 2-Acetyl-1,2,3,4,6,7,8-octahydro-2,3,8,8-tetramethylnaphthalin, Hexylsalicylat, 4-tert-Butylcyclohexylacetat, 2-tert-Butylcyclohexylacetat, alpha-Ionon, n-alpha-Methylionon, alpha-Isomethylionon, Cumarin, Terpinylacetat, 2-Phenylethylalkohol, 4-(4-Hydroxy-4-methylpentyl)-3-cyclohexencarbaldehyd, alpha-Amylzimtaldehyd, Ethylenbrassylat, (E)-3-Methylcyclopentadec- 5-enon, (Z)-3-Methylcyclopentadec-5-enon, 15-Pentadec-11-enolid, 15-Pentadec-12-enolid, 15-Cyclopentadecanolid, 1-(5,6,7,8-Tetrahydro-3,5,5,6,8,8-hexamethyl-2-naphthyl)ethanon, 2-Isobutyl-4-methyltetrahydro-2H-pyran-4-ol, 2-Ethyl-4-(2,2,3-trimethyl-3-cyclopenten-1-yl)-2-buten-1-ol, cis-3-Hexenylacetat, trans-3-Hexenylacetat, trans-2/cis-6-Nonadienol, 2,4-Dimethyl-3-cyclohexencarbaldehyd, 2,4,4,7-Tetramethyloct-6-en-3-on, 2,6-Dimethyl-5-hepten-1-al, Borneol, 3-(3-Isopropylphenyl)butanal, 2-Methyl-3-(3,4-methylendioxyphenyl)propanal, 3-(4-Ethylphenyl)-2,2-dimethylpropanal, 7-Methyl-2H-1,5-benzodioxepin-3(4H)-on, 3,3,5-Trimethylcyclohexylacetat (vorzugsweise mit einem Gehalt an cis-Isomeren von 70 Gew.-% oder mehr), 2,5,5-Trimethyl-1,2,3,4,4a,5,6,7-octahydronaphthalin-2-ol, 3-(4-tert-Butylphenyl)propanal, Ethyl-2-methylpentanoat, Ethoxymethoxycyclododecan, 2,4-Dimethyl-4,4a,5,9b-tetrahydroindeno[1,2-d][1,3]dioxin, (2-tert- Butylcyclohexyl)acetat, 3-[5,5,6-Trimethylbicyclo[2.2.1]hept-2-yl]cyclohexan-1-ol, Menthon, Isomenthon, Carvon, Campher, beta-Ionon, beta-n-Methylionon, beta-Isomethylionon, alpha-Iron, alpha-Damascon, beta-Damascon, beta-Damascenon, delta-Damascon, acetyliertes Zedernholzöl und Mischungen davon.

12. Die Zusammensetzung nach einem der Ansprüche 10 oder 11, die ausgewählt ist aus der Gruppe bestehend aus Parfümzusammensetzungen, Körperpflegezusammensetzungen, Produkten für die Mund- oder Zahnhygiene, Hygieneartikeln, Reinigungszusammensetzungen, Textilwaschmittelzusammensetzungen, Zusammensetzungen für Duftspender und Pflanzenschutzzusammensetzungen.

13. Ein Verfahren zur Herstellung einer Aromachemikalienzusammensetzung, insbesondere einer parfümierten Zusammensetzung, speziell einer parfümierten gebrauchsfertigen Zusammensetzung, umfassend das Einarbeiten mindestens einer Aromachemikalie, die eine oder mehrere Essigsäureester- oder Propionsäureestergruppen enthält, wie in einem der Ansprüche 1 und 4 bis 7 definiert, und Essigsäure in einer solchen Menge, dass sie in der genannten Zusammensetzung in einer Menge von 50 bis 600 ppm, vorzugsweise 70 bis 600 ppm, besonders bevorzugt 70 bis 550 ppm, noch bevorzugter 80 bis 500 ppm, z. B. 80 bis 150 ppm oder 100 bis 500 ppm oder 150 bis 400 ppm oder 200 bis 300 ppm, bezogen auf das Gesamtgewicht von Essigsäure und der genannten mindestens einen Aromachemikalie, in die genannte Zusammensetzung vorhanden ist; oder Mischen von mindestens einer Aromachemikalie, die eine oder mehrere Essigsäureester- oder Propionsäureestergruppen enthält, wie in einem der Ansprüche 1 und 4 bis 7 definiert, und Essigsäure in einer solchen Menge, dass sie in der resultierenden Zusammensetzung in einer Menge von 50 bis 600 ppm, vorzugsweise 70 bis 600 ppm, besonders bevorzugt 80 bis 550 ppm, noch bevorzugter 80 bis 500 ppm, z. B. 80 bis 150 ppm oder 100 bis 500 ppm oder 150 bis 400 ppm oder 200 bis 300 ppm, bezogen auf das Gesamtgewicht von Essigsäure und der genannten mindestens einen Aromachemikalie, gleichzeitig oder nacheinander mit den anderen Komponenten der genannten Zusammensetzung oder mit vorgeformten Mischungen eines Teils der anderen Komponenten der genannten Zusammensetzung vorhanden ist;
mit der Maßgabe, dass die Aromachemikalie, die eine oder mehrere Essigsäureester- oder Propionsäureestergruppen enthält, nicht 4-tert-Butylcyclohexylacetat ist, wenn Essigsäure in der genannten Zusammensetzung in einer Menge von weniger als 100 ppm vorhanden ist; insbesondere mit der Maßgabe, dass die Aromachemikalie, die eine oder mehrere Essigsäureester- oder Propionsäureestergruppen enthält, nicht 4-tert-Bu- tylcyclohexylacetat ist.

14. Ein Verfahren zur Verleihung eines Aromas, vorzugsweise eines Duftes, an eine Zusammensetzung, umfassend das Einarbeiten mindestens einer Aromachemikalie, die eine oder mehrere Essigsäureester- oder Propionsäureestergruppen enthält, wie in einem der Ansprüche 1 und 4 bis 7 definiert, und Essigsäure in einer solchen Menge, dass sie in der genannten Zusammensetzung in einer Menge von 50 bis 600 ppm, vorzugsweise 70 bis 600 ppm, besonders bevorzugt 70 bis 550 ppm, noch bevorzugter 80 bis 500 ppm, z. B. 80 bis 150 ppm oder 100 bis 500 ppm oder 150 bis 400 ppm oder 200 bis 300 ppm, bezogen auf das Gesamtgewicht von Essigsäure und der genannten mindestens einen Aromachemikalie, in die genannte Zusammensetzung vorhanden ist; oder umfassend das Mischen mindestens einer Aromachemikalie, die eine oder mehrere Essigsäureester- oder Propionsäureestergruppen enthält, wie in einem der Ansprüche 1 und 4 bis 7 definiert, und Essigsäure in einer solchen Menge, dass sie in der resultierenden Zusammensetzung in einer Menge von 50 bis 600 ppm, vorzugsweise 70 bis 600 ppm, besonders bevorzugt 70 bis 550 ppm,
noch bevorzugter 80 bis 500 ppm, z. B. 80 bis 150 ppm oder 100 bis 500 ppm oder 150 bis 400 ppm oder 200 bis 300 ppm, bezogen auf das Gesamtgewicht von Essigsäure und der genannten mindestens einen Aromachemikalie, gleichzeitig oder nacheinander mit den anderen Komponenten der genannten Zusammensetzung oder mit vorgeformten Mischungen eines Teils der anderen Komponenten der genannten Zusammensetzung vorhanden ist;
mit der Maßgabe, dass die Aromachemikalie, die eine oder mehrere Essigsäureester- oder Propionsäureestergruppen enthält, nicht 4-tert-Butylcyclohexylacetat ist, wenn Essigsäure in der genannten Zusammensetzung in einer Menge von weniger als 100 ppm vorhanden ist; insbesondere mit der Maßgabe, dass die Aromachemikalie, die eine oder mehrere Essigsäureester- oder Propionsäureestergruppen enthält, nicht 4-tert-Bu- tylcyclohexylacetat ist.

15. Die Verwendung oder die Zusammensetzung oder das Verfahren nach einem der vorhergehenden Ansprüche, wobei die Aromachemikalie, die eine oder mehrere Essigsäureester- oder Propionsäureestergruppen enthält, ausgewählt ist aus der Gruppe bestehend aus 1,4-Butandioldiacetat, Neopentylglykoldiacetat, Isopentylacetat, Linalylacetat, Benzylacetat, Citronellylacetat, Hexylacetat, 2-Phenylethylacetat, α-Terpinylacetat und Mischungen von zwei oder mehr dieser Aromachemikalien, und wobei Essigsäure in einer Menge von 50 bis 500, vorzugsweise 70 bis 500 ppm, besonders bevorzugt 80 bis 500 ppm, noch bevorzugter 100 bis 500 ppm, besonders bevorzugt 100 bis 300 ppm, bezogen auf das Gesamtgewicht von Essigsäure und der genannten mindestens einen Aromachemikalie, verwendet oder enthalten oder vorhanden ist.

## Revendications

1. L'utilisation d'acide acétique pour intensifier ou modifier l'arôme, de préférence le parfum, d'un produit chimique aromatique qui contient un ou plusieurs groupes ester d'acide acétique ou groupes ester d'acide propionique, où l'acide acétique est utilisé en une quantité de 50 à 600 ppm, par rapport au poids total de l'acide acétique et dudit produit chimique aromatique.

2. L'utilisation d'une composition contenant de l'acide acétique et au moins un produit chimique aromatique qui contient un ou plusieurs groupes ester d'acide acétique ou groupes ester d'acide propionique, où l'acide acétique est contenu en une quantité de 50 à 600 ppm, par rapport au poids total de l'acide acétique et dudit au moins un produit chimique aromatique, en tant que composition aromatique, de préférence en tant que parfum.

3. L'utilisation selon l'une quelconque des revendications précédentes, où l'acide acétique est utilisé ou contenu en une quantité de 70 à 600, de préférence de 70 à 550 ppm, plus préférablement de 80 à 500 ppm, par exemple de 80 à 150 ppm ou de 100 à 500 ppm ou de 150 à 400 ppm ou de 200 à 300 ppm, par rapport au poids total de l'acide acétique et dudit au moins un produit chimique aromatique.

4. L'utilisation selon l'une quelconque des revendications précédentes, où le produit chimique aromatique qui contient un ou plusieurs groupes ester d'acide acétique ou groupes ester d'acide propionique est sélectionné dans le groupe constitué du diacétate de 1,4-butanediol, du monoacétate de 1,4-butanediol, du dipropionate de 1,4-butanediol, du monopropionate de 1,4-butanediol, du diacétate de néopentylglycol, du monoacétate de néopentylglycol, de l'acétate d'isopentyle, de l'acétate de linalyle, de l'acétate de 2-tert-butylcyclohexyle, de l'acétate de 4-tert-butylcyclohexyle, de l'acétate de benzyle, de l'acétate de citronellyle, de l'acétate de géranyle, de l'acétate de néryle, de l'acétate de tétrahydrogéranyle, de l'acétate d'hexyle, de l'acétate de 2-octyle, de l'acétate de 2-phényléthyle, de l'acétate d'a-terpinyle, de l'acétate de verdyle, de l'acétate de bornyle, de l'acétate d'isobornyle, de l'acétate d'alcool foliaire (acétate de cis-3-hexényle), et de mélanges de deux ou plusieurs de ces produits chimiques aromatiques.

5. L'utilisation selon l'une quelconque des revendications précédentes, où le produit chimique aromatique qui contient un ou plusieurs groupes ester d'acide acétique ou groupes ester d'acide propionique est un produit chimique aromatique qui contient un ou plusieurs groupes ester d'acide acétique.

6. L'utilisation selon l'une quelconque des revendications 4 ou 5, où le produit chimique aromatique qui contient un ou plusieurs groupes ester d'acide acétique ou groupes ester d'acide propionique est sélectionné dans le groupe constitué du diacétate de 1,4-butanediol, du monoacétate de 1,4-butanediol, du monoacétate de néopentylglycol, du diacétate de néopentylglycol, de l'acétate d'isopentyle, de l'acétate de linalyle, de l'acétate de 2-tert-butylcyclohexyle, de l'acétate de 4-tert-butylcyclohexyle, de l'acétate de benzyle, de l'acétate de citronellyle, de l'acétate de géranyle, de l'acétate de néryle, de l'acétate de tétrahydrogéranyle, de l'acétate d'hexyle, de l'acétate de 2-octyle, de l'acétate de 2-phényléthyle, de l'acétate d'a-terpinyle, de l'acétate de verdyle, de l'acétate de bornyle, de l'acétate d'isobornyle, de l'acétate de cis-3-hexényle, et de mélanges de deux ou plusieurs de ces produits chimiques aromatiques ;
où le produit chimique aromatique qui contient un ou plusieurs groupes ester d'acide acétique ou groupes ester d'acide propionique est de préférence sélectionné dans le groupe constitué du diacétate de 1,4-butanediol, du diacétate de néopentylglycol, de l'acétate d'isopentyle, de l'acétate de linalyle, de l'acétate de 4-tert-butylcyclohexyle, de l'acétate de benzyle, de l'acétate de citronellyle, de l'acétate de géranyle, de l'acétate d'hexyle, de l'acétate de 2-octyle, de l'acétate de 2-phényléthyle, de l'acétate d'a-terpinyle, de l'acétate d'isobornyle, de l'acétate de cis-3-hexényle, et de mélanges de deux ou plusieurs de ces produits chimiques aromatiques.

7. L'utilisation selon la revendication 6, où le produit chimique aromatique qui contient un ou plusieurs groupes ester d'acide acétique ou groupes ester d'acide propionique est sélectionné dans le groupe constitué du diacétate de 1,4-butanediol, de mélanges de diacétate de 1,4-butanediol et de monoacétate de 1,4-butanediol, du diacétate de néopentylglycol, de mélanges de diacétate de néopentylglycol et de monoacétate de néopentylglycol, de l'acétate d'isopentyle, de l'acétate de linalyle, de l'acétate de benzyle, de l'acétate de citronellyle, de l'acétate d'hexyle, de l'acétate de 2-phényl-éthyle, de l'acétate d'a-terpinyle et de mélanges de deux ou plusieurs de ces produits chimiques aromatiques ;
où le produit chimique aromatique qui contient un ou plusieurs groupes ester d'acide acétique ou groupes ester d'acide propionique est de préférence sélectionné dans le groupe constitué du diacétate de 1,4-butanediol, du diacétate de néopentylglycol, de l'acétate d'isopentyle, de l'acétate de linalyle,
de l'acétate de benzyle, de l'acétate de citronellyle, de l'acétate d'hexyle, de l'acétate de 2-phényléthyle, de l'acétate d'a-terpinyle et de mélanges de deux ou plusieurs de ces produits chimiques aromatiques, et est de préférence le diacétate de 1,4-butanediol.

8. Un procédé pour intensifier ou modifier l'arôme, de préférence le parfum, d' un produit chimique aromatique qui contient un ou plusieurs groupes ester d'acide acétique ou groupes ester d'acide propionique tels que définis dans l'une quelconque des revendications 1 et 4 à 7, ou d'un mélange de différents produits chimiques aromatiques qui contiennent un ou plusieurs groupes ester d'acide acétique ou groupes ester d'acide propionique tels que définis dans l'une quelconque des revendications 1 et 4 à 7, comprenant la fourniture d'une composition contenant lesdits un ou plusieurs produits chimiques aromatiques et de 50 à 600 ppm, de préférence de 70 à 600 ppm, plus préférablement de 70 à 550 ppm, encore plus préférablement de 80 à 500 ppm, par exemple de 80 à 150 ppm ou de 100 à 500 ppm ou de 150 à 400 ppm ou de 200 à 300 ppm d'acide acétique, par rapport au poids total de l'acide acétique et desdits un ou plusieurs produits chimiques aromatiques ;
sous réserve que le produit chimique aromatique qui contient un ou plusieurs groupes ester d'acide acétique ou groupes ester d'acide propionique ne soit pas l'acétate de 4-tert-butylcyclohexyle si la composition contient moins de 100 ppm d'acide acétique ; en particulier sous réserve que le produit chimique aromatique qui contient un ou plusieurs groupes ester d'acide acétique ou groupes ester d'acide propionique ne soit pas l'acétate de 4-tert-butylcyclohexyle.

9. Le procédé selon la revendication 8, comprenant l'ajout auxdits un ou plusieurs produits chimiques aromatiques d'acide acétique en une quantité telle qu'une composition comprenant lesdits un ou plusieurs produits chimiques aromatiques et de 50 à 600 ppm, de préférence de 70 à 600 ppm, plus préférablement de 70 à 550 ppm, encore plus préférablement de 80 à 500 ppm, par exemple de 80 à 150 ppm ou de 100 à 500 ppm ou de 150 à 400 ppm ou de 200 à 300 ppm d'acide acétique, par rapport au poids total de l'acide acétique et desdits un ou plusieurs produits chimiques aromatiques, est obtenue ; ou, dans le cas où les un ou plusieurs produits chimiques aromatiques contiennent un ou plusieurs groupes ester d'acide acétique qui sont préparés en utilisant de l'acide acétique ou un dérivé de celui-ci, l'ajustement des conditions de réaction, de purification et/ou d'isolement dans le procédé de prépa-ration desdits un ou plusieurs produits chimiques aromatiques de telle sorte qu'une composition contenant lesdits un ou plusieurs produits chimiques aromatiques et de 50 à 600 ppm, de préférence de 70 à 600 ppm, plus préférablement de 70 à 550 ppm, encore plus préférablement de 80 à 500 ppm, par exemple de 80 à 150 ppm ou de 100 à 500 ppm ou de 200 à 300 ppm d'acide acétique, par rapport à au poids total de l'acide acétique et desdits un ou plusieurs produits chimiques aromatiques est obtenue.

10. Composition comprenant
- au moins un produit chimique aromatique qui contient un ou plusieurs groupes ester d'acide acétique ou groupes ester d'acide propionique tels que définis dans l'une quelconque des revendications 1 et 4 à 7,
- de l'acide acétique en une quantité de 50 à 600 ppm, de préférence de 70 à 600 ppm, plus préférablement de 70 à 550 ppm, encore plus préférablement de 80 à 500 ppm, par exemple de 80 à 150 ppm ou de 100 à 500 ppm ou de 150 à 400 ppm ou de 200 à 300 ppm, par rapport au poids total de l'acide acétique et dudit au moins un produit chimique aromatique, et
- au moins un autre composant sélectionné dans le groupe constitué des produits chimiques aromatiques différents desdits produits chimiques aromatiques qui contiennent un ou plusieurs groupes ester d'acide acétique ou groupes ester d'acide propionique, des supports non-aromatiques chimiques, des anti-oxydants et des agents actifs déodorants ; et en particulier dans le groupe constitué des produits chimiques aromatiques différents desdits produits chimiques aromatiques qui contiennent un ou plusieurs groupes ester d'acide acétique ou groupes ester d'acide propionique, des tensioactifs, des composants huileux, des solvants, des anti-oxydants et des agents actifs déodorants, sous réserve que le produit chimique aromatique qui contient un ou plusieurs groupes ester d'acide acétique ou groupes ester d'acide propionique ne soit pas l'acétate de 4-tert-butylcyclohexyle
si la composition contient moins de 100 ppm d'acide acétique ; en particulier sous réserve que le produit chimique aromatique qui contient un ou plusieurs groupes ester ou groupes ester d'acide propionique ne soit pas l'acétate de 4-tert-butylcyclohexyle.

11. La composition selon la revendication 10, où l'une ou les deux conditions suivantes (a) et/ou (b) s'appliquent :
(a) dans le cas où les supports non-aromatiques chimiques comprennent un ou plusieurs solvants, ceux-ci sont sélectionnés dans le groupe constitué de l'éthanol, de l'isopropanol, du dipropylène glycol (DPG), du propylène glycol, du 1,2-butylène glycol, du glycérol, de l'éther monoéthylique de diéthylène glycol, du phtalate de diéthyle (DEP), du myristate d'isopropyle (IPM), du citrate de triéthyle (TEC), du benzoate de benzyle et de mélanges
de ceux-ci ;
et/ou
(b) les produits chimiques aromatiques différents desdits produits chimiques aromatiques qui contiennent un ou plusieurs groupes ester d'acide acétique ou groupes ester d'acide propionique sont sélectionnés dans le groupe constitué de l'acétate de géranyle, de l'alpha-hexylcinnamaldéhyde, de l'isobutyrate de 2-phénoxyéthyle, du dihydromyrcénol, du méthyl dihydrojasmonate (de préférence avec une teneur en isomère cis supérieure à 60 % en poids), du 4,6,6,7,8,8-hexaméthyl-1,3,4,6,7,8-hexahydrocyclopenta[g]benzopyrane, du tétrahydrolinalool, de l'éthyllinalool, du salicylate de benzyle, du 2-méthyl-3-(4-tert-butylphényl)propanal, de l'alcool cinnamylique, de l'acétate de 4,7-méthano-3a,4,5,6,7,7a-hexahydro-5-indényle, de l'acétate de 4,7-méthano-3a,4,5,6,7,7a-hexahydro-6-indényle, du citronellol, de l'acétate de citronellyle, du tétrahydrogéraniol, de la vanilline, de l'acétate de linalyle, de l'acétate de styrolyle, de l'octahydro-2,3,8,8-tétraméthyl-2-acétonaphthone, de la 2-acétyl-1,2,3,4,6,7,8-octahydro-2,3,8,8-tétraméthylnaphtalène, du salicylate d'hexyle, de l'acétate de 4-tert-butylcyclohexyle, de l'acétate de 2-tert-butylcyclohexyle, de l'alpha-ionone, de la n-alpha-méthylionone, de l'alpha-isométhylionone, de la coumarine, de l'acétate de terpinyle, du 2-phényléthanol, du 4-(4-hydroxy-4-méthylpentyl)-3-cyclohexènecarboxaldéhyde, de l'alpha-amylcinnamaldéhyde, du brassylate d'éthylène, de la (E)-3-méthylcyclopentadéc-5-énone, de la (Z)-3-méthylcyclopentadéc-5-énone, de la 15-pentadéc-11-énolide,de la 15-pentadéc-12-énolide, de la 15-cyclopentadécanolide, de la 1-(5,6,7,8-tétrahydro-3,5,5,6,8,8-hexaméthyl-2-naphthalényl)éthanone, du 2-isobutyl-4-méthyltétrahydro-2H-pyran-4-ol, du 2-éthyl-4-(2,2,3-triméthyl-3-cyclopentén-1-yl)-2-butén-1-ol, de l'acétate de cis-3-hexényle, de l'acétate de trans-3-hexényle,du trans-2/cis-6-nonadiénol, du 2,4-diméthyl-3-cyclohexènecarboxaldéhyde, de la 2,4,4,7-tétraméthyloct-6-én-3-one, du 2,6-diméthyl-5-heptén-1-al, du bornéol, du 3-(3-isopropylphényl)butanal,du 2-méthyl-3-(3,4-méthylènedioxyphényl)propanal,du 3-(4-éthylphényl)-2,2-diméthylpropanal, de la 7-méthyl-2H-1,5-benzodioxépine-3(4H)-one, de l'acétate de 3,3,5-triméthylcyclohexyle (de préférence avec une teneur en isomères cis de 70 % en poids ou plus), du 2,5,5-triméthyl-1,2,3,4,4a,5,6,7-octahydronaphtalén-2-ol, du 3-(4-tert-butylphényl)propanal, du 2-méthylpentanoate d'éthyle, de l'éthoxyméthoxycy clododécane, du 2,4-diméthyl-4,4a,5,9b-tétrahydroindéno[1,2-d][1,3]dioxine, de l'acétate de (2-tert-butylcyclohexyle), du 3-[5,5,6-triméthylbicyclo[2.2.1]hept-2-yl]cyclohexan-1-ol,de la menthone, de l'isoménthone, de la carvone, du camphre, de la bêta-ionone, de la bêta-n-méthylionone,de la bêta-isométhylionone, de l'alpha-irone, de l'alpha-damascone, de la bêta-damascone, de la bêta-damascénone, de la delta-damascone, de l'huile de bois de cèdre acétylée, et de mélanges de ceux-ci.

12. La composition selon l'une quelconque des revendications 10 ou 11, qui est sélectionnée dans le groupe constitué des compositions de parfum, des compositions de soins corporels, des produits pour l'hygiène buccale ou dentaire, des articles d'hygiène, des compositions de nettoyage, des compositions de détergents textiles, des compositions pour diffuseurs de parfum, et des compositions de protection des cultures.

13. Un procédé pour préparer une composition de produits chimiques aromatiques, en particulier une composition parfumée, spécifiquement une composition parfumée prête à l'emploi, comprenant l'incorporation d'au moins un produit chimique aromatique qui contient un ou plusieurs groupes ester d'acide acétique ou groupes ester d'acide propionique tels que définis dans l'une quelconque des revendications 1 et 4 à 7, et d'acide acétique en une quantité telle qu'il est présent dans ladite composition en une quantité de 50 à 600 ppm, de préférence de 70 à 600 ppm, plus préférablement de 70 à 550 ppm, encore plus préférablement de 80 à 500 ppm, par exemple de 80 à 150 ppm ou de 100 à 500 ppm ou de 150 à 400 ppm ou de 200 à 300 ppm, par rapport au poids total de l'acide acétique et dudit au moins un produit chimique aromatique, dans ladite composition ; ou le mélange d'au moins un produit chimique aromatique qui contient un ou plusieurs groupes ester d'acide acétique ou groupes ester d'acide propionique tels que définis dans l'une quelconque des revendications 1 et 4 à 7 et d'acide acétique en une quantité telle qu'il est présent dans la composition résultante en une quantité de 50 à 600 ppm, de préférence de 70 à 600 ppm, plus préférablement de 80 à 550 ppm, encore plus préférablement de 80 à 500 ppm, par exemple de 80 à 150 ppm ou de 100 à 500 ppm ou de 150 à 400 ppm ou de 200 à 300 ppm, par rapport au poids total de l'acide acétique et dudit au moins un produit chimique aromatique, simultanément ou consécutivement avec les autres composants de ladite composition ou avec des mélanges préformés d'une partie des autres composants de ladite composition ;
sous réserve que le produit chimique aromatique qui contient un ou plusieurs groupes ester d'acide acétique ou groupes ester d'acide propionique ne soit pas l'acétate de 4-tert-butylcyclohexyle si l'acide acétique est présent dans ladite composition en une quantité inférieure à 100 ppm ; en particulier sous réserve que le produit chimique aromatique qui contient un ou plusieurs groupes ester d'acide acétique ou groupes ester d'acide propionique ne soit pas le 4-tert-butylcyclohexyle acétate.

14. Un procédé pour conférer un arôme, de préférence un parfum, à une composition, comprenant l'incorporation d'au moins un produit chimique aromatique qui contient un ou plusieurs groupes ester d'acide acétique ou groupes ester d'acide propionique tels que définis dans l'une quelconque des revendications 1 et 4 à 7, et d'acide acétique en une quantité telle qu'il est présent dans ladite composition en une quantité de 50 à 600 ppm, de préférence de 70 à 600 ppm, plus préférablement de 70 à 550 ppm, encore plus préférablement de 80 à 500 ppm, par exemple de 80 à 150 ppm ou de 100 à 500 ppm ou de 150 à 400 ppm ou de 200 à 300 ppm, par rapport au poids total de l'acide acétique et dudit au moins un produit chimique aromatique, dans ladite composition ; ou comprenant
le mélange d'au moins un produit chimique aromatique qui contient un ou plusieurs groupes ester d'acide acétique ou groupes ester d'acide propionique tels que définis dans l'une quelconque des revendications 1 et 4 à 7 et d'acide acétique en une quantité telle qu'il est présent dans la composition résultante en une quantité de 50 à 600 ppm, de préférence de 70 à 600 ppm, plus préférablement de 70 à 550 ppm, encore plus préférablement de 80 à 500 ppm, par exemple de 80 à 150 ppm ou de 100 à 500 ppm ou de 150 à 400 ppm ou de 200 à 300 ppm, par rapport au poids total de l'acide acétique et dudit au moins un produit chimique aromatique, simultanément ou consécutivement avec les autres composants de ladite composition ou avec des mélanges préformés d'une partie des autres composants de ladite composition ;
sous réserve que le produit chimique aromatique qui contient un ou plusieurs groupes ester d'acide acétique ou groupes ester d'acide propionique ne soit pas l'acétate de 4-tert-butylcyclohexyle si l'acide acétique est présent dans ladite composition en une quantité inférieure à 100 ppm ; en particulier sous réserve que le produit chimique aromatique qui contient un ou plusieurs groupes ester d'acide acétique ou groupes ester d'acide propionique ne soit pas le 4-tert-butylcyclohexyle acétate.

15. L'utilisation ou la composition ou le procédé selon l'une quelconque des revendications précédentes, où le produit chimique aromatique qui contient un ou plusieurs groupes ester d'acide acétique ou groupes ester d'acide propionique est sélectionné dans le groupe constitué du 1,4- diacétate de butanediol, du diacétate de néopentylglycol, de l'acétate d'isopentyle, de l'acétate de linalyle, de l'acétate de benzyle, de l'acétate de citronellyle, de l'acétate d'hexyle, de l'acétate de 2-phényléthyle, de l'acétate d'a-terpinyle et de mélanges de deux ou plusieurs de ces produits chimiques aromatiques, et où l'acide acétique est utilisé, contenu ou présent en une quantité de 50 à 500, de préférence de 70 à 500 ppm, plus préférablement de 80 à 500 ppm, encore plus préférablement de 100 à 500 ppm, particulièrement de préférence de 100 à 300 ppm, par rapport au poids total de l'acide acétique et dudit au moins un produit chimique aromatique.
